(19) Europäisches Patentamt
European Patent Office
Office européen des brevets

(11) **EP 4 073 264 B1**

## (12) EUROPEAN PATENT SPECIFICATION

(45) Date of publication and mention
of the grant of the patent:
**20.03.2024 Bulletin 2024/12**

(21) Application number: **20828067.7**

(22) Date of filing: **09.12.2020**

(51) International Patent Classification (IPC):
**C12Q 1/6806** *(2018.01)*

(52) Cooperative Patent Classification (CPC):
(C-Sets available)
**C12Q 1/6806** (Cont.)

(86) International application number:
**PCT/GB2020/053151**

(87) International publication number:
**WO 2021/116677 (17.06.2021 Gazette 2021/24)**

(54) **METHOD FOR WHOLE GENOME SEQUENCING OF PICOGRAM QUANTITIES OF DNA**

METHODE ZUM GANZEN GENOMISCHEN SEQUENZIEREN VON PIKOGRAMM-DNA-MENGEN

PROCÉDÉ DE SÉQUENÇAGE DE GÉNOME ENTIER DE QUANTITÉS DE PICOGRAMME D'ADN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2019 GB 201918043**

(43) Date of publication of application:
**19.10.2022 Bulletin 2022/42**

(73) Proprietor: **Oxford University Innovation Limited**
**Oxford, Oxfordshire OX2 0JB (GB)**

(72) Inventors:
• **AHMED, Ahmed Ashour**
**Oxford, Oxfordshire OX2 0JB (GB)**
• **KERAMI NEJAD RANJPAR, Mohammad**
**Oxford, Oxfordshire OX2 0JB (GB)**

(74) Representative: **Barker Brettell LLP**
**100 Hagley Road**
**Edgbaston**
**Birmingham B16 8QQ (GB)**

(56) References cited:
**WO-A1-2016/138490 WO-A1-2018/152129**

• **SHO SHONAN ET AL: "Precision oncology using a limited number of cells: optimization of whole genome amplification products for sequencing applications", BMC CANCER, vol. 17, no. 1, 1 July 2017 (2017-07-01), XP055775801, DOI: 10.1186/s12885-017-3447-6 Retrieved from the Internet: URL:http://link.springer.com/content/pdf/1 0.1186/s12885-017-3447-6.pdf>**
• **SARAH A VITAK ET AL: "Sequencing thousands of single-cell genomes with combinatorial indexing", NATURE METHODS, vol. 14, no. 3, 30 January 2017 (2017-01-30), pages 302-308, XP055416420, New York ISSN: 1548-7091, DOI: 10.1038/nmeth.4154**
• **FAN ZHANG ET AL: "Haplotype phasing of whole human genomes using bead-based barcode partitioning in a single tube", NATURE BIOTECHNOLOGY, vol. 35, no. 9, 26 June 2017 (2017-06-26), pages 852-857, XP055584000, us ISSN: 1087-0156, DOI: 10.1038/nbt.3897**

Remarks:
The complete document including Reference Table(s) and the Sequence Listing(s) can be downloaded from the EPO website

EP 4 073 264 B1

(52) Cooperative Patent Classification (CPC): (Cont.)

C-Sets
**C12Q 1/6806, C12Q 2535/122, C12Q 2537/143, C12Q 2563/159, C12Q 2563/179**

## Description

This invention relates to a method of preparing an indexed DNA library for sequencing, such as whole genome sequencing of single cells or cell-groups for the identification of single nucleotide variants (SNVs), determining chromosome structural variations, or determining phasing information in the genome of the single cells or cell-groups.

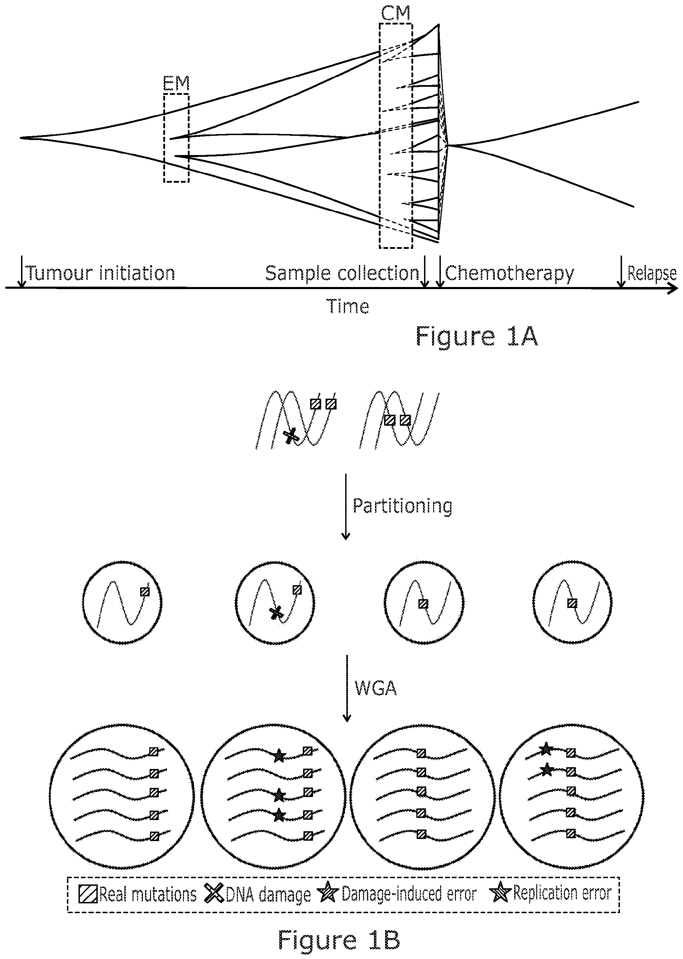

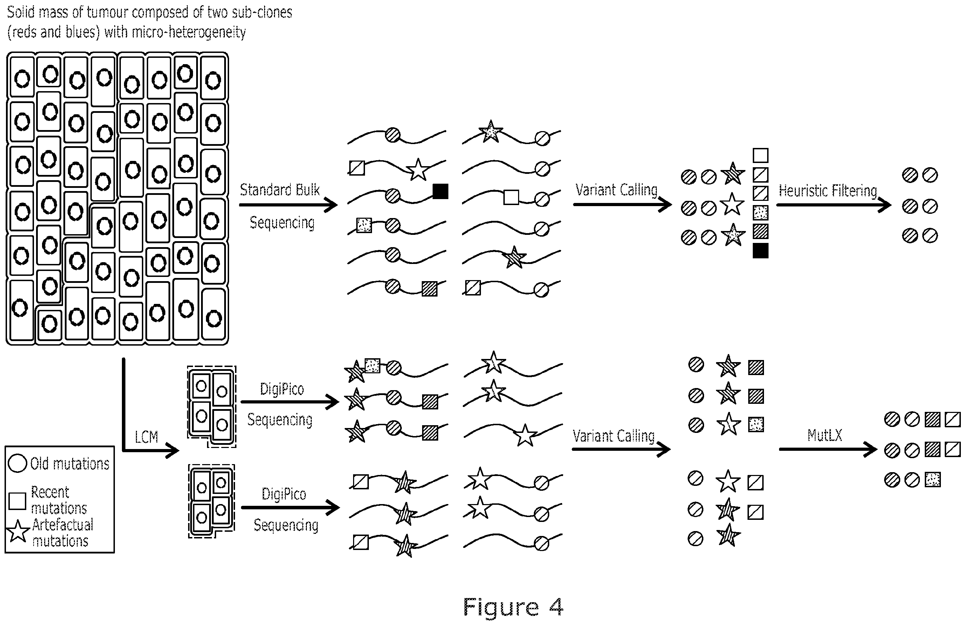

Next generation sequencing has revolutionized our understanding of the genetic evolution of human cells in health and disease. In bulk cancer genome sequencing, inferring the prevalence of variants, the fraction of cells that harbor a variant, enables the computation of the clonal composition of a tumor. In turn, knowledge of the clonal composition enables the construction of evolutionary trees that tell the story of how a particular tumor has evolved over time (1-3). Analyzing shared mutations within individual clones can be used to deduce mutational processes that may have been operational during the evolution of a tumor. Understanding what mutational processes have taken place within a tumor and what drives them mechanistically, is highly desirable since this could provide opportunities for therapeutic intervention or for predicting the evolutionary trajectory of a tumor. However, the limitation of the depth of sequencing means that only highly prevalent mutations that occurred early in tumorigenesis can be detected using standard bulk whole genome sequencing (WGS) approaches (**Figure 1A and Figure 4**). Consequently, the ability to model evolutionary events is limited to early events that have been fixed during the tumor evolution and not recent or current processes (1, 4). This limits the practical application of understanding mutational processes. Studying current or recent evolutionary events requires the confident identification of mutations with very low prevalence (1, 4).

Sequencing of single cells or small populations of spatially-related cells offer the promise to resolve this issue by the detection of cell-specific or clone-specific mutations (**Figure 4**). This gives a readout of mutational processes that are occurring in currently observed cells (**Figure 1A**). However, accurate sequencing of small (picogram) quantities of DNA that can be obtained from single cells or spatially-related cells is highly challenging. Unavoidable DNA damage through oxidation or spontaneous deamination is particularly troublesome when dealing with small quantities of DNA (5). These sources of damage result in disproportionate numbers of artefactual C>A and C>T mutations, respectively (5-7). The identification of these artefactual mutations as variants during variant calling results in a large number of false positive (FP) variant calls. Therefore, whole genome amplification induces profound errors in single nucleotide variant (SNV) calling that preclude accurate estimation of mutation load (6). An important concern is that such mutations can also be attributed to biological processes such as the accumulation of oxidative DNA damage with age or overactivity of members of the APOBEC family of deaminases (8-10).

It was not previously possible to differentiate between biologically-driven C>A and C>T mutations and the artefactual ones that have arisen during library preparation when working with picogram quantities of DNA. In addition, the usual step of whole genome amplification (WGA) prior to sequencing inflates the number of artefactual mutations and propagates the errors caused by DNA damage (5). Several approaches have been proposed to reduce DNA-damage during library preparation or to filter out false positive results during analysis (5, 11-13). However, to date, such techniques still result in the retention of thousands of false positive mutations and, therefore, require extensive validation before firm biological conclusions can be made (5, 11, 12). As extensive validation is not possible in the majority of cases (5), a robust method that eliminates false positive variants from whole genome amplified sequencing data is needed.

A long fragment read (LFR) method for whole-genome sequencing and haplotyping from 10 to 20 human cells was previously published by Complete Genomics Inc. (Peters BA, et al. Accurate whole-genome sequencing and haplotyping from 10 to 20 human cells. Nature. 2012 Jul 11;487(7406):190-5. doi: 10.1038/nature11236. PubMed PMID:22785314; PubMed Central PMCID: PMC3397394). However, this method is highly complex, liable for biases from cross contamination of indices and produces a large number of false positives.

Sho Shonan et al. (BMC CANCER, vol. 17, no. 1, doi: 10.1186/s 12885-017-3447-6), is a publication that describes precision oncology using a limited number of cells, and optimization of whole genome amplification products for sequencing applications.

WO2018152129A1 is a patent application publication that describes methods of preparing nucleic acid libraries, including e.g., expression libraries and/or immune cell receptor repertoire libraries, from double stranded complementary DNA (cDNA) generated through a template-switching reaction involving a RNA sample. In some aspects, the methods include preparing a library from a single cell and/or a library indexed at the single cell level.

Therefore, an aim of the present invention is to provide an improved method to prepare a DNA library for sequencing, SNV analysis, determining chromosome structural variations, or determining phasing information.

According to first aspect of the present invention, there is provided a method of whole genome sequencing in accordance with claim 1 herein.

Described is a method of whole genome sequencing of a single cell or cell-group for identification of single nucleotide variants, determining chromosome structural variations, or determining phasing information in the genome of the single cell or cell-group, the method comprising:

   i) providing a multi-well array plate comprising rows and columns of reaction wells;

ii) providing genomic DNA of single cells or cell-groups, wherein the genomic DNA is distributed into a plurality of reaction wells on the multi-well array plate, such that there is no more than one single-stranded genomic DNA molecule of any given locus per reaction well,

iii) carrying out whole genome amplification (WGA) of each genomic DNA molecule to provide multiple copies of the genomic DNA molecule in each reaction well;

iv) fragmenting the DNA molecules of each reaction well and ligating a pair of looped adapters at each end or tagmenting using transposase-delivered adapters to form adapted-DNA fragments, wherein the looped adapters or transposase-delivered adapters comprise either a Column Index (Ci) sequence or a Row Index (Ri) sequence, wherein the Ci sequence is common to each looped adapter or transposase-delivered adapter of every reaction well in a column of the multi-well array plate, or wherein each Ri sequence is common to each looped adapter or transposase-delivered adapter of every reaction well in a row of the multi-well array plate;

vi) providing the indexed DNA library by performing indexing PCR on the adapted-DNA fragments, wherein the adapted-DNA fragments are amplified to form indexed PCR products using forward and reverse indexing primers, wherein either a Row Index (Ri) sequence or Column Index (Ci) sequence is introduced by each forward and reverse indexing primers onto each end of the adapted-DNA fragments, such that the resulting indexed PCR products comprise both a pair of flanking Column Index (Ci) sequences that are common to each well of a column and a pair of flanking Row Index (Ri) sequences that are common to each well of a row, and

vii) sequencing the indexed DNA library to provide data for determining any single nucleotide variants, determining chromosome structural variations, or determining phasing information in the genome of the single cell or cell-group.

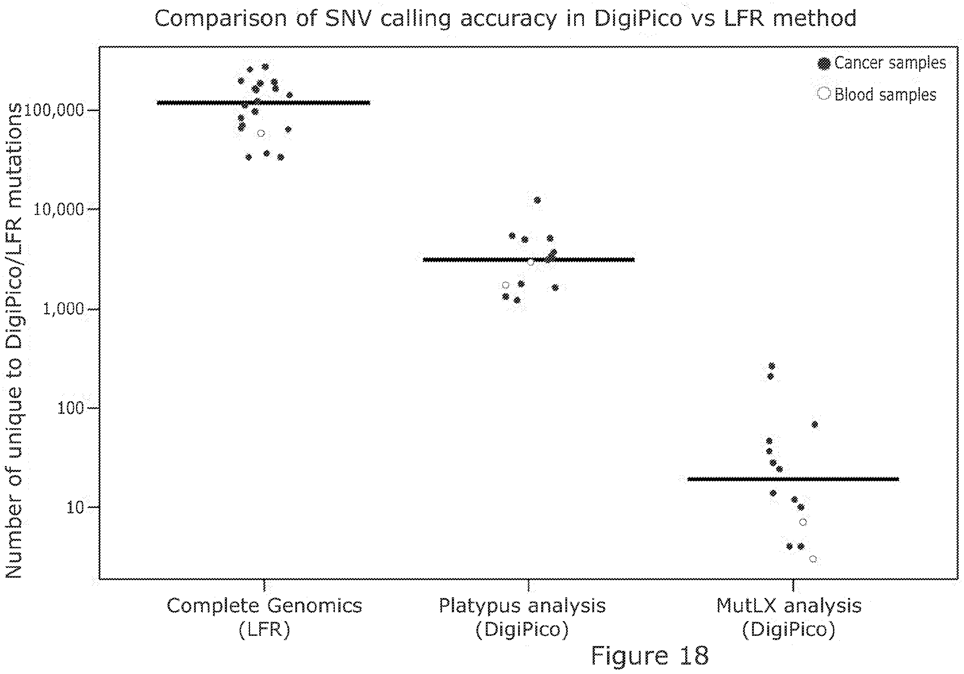

Advantageously, the description herein provides an indexed DNA library for a single DNA molecule sequencing approach to obtain high-quality and data-rich sequencing results from picogram quantities of DNA obtained from clinical samples (termed DigiPico; for Digital sequencing of Picograms of DNA). The description further provides an advantageous indexing strategy to virtually eliminate cross contamination and to improve ligation efficiency. One set of indices are first introduced in the stem loop of the common adapter. At the ligation step, all wells in each column of the plate will receive a different indexing looped adapter or transposase-delivered adapters, therefore a total of 24 different oligos would be sufficient to index all columns of the plate with the first set of indices (Column indices). After the ligation step, all wells in each row can be pooled into a separate tube, resulting in 16 different pools. These 16 different pools can conveniently be purified to be used for the next step of indexing. At the next step the purified products from each pool can be indexed through a PCR reaction using only 16 different indexing primers (Row indices). This allows single cell sequencing at unprecedented accuracy which is a major improvement in the technology over known methods. The described method can be used to identify private mutations and potential neo-antigens in single cells or very small number of cells, and such mutations or neo-antigens can be used as targets for therapy. The described method can also be used to determine chromosome structural variations, such as numerical or structural aberrations, or for determining phasing information. Figure 18 herein clearly shows that the method of the description can greatly enhance the accuracy of determining real nucleotide variants, with a number of false positives removed relative to the LFR method of Complete Genomics Inc., and a more accurate differentiation between samples displaying different numbers of mutations.

**Cells and cell-groups**

The cells or cell-groups may comprise eukaryote cells, such as mammalian cells. In one embodiment, the cells are human. In one embodiment, the cells are at least diploid cells. The cells may be cancerous cells, or pre-cancerous cells. The cells may comprise tumour islets. In one embodiment, the cell may be derived from a tissue biopsy from a subject.

The cells, such as tumour islets, may be laser-captured micro-dissected cells.

Where DNA from a plurality of cells is being sequenced, the cells may be spatially - related cells. The cells may be co-located in a tumour or a region of a tumour. In another embodiment, the cells may or may not be immediate neighbours.

The SNV to be determined may comprise a single nucleotide mutation. The method may be used to determine a plurality of different SNVs in the genomic DNA.

**Providing nucleic acid molecules and well distribution**

The nucleic acid may be purified nucleic acid or partial purified nucleic acid. In another embodiment, the nucleic acid may be provided in a cell lysate. The genomic DNA may be provided as purified DNA. In another embodiment, the genomic DNA may be provided from resuspended nuclei, or whole cells, such as laser-captured micro-dissected cells.

The genomic DNA may comprise DNA of a single cell, or a group of cells (cell-group), such as spatially related cells. The genomic DNA may comprise DNA of between about 1 and 30 cells. The genomic DNA may comprise DNA of between about 1 and 100 cells. In another embodiment, the genomic DNA may comprise DNA of between about 1 and 80 cells. In another embodiment, the genomic DNA may comprise DNA of between about 1 and 50 cells. In another embodiment, the genomic DNA may comprise DNA of between about 1 and 40 cells. In another embodiment, the genomic DNA may comprise DNA of between about 10 and 30 cells. In another embodiment, the genomic DNA may comprise DNA of between about 20 and 30 cells. In another embodiment, the genomic DNA may comprise DNA of between about 20 and 40 cells. In another embodiment, the genomic DNA may comprise DNA of between about 10 and 40 cells.

Where the nucleic acid, such as DNA, is double stranded, the nucleic acid may be denatured prior to distribution into the wells. The denaturing may be achieved by heat and/or denaturing buffer. In one embodiment, the nucleic acid, such as genomic DNA, or nuclei or cells containing genomic DNA may be denatured using a denaturing buffer, such as the D2 buffer from Repli-g single cell kit (Qiagen).

The nucleic acid, such as DNA, may be distributed into the wells such that such that there is no more than one single-stranded genomic DNA molecule of any given locus per reaction well. Distribution of the nucleic acid may be facilitated by dilution of the nucleic acid. Therefore, in one embodiment, the nucleic acid solution may be diluted. The skilled person will readily determine the level of dilution and the volume of the solution necessary for achieving no more than one single-stranded genomic DNA molecule of any given locus per reaction well. The skilled person will recognise that the level of dilution necessary may be determined mathematically such that there is a statistically high probability of there being no more than one single-stranded genomic DNA molecule of any given locus per reaction well. For example Poisson distribution may be used for the calculation when the number of cells is known.

In one embodiment, the DNA content of a single cell may be distributed amongst wells of a single row or column. Therefore, a multi-well array plate may be used to analyse multiple different single cells, such as one per row or column. At least one of the wells may be used for adding the cell and extracting the DNA content. In another embodiment the DNA content of a cell or cell group is distributed amongst wells of both rows and columns of a single multi-well array plate.

The skilled person will recognise that any standard multi-well array plate may be used in the method of the description. Preferably the multi-well array plate is compatible with any PCR and/or sequencing instruments that may be used. The multi-well array plate may comprise a 384 well plate, such as a 24x16 well plate. In another embodiment, the multi-well array plate may comprise a 1536 well plate. The skilled person will appreciate that a larger number of Ri and/or Ci sequences may be required for indexing larger array plates.

The use of a 384 well multi-well array plate can advantageously provide enough wells for distributing diluted genomic DNA strands of about 20-30 cells, such that wells can be provided with a single DNA molecule.

## Amplification

In an embodiment wherein the nucleic acid is genomic DNA, the amplification of the genomic DNA molecules may comprise Whole Genome Amplification (WGA). The WGA may comprise the step of adding amplification reagent for DNA amplification to the genomic DNA. The amplification reagent for DNA amplification may otherwise be termed "amplification mix". The skilled person will understand that an amplification mix may comprise all the reagents necessary for amplification of the DNA (i.e. creating multiple copies of the DNA). Such components may comprise reaction buffer, polymerase, and dNTPs. A DNA polymerisation reporter molecule, such as a DNA-binding dye (e.g. Evagreen™) may be provided in the amplification mix, for example to allow monitoring of the amplification reaction using a real-time PCR. ). The DNA-binding dye may be constructed of two monomeric DNA-binding dyes linked by a flexible spacer. In the absence of DNA, the dimeric dye can assume a looped conformation that is inactive in DNA binding. When DNA is available, the looped conformation can shift via an equilibrium to a random conformation that is capable of binding to DNA to emit fluorescence.

The amplification reagents may be provided in each well prior to or after the addition of the DNA to the wells.

The skilled person will be able to provide suitable conditions for the amplification reaction to occur, including suitable temperature and incubation times. For example, the plate may be incubated at about 30°C for at least about 1 hour followed by heat inactivation, for example at about 65°C for at least 5 minutes.

## Fragmenting and ligation of looped adapters or transposase-delivered adapters

In one embodiment, looped adapters are provided, such that the method comprises a step of fragmenting the DNA molecules of each reaction well and a subsequent ligation reaction to ligate looped adapters to the fragmented DNA. The fragmented DNA may be end repaired prior to the ligation. In an alternative embodiment, transposase-delivered adapters may be provided such that the method comprises fragmenting the DNA molecules by the process of tagmentation. The tagmentation may comprise the provision of a transposase, such as Tn5, carrying oligonucleotides, which are herein termed transposase-delivered adapters. The skilled person will be familiar with the routine technique and reagents for carrying out tagmentation to form the adapted-DNA molecules.

Fragmenting the DNA molecules of each reaction well into multiple dsDNA fragments may comprise direct fragmentation, such as enzymatic or mechanical fragmentation. In one embodiment, the fragmentation of the DNA comprises enzymatic fragmentation.

Fragmenting or tagmentation of the DNA may be provided by the addition of a fragmenting or tagmentation reagent to the DNA in each well. The fragmenting or tagmentation reagent may be concurrently added to each well, for example by the use of a multi-well dispenser, such as an I-DOT (Dispendix, Germany) dispenser or similar. The fragmenting or tagmentation reaction may be timed to provide fragments of the desired size. The skilled person will understand that the timing of the fragmenting or tagmentation reaction can be dependent on the method used, such as the type and level of enzymes provided for the reaction. Therefore, the skilled person may follow standard protocol timings for a given reaction, such as those of a reaction kit.

The fragmenting reagent may comprise restrictions enzymes or nicking enzymes, such as DNase I. Where a nicking enzyme is provided, a single-strand-specific enzyme may be provided that recognizes nicked sites then cleaves the second strand. In one embodiment, a library preparation kit may be used, such as the Lotus DNA library preparation kit (IDT, USA).

Following fragmenting of the DNA to form dsDNA fragments, the dsDNA fragments may be end-repaired and dA-tailed, such that they can be ligated to other DNA molecules, such as the looped adapters. Enzymes for end-repaired and/or dA-tailing may comprise a DNA polymerase, such as T4 DNA polymerase, and a polynucleotide kinase (PNK), such as a T4 polynucleotide kinase. T4 DNA polymerase (in the presence of dNTPs) can fill-in 5' overhangs and trim 3' overhangs down to the dsDNA interface to generate the blunt ends. The T4 PNK can then phosphorylate the 5' terminal nucleotide. A DNA polymerase, such as Taq DNA polymerase, that has terminal transferase activity and leaves a 3' terminal adenine may be provided for A-tailing.

In one embodiment, fragmentation, end-repair, and dA-tailing of dsDNA are all performed in a single reaction.

In one embodiment, the looped adapters may be introduced onto the fragmented DNA by ligation. Ligation of the looped adapters to the dsDNA fragments may comprise the addition of looped adapters and a ligase, such as T4 DNA ligase.

The looped adapters may comprise an oligonucleotide, such as DNA, having a secondary stem-loop structure. The stem-loop structure may be provided by a single oligonucleotide molecule comprising a pair of complementary sequence regions flanking a loop region, wherein the pair of complementary sequences are arranged to hybridise with each other to form the stem-loop structure of the looped adapter. The looped adapters further encode a Column Index (Ci) sequence or Row Index (Ri) sequence in the stem region.

The Column Index (Ci) sequence or Row Index (Ri) sequence may comprise a predetermined sequence that is capable of labelling the DNA as from a row or column respectively. The Column Index (Ci) sequence or Row Index (Ri) sequence may be at least three nucleotides in length.

In one embodiment, the ends of the adapted-DNA fragments may be symmetrical. In particular, the looped adapters or transposase-delivered adapters ligated to each end of the dsDNA fragment are identical, such that each dsDNA fragment receives a pair of identical flanking looped adapters or transposase-delivered adapters. The pair of Ci sequences on the same adapted-DNA fragment may be the same. Alternatively, if Ri sequences are provided, the pair of Ri sequences on the same adapted-DNA fragment may be the same.

Advantageously, the provision of two identical Ci or Ri sequences on the adapted-DNA fragments provides a marker to avoid analysis of indexed DNA library sequences that are a result of cross-contamination between different columns, or different rows respectively. In particular, any indexed DNA library sequence that does not have matching Ci sequences at each end can be discarded from the data analysis. Alternatively, if Ri sequences are provided, any indexed DNA library sequence that does not have matching Ri sequences at each end can be discarded from analysis. This can provide a first level of redundancy to eliminate index cross-contamination from the subsequent data, which is a significant problem in the preparation of indexed libraries and their subsequent analysis.

The looped adapters may provide a 3' or 5' overhang to aid ligation to the dsDNA fragments. The 3' or 5' overhang may be provided when the stem region of looped adapter is hybridised together (i.e. the looped adapter is in a secondary/stem-loop structure). The 3' or 5' overhang may correspond to a complementary overhang on the dsDNA fragments that have been end repaired and prepared for ligation. The overhang may comprise a single thymine.

The looped adapter sequence may comprise the sequence of SEQ ID NO: 1, or a functional variant thereof.

Following ligation of the looped adapters, the single-stranded region of looped DNA may be cleaved. The single-stranded region of looped DNA may be enzymatically cleaved, for example by USER (Uracil-Specific Excision Reagent) enzyme, which generates a single nucleotide gap at the location of a uracil present in the loop. Therefore, in one embodiment, the looped adapters may comprise a uracil in the loop region.

## Pooling of a row of wells

If Ci sequences are provided in the adapted-DNA fragments, the method may additionally comprise the step of pooling the adapted-DNA fragments of each reaction well in a row prior to the indexing PCR. Alternatively, if Ri sequences are provided in the adapted-DNA fragments, the method may additionally comprise the step of pooling the adapted-DNA fragments of each reaction well in a column prior to the indexing PCR. The pooled adapted-DNA fragments may then be used for indexing PCR in a single pooled reaction for each row, or each column, depending on which is pooled. In an alternative embodiment, the columns or rows may not be pooled prior to carrying out index PCR.

Advantageously, the pooling of the rows or columns prior to indexing PCR greatly improves the efficiency of the library preparation. For example, for a 16x24 (384) well plate only 16 separate indexing PCR reactions are required if the 16 rows are pooled between the introduction of the looped adapters or transposase-delivered adapters and the indexing PCR steps, instead of 384 indexing PCR reactions if they are not pooled.

**Size Selection and Indexing PCR**

Prior to the indexing PCR, the adapted-DNA fragments may be selected for size, for example to also remove the self-ligated adapters from the reaction when applicable. An example of a desired size may be about 300-400 bp in length. The size selection may be provided by isolating or purifying the adapted-DNA fragments of the desired length, for example using a gel, or beads. SPRI beads (Solid Phase Reversible Immobilisation beads) may be used for size selection. SPRI beads can comprise magnetic particles coated with carboxyl groups (in the form of succinic acid) that can bind DNA non-specifically and reversibly.

The indexing PCR may comprise the step of mixing the adapted-DNA fragments with a set of forward and reverse indexing PCR primers and reagents for PCR. The forward and reverse indexing PCR primers may comprise a sequence that is arranged to hybridise to a sequence of the adapted-DNA fragments for priming the polymerisation. The sequences that are arranged to hybridise to sequences of the adapted-DNA fragments for priming the polymerisation may be complementary sequences. The sequences for priming the polymerisation from the forward and reverse indexing PCR primers may be provided by the looped-adapters or transposase-delivered adapters. The sequences for priming the polymerisation from the forward and reverse indexing PCR primers may be flanking the Ci or Ri sequences of the adapted-DNA fragments, such that the Ci or Ri sequences become incorporated in the indexed PCR product.

The complementary sequences for hybridisation, which are provided by the forward and reverse primers, may each be between about 15 and 30 nucleotides in length, such as about 26 nucleotides in length.

In an embodiment wherein the adapted-DNA fragments comprise Ci sequences, the forward and reverse indexing PCR primers may each comprise Ri sequences, for providing a pair of Ri sequences in the indexed PCR product. Where the rows are pooled, the Ri sequences will be added to each adapted-DNA fragment in the pool (from all the wells of a row). Alternatively, where the rows are not pooled, the same Ri sequence may be provided for each well in a row.

In an alternative embodiment wherein the adapted-DNA fragments comprise Ri sequences, the forward and reverse indexing PCR primers may each comprise Ci sequences, for providing a pair of Ci sequences in the indexed PCR product. Where the columns are pooled, the Ci sequences will be added to each adapted-DNA fragment in the pool (from all the wells of a column). Alternatively, where the columns are not pooled, the same Ci sequence may be provided for each well in a column.

The Row Index (Ri) sequences, which may be provided by the forward and reverse primers, may be the same for each adapted-DNA fragment of, or from, a row. Alternatively, the Column Index (Ci) sequences, which may be provided by the forward and reverse primers, may be the same for each adapted-DNA fragment of, or from, a column.

The Row Index (Ri) sequences or Column Index (Ci) sequences provided by the forward and reverse primers may each be at least three nucleotides in length, such as about 8 nucleotides in length.

The resulting ends of the indexed PCR products may be symmetrical. For example, the sequences flanking the original DNA fragment sequence may be symmetrical. The indexed PCR products may comprise the DNA fragments sequence flanked by a pair of identical Ci sequences (i.e. inner flank), and further flanked by a pair of identical Ri sequences (i.e. outer flank). In an alternative embodiment, the indexed PCR products may comprise the DNA fragments sequence flanked by a pair of identical Ri sequences (i.e. inner flank), and further flanked by a pair of identical Ci sequences (i.e. outer flank).

Advantageously, the provision of two identical pairs of Ci or Ri sequences on the indexed DNA fragments in addition to the previously provided Ri or Ci sequences (provided by the looped adapters or transposase-delivered adapters) respectively, provides a further marker to avoid analysis of indexed DNA library sequences that are a result of cross-contamination between different columns, or different rows respectively. In particular, any indexed DNA library sequence that does not have matching Ci sequences at each end can be discarded from the data analysis. Alternatively, if Ri sequences are provided, any indexed DNA library sequence that does not have matching Ri sequences at each end can be discarded from analysis. Providing both pairs of matching Ci and Ri sequences on an indexed DNA fragment can provide a first and second level of redundancy to eliminate index cross-contamination from the subsequent data, which is a significant problem in the preparation of indexed libraries and their subsequent analysis.

The forward and reverse indexing PCR primers may further comprise sequencing adapter sequences, such that sequencing adapters are incorporated into the indexed PCR product. The sequencing adapter sequences on the primers may be 5'.

The sequencing adapters may be terminal on the indexed PCR product. Where sequencing adapter sequences are provided, the resulting ends of the indexed PCR products may not be symmetrical. For example, one end of the indexed PCR product may be adapted with a sequencing adapter that is different to the sequencing adapter of the other end. The skilled person will be aware of sequencing adapters that may be required for a given sequencing technology. For example, in the case of dye sequencing (e.g. Illumina dye sequencing), the sequencing adapters may be P5 and P7 sequencing adapters (i.e. P5 at one end of the indexed PCR product, and P7 at the other). The indexing primer providing the P5 sequence may comprise the sequence of SEQ ID NO: 2. The indexing primer providing the P7 sequence may comprise the sequence of SEQ ID NO: 3.

Once formed, an indexed PCR product may be termed an "indexed DNA library sequence" or "indexed DNA fragment". The pooled indexed PCR products, indexed DNA sequences, or indexed DNA fragments, may be termed an "indexed DNA library".

**The indexed DNA Library**

The indexed DNA fragment sizes of the indexed DNA library may be filtered, such that only indexed DNA fragments of a desired or suitable length are available for sequencing. After the indexing PCR the indexed PCR fragments may be purified/isolated, for example by beads (e.g. SPRI beads). The purification may remove undesirable short fragments, primer-dimers or other PCR artefacts or reagents.

The indexed library may be checked for appropriate size distribution, We usually check the library size distribution, for example on tapestation or bioanalyzer instruments (Agilent), or similar.The size of the indexed DNA library may be adjusted by dilution after preparation for sequencing, for example to about 4nM.

The indexed DNA library may be stored for later use, such as sequencing. For example, the DNA library may be stored frozen or chilled.

**Sequencing of the indexed DNA library**

The indexed DNA library may be sequenced, or adapted to be sequenced. The sequencing may be next generation sequencing (NGS). The sequencing may be dye-sequencing (e.g. Illumina dye-sequencing), nanopore sequencing, or ion torrent sequencing. The skilled person will be familiar with a number of different sequencing technologies/methods that may be used, and the required sequencing adapters therefor.

The sequencing may be multiplexed sequencing, where multiple indexed DNA libraries are sequenced simultaneously.

**Determination of mutations/nucleotide variations and data analysis**

The method may comprise determining any real SNVs in the genome of the single cell or cell-group by determining if substantially all indexed DNA library sequences originating from a single well comprise the same SNV, or if only a fraction of the indexed DNA library sequences comprise the same SNV. A SNV represented in substantially all indexed DNA library sequences originating from a single well may be determined to be a real SNV in the genomic DNA. Additionally or alternatively, a SNV found in only a fraction of the indexed DNA library sequences originating from a single well may be determined to be a false positive (FP) SNV. The false positive SNV may be a damage-induced error or a replication error.

The method may further comprise matching indexed DNA library sequences originating from a single well representing one strand of the genomic DNA with indexed DNA library sequences originating from another well representing the complementary strand of genomic DNA. A SNV substantially present in all indexed DNA library sequences of both complementary strands of the genomic DNA may be determined to be a real SNV. A SNV not substantially present in all indexed DNA library sequences of both complementary strands of genomic DNA may be determined to be false positive (i.e. not a real SNV).

The step of determining if substantially all indexed DNA library sequences originating from a single well comprise the same SNV, or if only a fraction of the indexed DNA library sequences comprise the same SNV, may be carried out *in silico,* for example using the BAM file data. Additionally or alternatively, the step of matching indexed DNA library sequences originating from a single well representing one strand of the genomic DNA with indexed DNA library sequences originating from another well representing the complementary strand of genomic DNA may be carried out *in silico,* for example using the BAM file data.

In one embodiment, the sequencing data from a tumour cell, suspected tumour cell, or pre-cancerous cell may be compared to sequencing data obtained from a normal cell (i.e. non-cancerous cell) taken from normal tissue (i.e.

non-cancerous tissue), for example as a control. Therefore, in one embodiment, the method comprises the preparation of an indexed DNA library from both a tumour cell, suspected tumour cell, or pre-cancerous cells, and a normal (i.e. non-cancerous) cell. The indexed DNA library may be prepared for each cell type in parallel, for example in different wells of the same multi-well plate, or separately. The sequencing of indexed DNA libraries from different types of cell may run in the same sequencing run. The different types of cell (e.g. cancerous or normal) may be from the same subject.

A probability score of a particular nucleotide variant being a real SNV or a false positive may be calculated *in silico,* such that a given variant nucleotide is determined to have a statistically significant probability of being a real SNV or a false positive.

In one embodiment, sequencing the DNA library to determine SNVs within the library comprises generating multiplexed sequencing data from multiple wells and analysing the data for SNVs.

In one embodiment, analysing the data for SNVs comprises de-multiplexing the sequencing data, such that the data from each well is assigned to individual well groups. Additionally, separate indexed DNA libraries may be sequenced in the same sequencing run, therefore, the method may further comprise de-multiplexing the sequencing data such that different indexed DNA libraries are identified/grouped.

The sequence data provided may be in the form of paired-read FastQ files. The sequence data, for example Paired-read FastQ files, may be trimmed for removal of adapter sequences. The sequence data, for example paired-read FastQ files, may also be trimmed for quality. The skilled person will be able to readily adjust the desired threshold for the quality score of each base read in the sequence, for example using a program such as TrimGalore. The resulting data may be termed "trimmed data".

In one embodiment, analysing the data for SNVs comprises mapping the sequence data to a reference genome, such as human hg19 reference genome, to generate aligned sequencing data in the form of a sequence alignment map (SAM) or a binary file version thereof (e.g. a BAM file). The mapping to a reference genome may use the trimmed read data. The SAM or BAM file data may be used in the determination of SNVs present for each well.

Mapping may use a program such as Bowtie2 with the ignore-quals parameter activated and duplicate reads marked, for example using Picard Tools.

Joint variant calling may be performed on all individual BAM files together with a merged BAM file from all wells, for example using a variant caller program, such as the Platypus variant caller.

Low quality (i.e. low confidence) variants may be filtered out of the data. For example, low-quality (i.e. low confidence) variants may be removed from the data by applying quality filters. Example quality filters in the Platypus caller may comprise QUAL > 60, FR > 0.1, HP ≤ 4, QD > 10, and SbPval ≤ 0.95. The skilled person will recognise that filtering out low confidence variants is a routine procedure and each variant caller may have various confidence scores for each variant depending on their algorithm, and which can be used to filter low confidence (quality) ones. Therefore, the particular parameters can depend on the variant caller employed.

The total number of wells covering each locus (Tw) and the number of wells supporting each variant (Vw) may be determined. Well count filters, such as Tw > 5, Vw > 2, and Vw/Tw > 0.1, may be applied to only retain the high confidence loci for analysis.

Regions of the genome with bad mappability (i.e. known regions where it is more likely to misalign a read) may be removed from the analysis, for example using VCFtools.

A resulting list of high confidence variants that have been identified in the data may then be used to perform variant re-calling (genotyping) on WGS data, for example from blood, and the bulk of a tumour, for example using Platypus. The Platypus minPosterior parameter may be set to 0 and minMapQual parameter may be set to 5. Any variant that is confidently unsupported in both of the standard WGS data may be extracted as a UTD (Unique to DigiPico) variant. Any variant that is confidently also present in the bulk sequencing data of the blood sample (based on GATK analysis) may be extracted as a TP (True Positive) variant.

## Use of an artificial neural network (ANN)

*In silico* determinations or matching of indexed DNA sequences, and/or the calculation of probability scores may be carried out in accordance with the methods and calculations described herein. In one embodiment, the *in silico* determinations or matching of indexed DNA sequences, and/or the calculation of probability scores may be carried out by an artificial neural network (ANN) model, such as by a multilayer perceptron.

The multilayer perceptron may have an input layer consisting of N neurons (e.g. N=41), where N is the number of features used in each experiment. The ANN model may comprise at least two hidden layers with ReLU (Rectified Linear Unit) activations. The last layer of the ANN may be a single output neuron with a sigmoid activation. The loss function may be binary cross-entropy.

The ANN may be programmed for example in Python3 using Keras. The skilled person will recognise that Keras is a free open source Python library for developing and evaluating deep learning models. However, other libraries may be used.

The ANN may be pre-trained with one or more datasets. For example, the ANN maybe trained with datasets comprising known nucleotide variants.

**Other Aspects**

Also described is a method of preparing an indexed DNA library for sequencing of nucleic acid molecules the method comprising:

i) providing a multi-well array plate comprising rows and columns of reaction wells;

ii) providing nucleic acid molecules, wherein the nucleic acid molecules are distributed into a plurality of reaction wells on the multi-well array plate, such that there is no more than one single-stranded nucleic acid molecule from any given locus per reaction well,

iii) carrying out amplification of the nucleic acid molecule to provide multiple DNA copies of the nucleic acid molecule in each reaction well;

iv) fragmenting the DNA molecules of each reaction well and ligating a pair of looped adapters at each end or tagmenting using transposase-delivered adapters to form adapted-DNA fragments, wherein the looped adapters or transposase-delivered adapters comprise either a Column Index (Ci) sequence or a Row Index (Ri) sequence, wherein the Ci sequence is common to each looped adapter or transposase-delivered adapter of every reaction well in a column of the multi-well array plate, or wherein each Ri sequence is common to each looped adapter or transposase-delivered adapter of every reaction well in a row of the multi-well array plate;

vi) providing the indexed DNA library by performing indexing PCR on the adapted-DNA fragments, wherein the adapted-DNA fragments are amplified to form indexed PCR products using forward and reverse indexing primers, wherein either a Row Index (Ri) sequence or Column Index (Ci) sequence is introduced by each forward and reverse indexing primers onto each end of the adapted-DNA fragments, such that the resulting indexed PCR products comprise both a pair of flanking Column Index (Ci) sequences that are common to each well of a column and a pair of flanking Row Index (Ri) sequences that are common to each well of a row, and

optionally wherein the forward and reverse indexing primers further provide respective 5' and 3' sequencing adapters onto the indexed PCR products that are suitable for use in a sequencing reaction.

The nucleic acid may be DNA or RNA. In one embodiment, the nucleic acid is genomic DNA. In another embodiment, the nucleic acid may be mRNA.

Also described is a method of preparing an indexed DNA library for whole genome sequencing of single cells or cell-groups for the identification of single nucleotide variants, determining chromosome structural variations, or determining phasing information in the genome of the single cells or cell-groups, the method comprising:

i) providing a multi-well array plate comprising rows and columns of reaction wells;

ii) providing genomic DNA of single cells or cell-groups, wherein the genomic DNA is distributed into a plurality of reaction wells on the multi-well array plate, such that there is no more than one single-stranded genomic DNA molecule of any given locus per reaction well,

iii) carrying out whole genome amplification (WGA) of each genomic DNA molecule to provide multiple copies of the genomic DNA molecule in each reaction well;

iv) fragmenting the DNA molecules of each reaction well and ligating a pair of looped adapters at each end or tagmenting using transposase-delivered adapters to form adapted-DNA fragments, wherein the looped adapters or transposase-delivered adapters comprise either a Column Index (Ci) sequence or a Row Index (Ri) sequence, wherein the Ci sequence is common to each looped adapter or transposase-delivered adapter of every reaction well in a column of the multi-well array plate, or wherein each Ri sequence is common to each looped adapter or transposase-delivered adapter of every reaction well in a row of the multi-well array plate;

vi) providing the indexed DNA library by performing indexing PCR on the adapted-DNA fragments, wherein the adapted-DNA fragments are amplified to form indexed PCR products using forward and reverse indexing primers, wherein either a Row Index (Ri) sequence or Column Index (Ci) sequence is introduced by each forward and reverse indexing primers onto each end of the adapted-DNA fragments, such that the resulting indexed PCR products comprise both a pair of flanking Column Index (Ci) sequences that are common to each well of a column and a pair of flanking Row Index (Ri) sequences that are common to each well of a row, and

optionally wherein the forward and reverse indexing primers further provide respective 5' and 3' sequencing adapters onto the indexed PCR products that are suitable for use in a sequencing reaction.

The indexed nucleic acid may be sequenced, for example as described herein.

Therefore, also described is a method of whole genome sequencing of a single cell or cell-group to provide data for the identification of single nucleotide variants (SNVs) in the genome of the single cell or cell-group, the method comprising:

i) preparing an indexed DNA library by carrying out the method according to the description herein, or providing an indexed DNA library prepared in accordance with the method of the description herein;
ii) sequencing the indexed DNA library to provide data for determining any single nucleotide variants (SNVs) in the genome of the single cell or cell-group.

The sequencing data may be used to determine SNVs, for example as described herein. Additionally or alternatively, the sequencing data may be used to determine genetic changes relating to chromosome structural variations. The chromosome aberration may comprise a numerical and/or structural aberration.

Additionally or alternatively, the sequencing data may be used to determine phasing information in the cell or cell group.

The description may also include one or more features, either singularly or in combination, as disclosed in the description and/or in the drawings.

## DEFINITIONS

The term "spatially related cells" is understood to mean cells that are immediate neighbours to each other.

The term "false positive (FP) mutation" or "false positive (FP) SNV" is understood to mean a variant nucleotide that was not present in the genome prior to DNA extraction from intact cells, for example, a false mutation may be a damage-induced error or a replication error.

The terms "real mutation/SNV" or "true positive mutation/SNV" may be used interchangeably, and are understood to mean a variant nucleotide that is present in genomic DNA of living cells prior to DNA extraction.

The term "single nucleotide variant" (SNV) may include single nucleotide polymorphisms (SNPs), or any other variation in sequence, such as a mutation. Mutations or variations may include nucleotide substitutions, additions or deletions in a given sequence.

A "chromosomal aberration" is understood to be a missing, extra, or irregular portion of chromosomal DNA. It can be from a typical number of chromosomes or a structural abnormality in one or more chromosomes. They include a variety of aberrations such as deletions, duplications, and insertions. Balanced aberrations such as inversions and inter-chromosomal and intra-chromosomal translocations can occur. In addition, mobile element insertion, segmental duplications, multi-allelic chromosome numeric aberrations can occur. Final multiple combinations of the above can produce complex rearrangements.

"Phasing" is understood to be the task or process of assigning alleles (the As, Cs, Ts and Gs) to the paternal and maternal chromosomes. Phasing can help to determine whether matches are on the paternal side or the maternal side, on both sides or on neither side. Phasing can also help with the process of chromosome mapping - assigning segments to specific ancestors.

The skilled person will understand that optional features of one embodiment or aspect of the description may be applicable, where appropriate, to other embodiments or aspects of the description.

Embodiments of the description will now be described in more detail, by way of example only, with reference to the accompanying drawings.

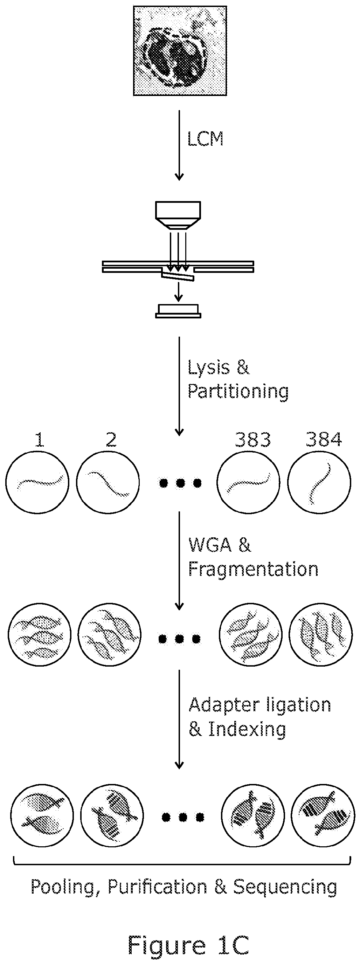

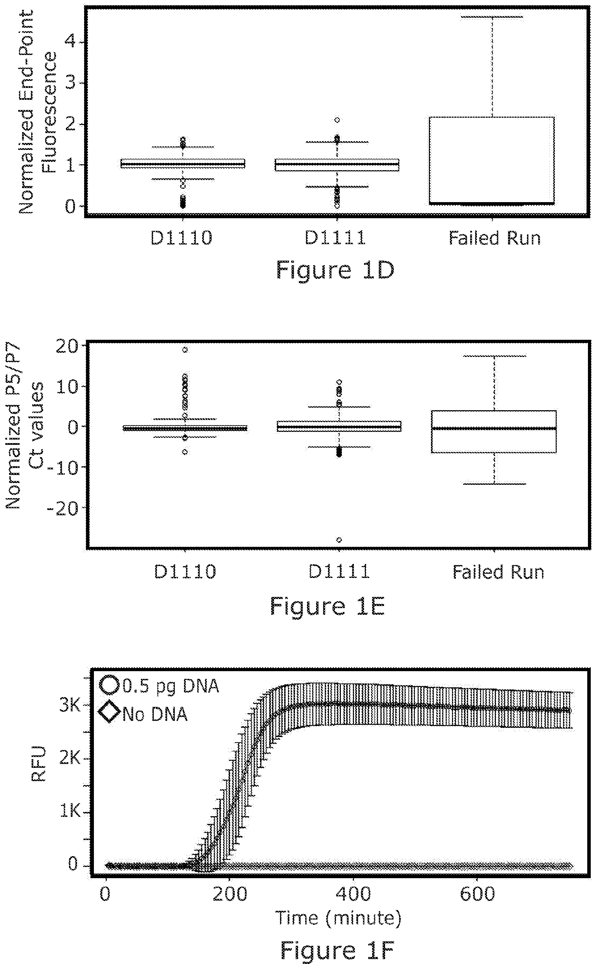

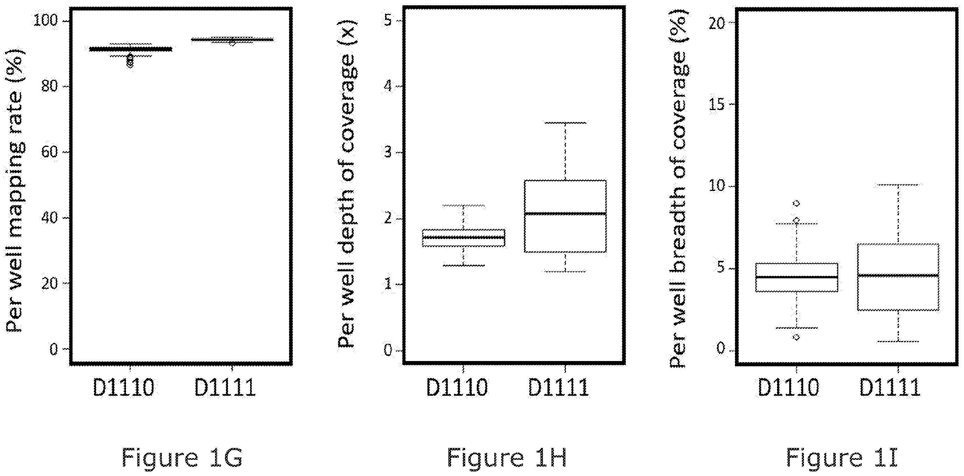

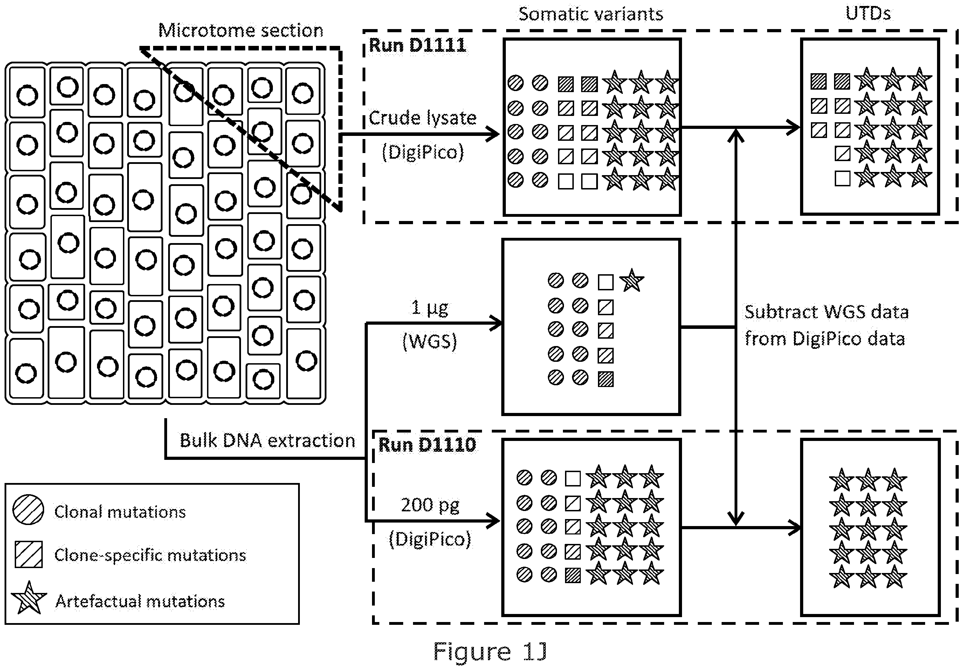

**Figure 1. DigiPico sequencing rationale, workflow, and performance. (A)** WGS approaches can only identify early mutational processes (EM) in dominant expanded clones in a tumor (red and blue). Currently active mutational processes (CM) result in a diverse set of sub-clones with different clone-specific mutations. This diversity determines the evolutionary trajectory of the tumor. **(B)** Template partitioning prior to WGA so that each compartment receives no more than one DNA molecule from each locus allows for the identification of artificial mutations. Since damage-induced errors (red) and replication errors (cyan) occur stochastically during replication, artefactual mutations result in dual-allelic compartments. Note that real mutations are always present in all product DNA strands within the same compartment. **(C)** DigiPico sequencing workflow. LCM: Laser-capture micro-dissection. **(D)** End-point relative fluorescent unit (RFU) from EvaGreen-labeled DNA was used to ensure homogeneous distribution of template and WGA process across the plate. RFU values were normalized to achieve a median of 1 in each run. **(E)** Per well qPCR using Illumina adapter primers (P5 and P7) measures the relative quantity of adapter-ligated products in each well. Ct values were normalized to achieve a median of 0 in each run. **(F)** Streamlining the DigiPico library preparation process required miniaturized a WGA that can specifically and sensitively amplify sub-picogram quantities of DNA in every well. Values represent the mean RFU values across 9 replicates. Error bars represent SD. **(G, H, and I)** preliminary analysis of DigiPico sequencing data from individual wells in each run confirms sequencing high-quality and homogeneity of mapping rate, depth of coverage and breadth of coverage as indicated. **(J)** Definition of unique to DigiPico (UTD) variants. Subtracting the SNVs that are identifiable in standard WGS data from corresponding DigiPico data results in UTD variants. These will mainly be consisted of artefactual mutations as well as some clone-specific mutations. Since the template in run D1110 is practically a subset of the template used for the standard WGS, all true variants in the DigiPico run D1110 are expected to also be present in the standard WGS data. In contrast, clone-specific variants in run D 1111 are likely to be absent in the standard WGS data because of depth limitation, even though the DNA molecules supporting such variants might have been present in the bulk DNA sample at very low frequencies. In all boxplots the horizontal line represents the median. Boxes represent interquartile range (between the 25th and the 75th percentile). Whiskers represent the range excluding outliers. Outliers are defined as data points above or below 1.5 times the interquartile range.

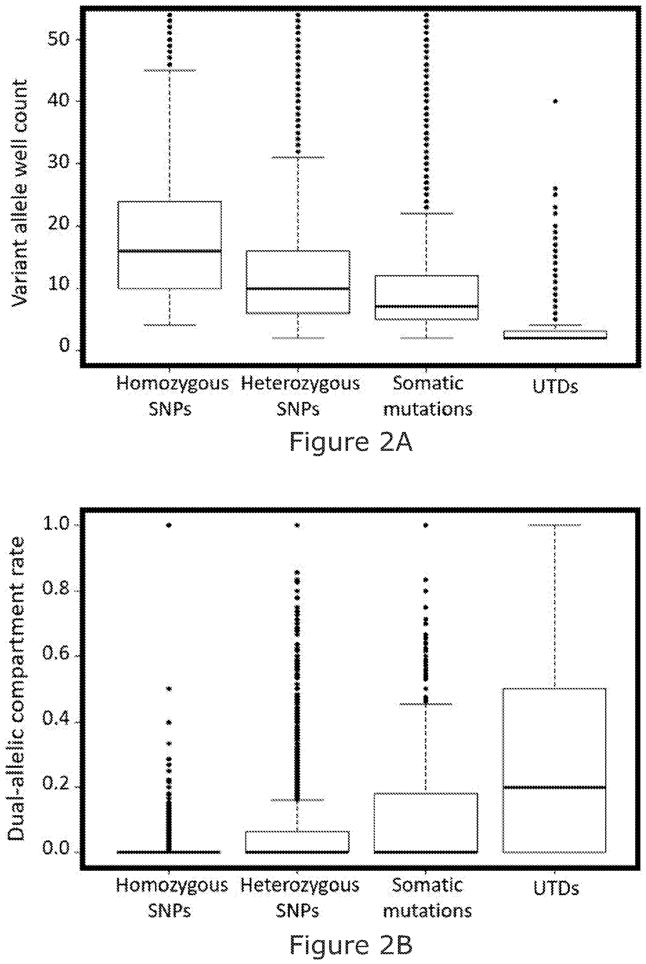

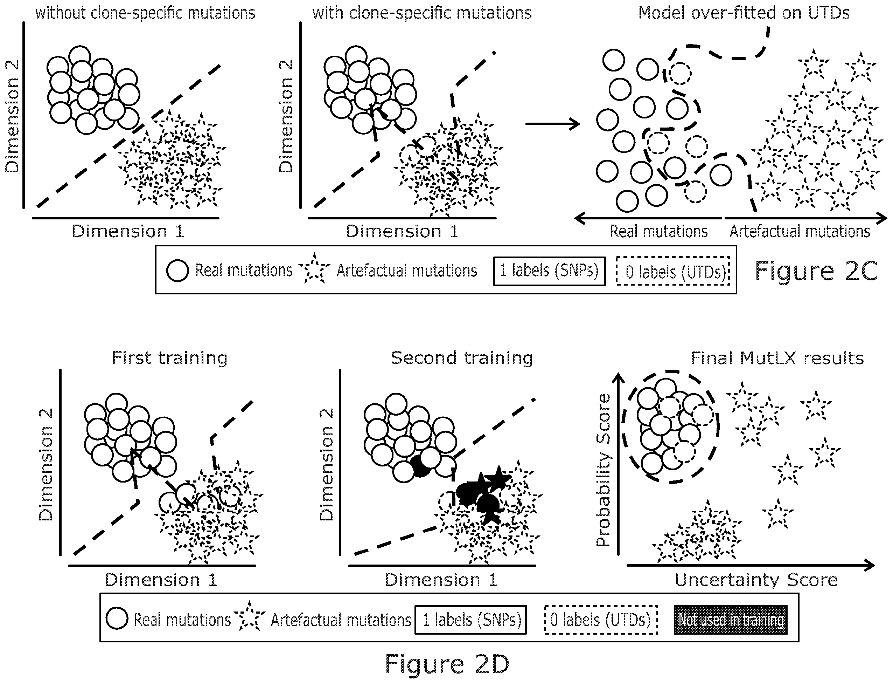

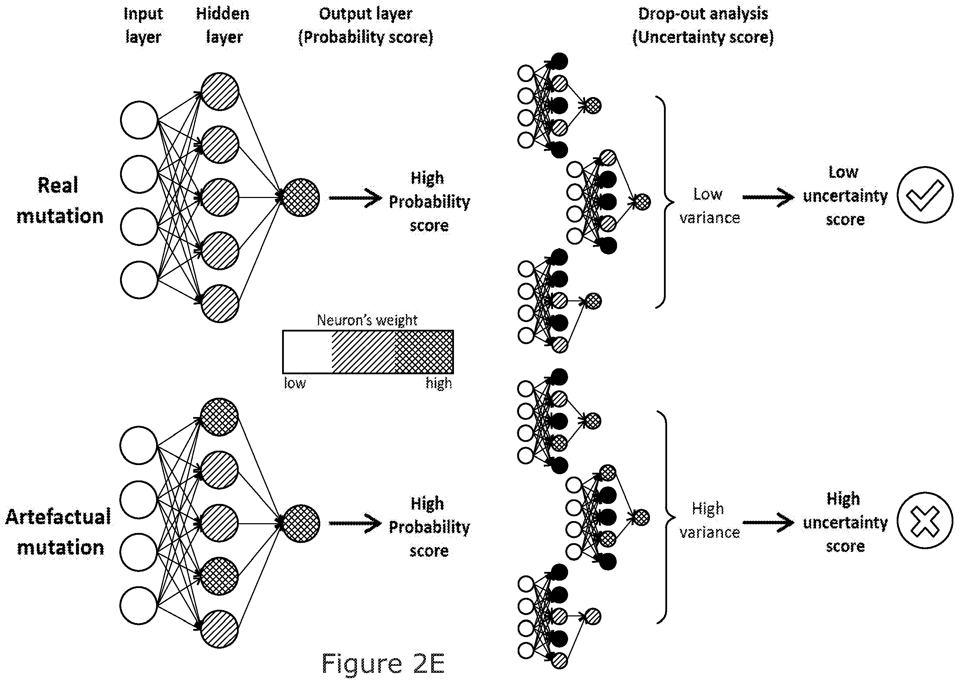

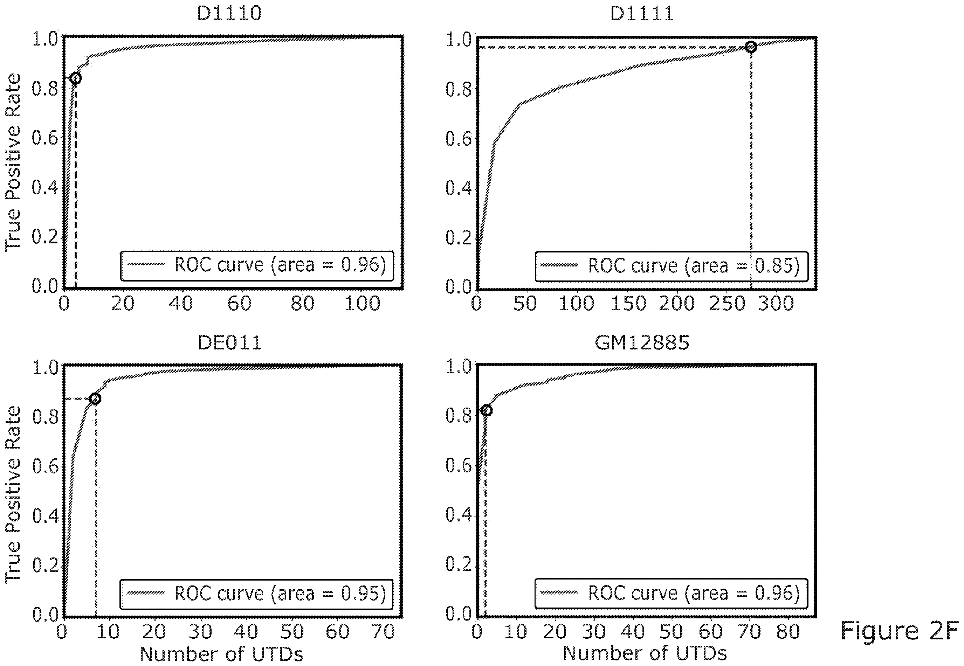

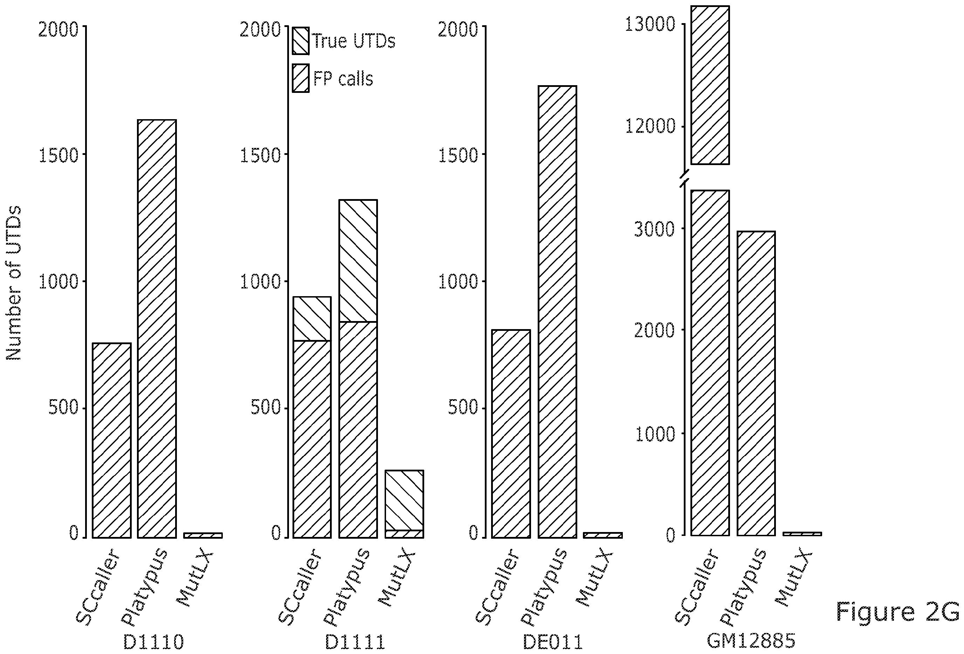

**Figure 2. MutLX algorithm, design and results. (A)** comparing the number of wells supporting various mutation types in run D1110 confirms that, as hypothesized, the majority of UTDs are present in only a few wells. Horizontal lines represent median. Boxes represent interquartile range. Whiskers show the range excluding outliers which are defined as being outside 1.5 times the interquartile range. **(B)** Similarly, the dual-allelic compartment rate of UTDs appears to be significantly higher when compared with true variants. This value was calculated by dividing the number of wells with co-presence of variant and reference alleles to the total number of wells with evidence for variant allele. **(C)** A diagram showing the main challenges in analyzing DigiPico data using ANNs. Each circle/star indicates one variant. Red lines show the behavior of the classification model. All variants above and/or to the left of the lines are predicted to be true variants by the model. The analysis of a sample without clone-specific variant would result in precise separation between real and artefactual mutations. In contrast, the analysis of a sample with true clone-specific mutations would result in a suboptimal model, which could lead to an over-fitting against true UTDs. This will enforce a model that removes all FP calls at the cost of losing nearly all clone-specific variants. **(D)** A diagram showing the two-step training process in MutLX. The first training step identifies some of the mislabeled true mutations (grey circles) among UTDs. All potentially mislabeled data points are temporarily removed from the analysis in the second training (colored in black) so that a better model is obtained for assigning a probability score to all mutations. Finally, combining the probability scores obtained from the model with the uncertainty estimate (as described in E) of these probability scores allows for effective elimination of FP calls while maintaining an excellent sensitivity for true clone-specific variants. **(E)** A diagram showing the test-time drop-out analysis to compute the uncertainty estimate of probability scores. Black neurons indicate the neurons that had been turned off during the drop-out analysis. Accepting only variants with a high probability score and a low uncertainty score should allow for elimination of FP variant calls. **(F)** The ROC curves of the output of MutLX analysis for runs D1110, D1111, DE011, and GM12885 are presented. Circles represent the default cut-off values determined by MutLX. **(G)** Bar plots representing the number of passed UTDs in the output of SCcaller, Platypus and MutLX. Since no true UTDs are expected to be present in runs D 1110, DE011, and GM12885 the number of UTDs in these runs represent the FP rate for each analysis method. Values for Platypus are based on DigiPico specific filtering criteria prior to the application of MutLX.

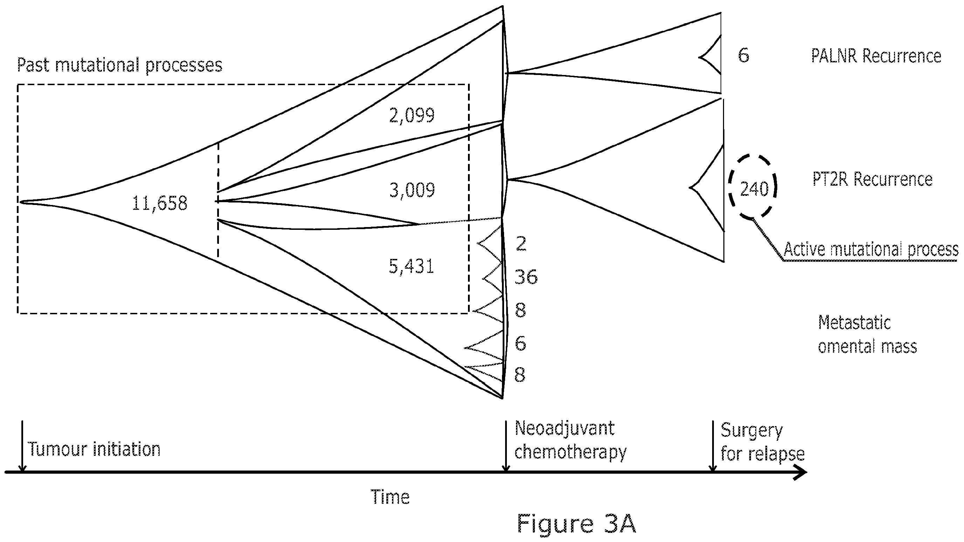

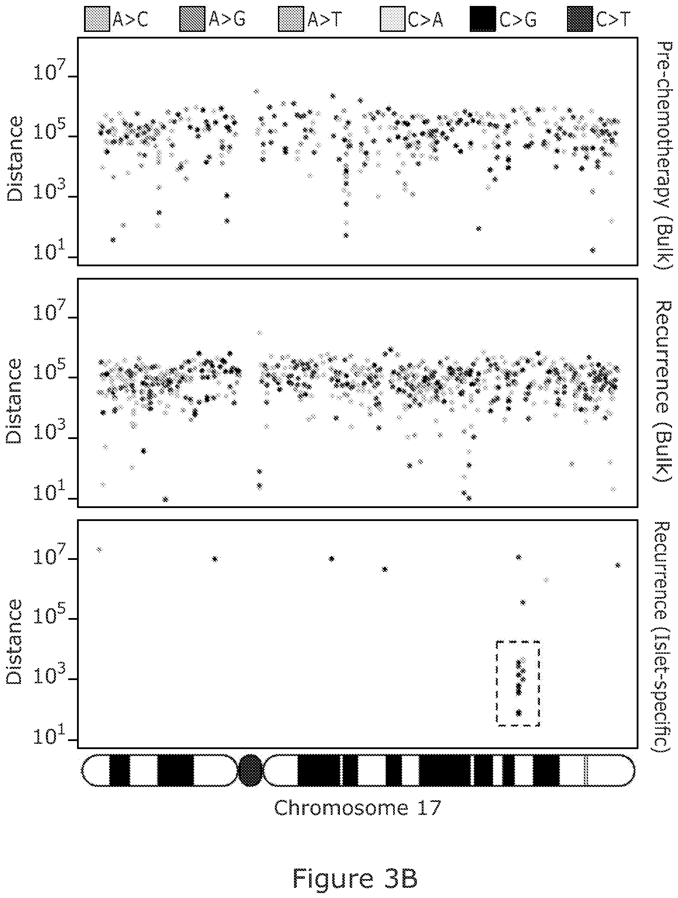

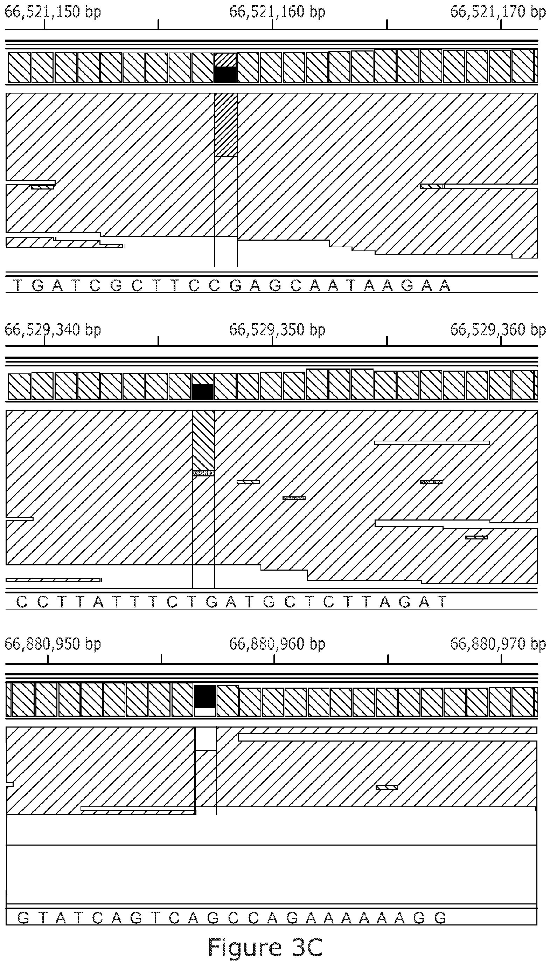

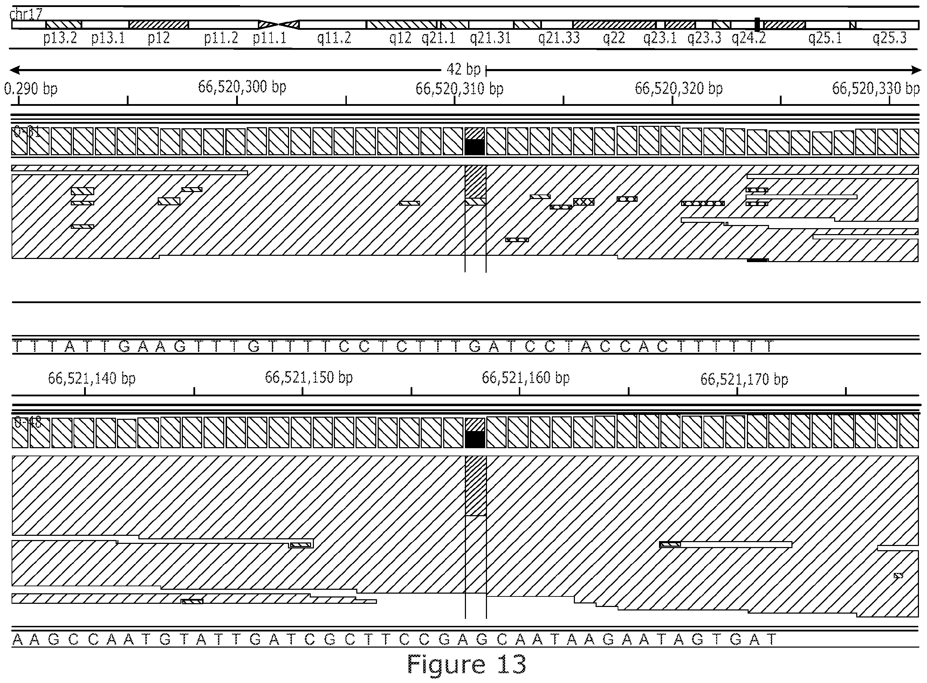

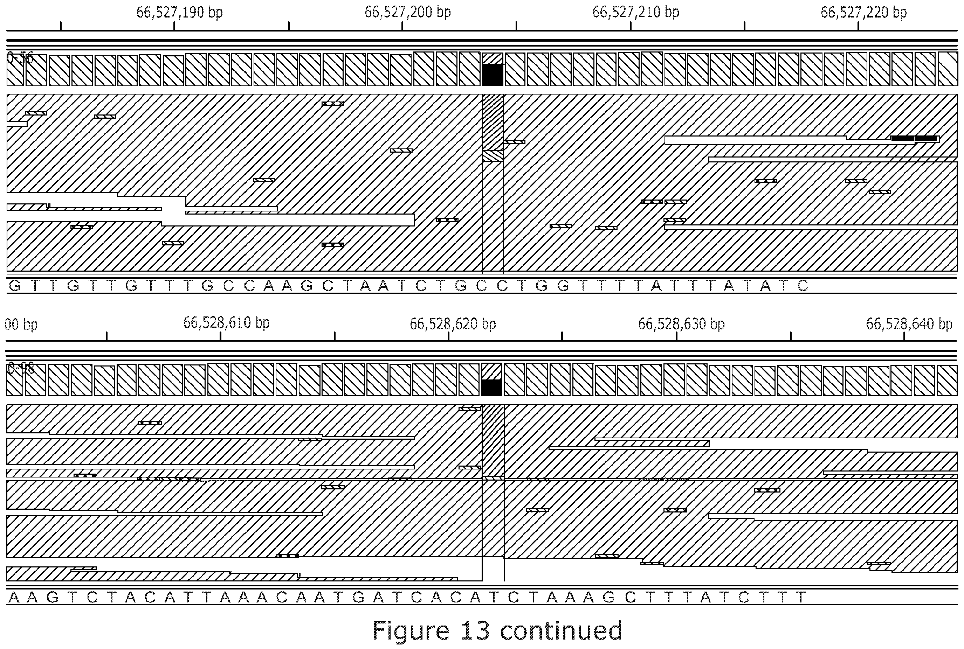

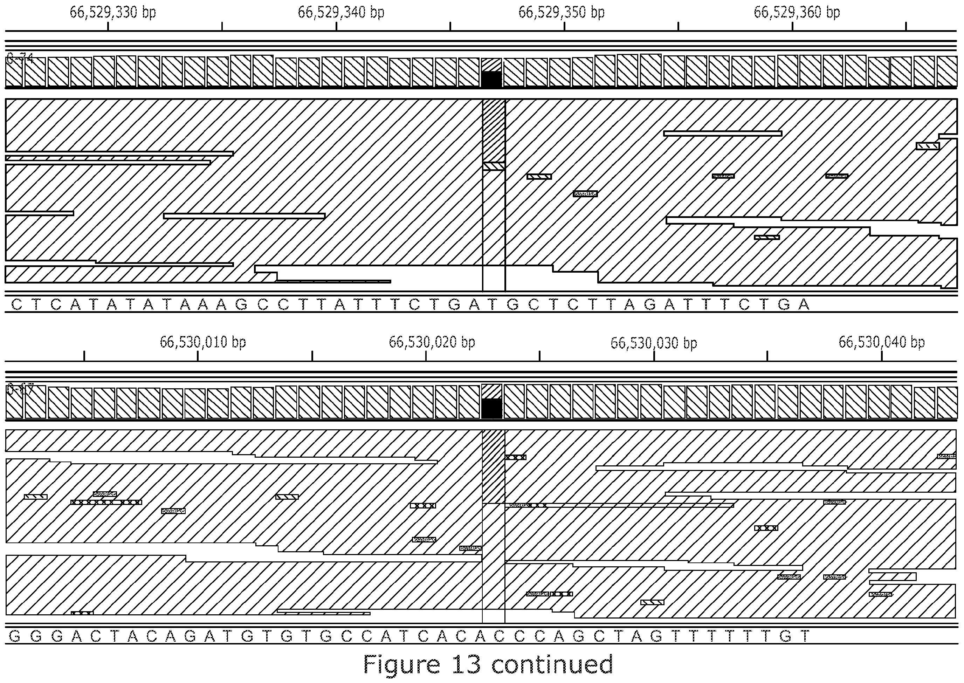

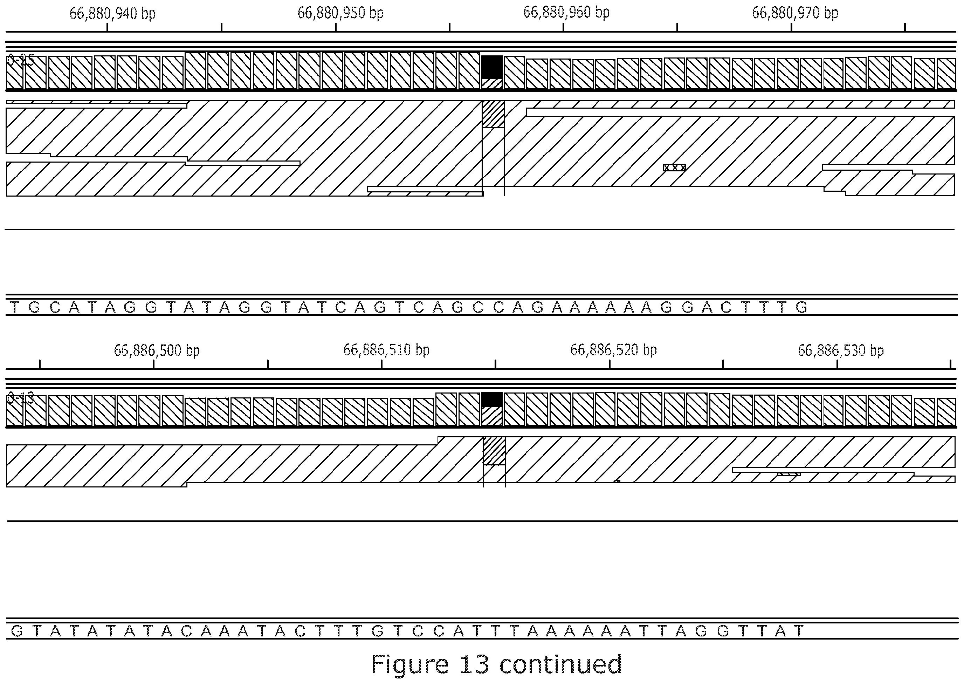

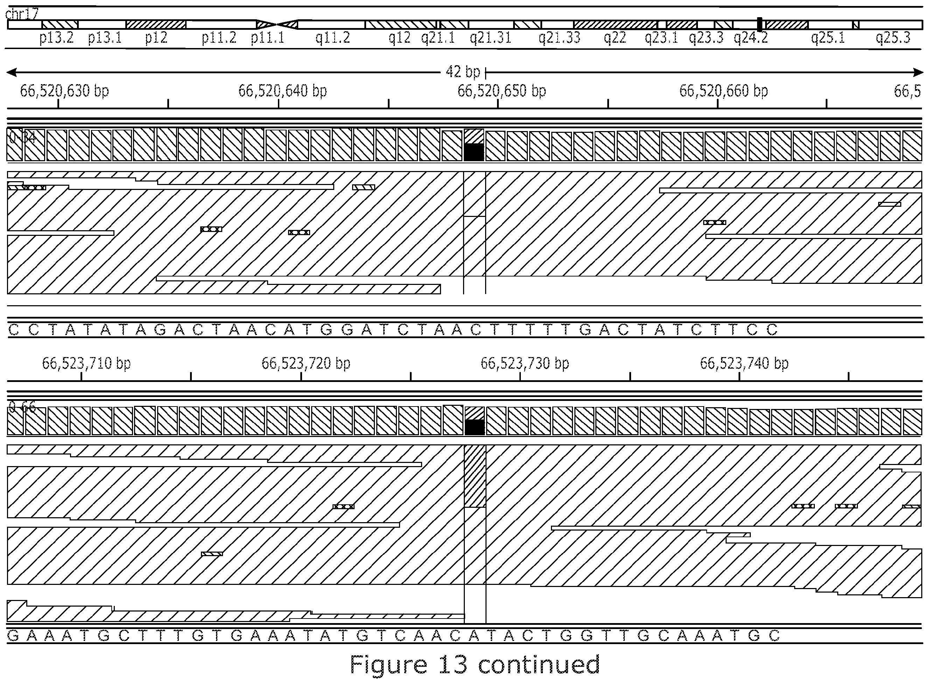

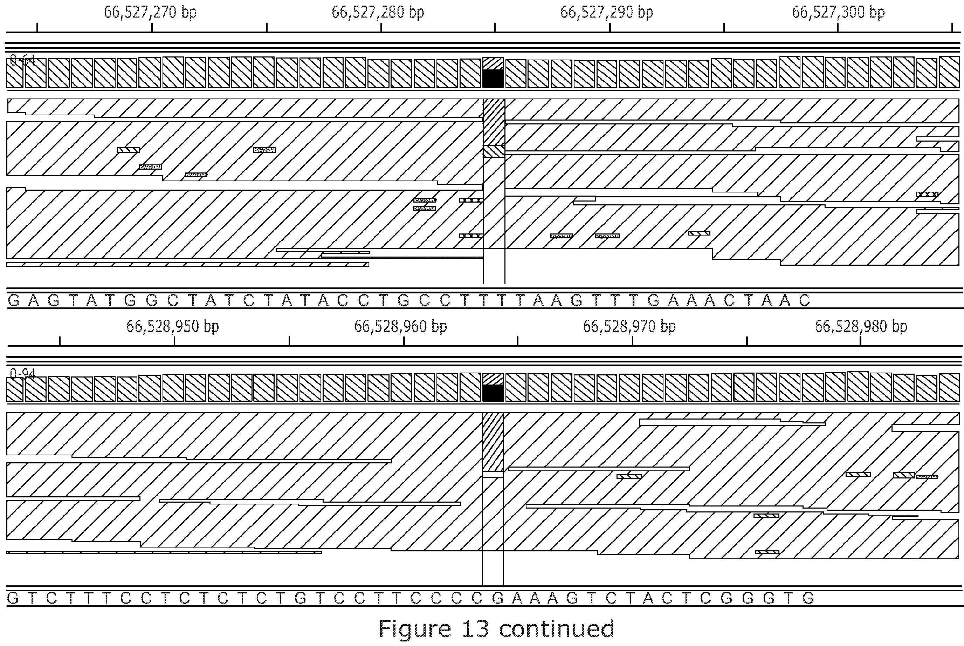

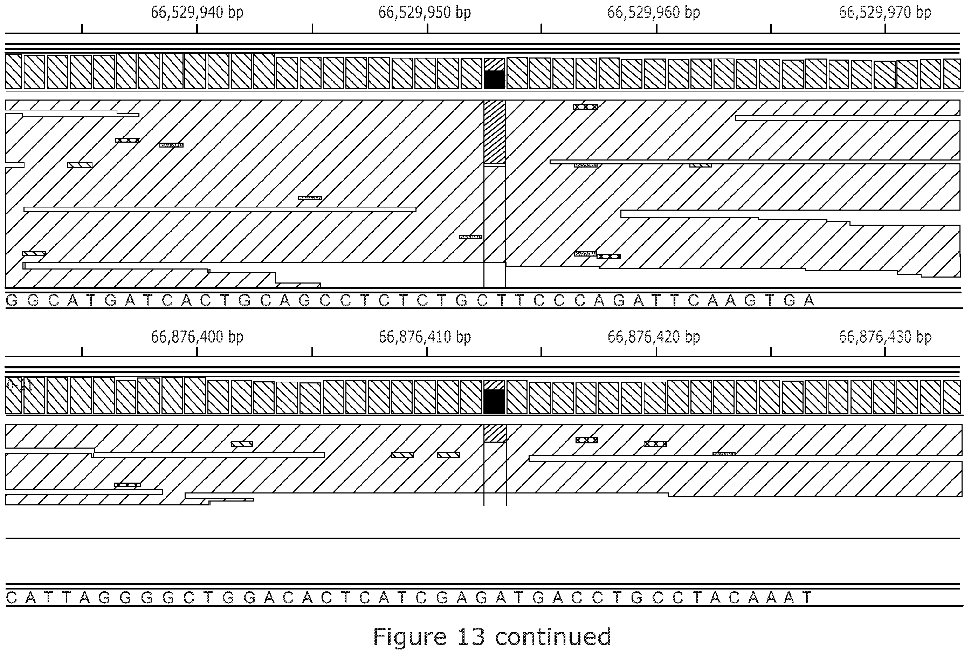

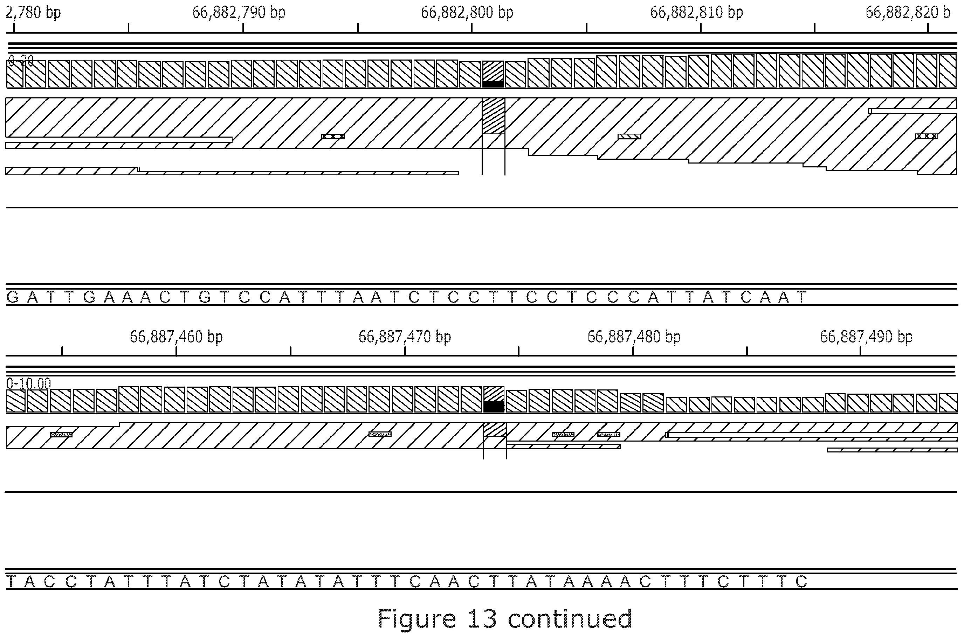

**Figure 3. Identification of an active mutational process using DigiPico/MutLX. (A)** schematic representation of tumor evolution in HGSOC patient #11152. Standard bulk WGS of various tumor samples identified ~ 11000 shared somatic mutations among all sites. Dotted purple line indicates the point at which the most recent common ancestor of the studied tumor samples has diverged. Bulk sequencing also identified nearly 5000, 3000, and 2000 sub-clonal mutations specific to the pre-chemotherapy omental mass, PT2R recurrence, and PALNR recurrence, respectively. These mutations however could have occurred anytime during the expansion of these clones and is biased towards older mutations. This is due to the limitations in identifying low-prevalence somatic mutations. DigiPico sequencing of five pre-chemotherapy tumor islets, PT2R and PALNR recurrence sites, however, identified various numbers of recently emerged clone-specific mutations in each of these samples (represented in red numbers). The significantly higher number of clone-specific variants in PT2R indicates the presence of an active mutational process. **(B)** This active mutational process is highlighted by the presence of a strong clone-specific kataegis event on chromosome 17 in run D1111. Y-axis represents the pair-wise distance of consecutive somatic mutations in log scale. Only mutations from chromosome 17 are shown. Mutations involved with the sub-clonal Kataegis event are highlighted in the box, nearly all of which are in the form of strand-specific C>T or C>G mutations. This suggests the involvement of APOBEC enzymes in this hyper-mutagenesis process. **(C)** Representative examples of some of the mutations involved in the kataegis. The presence of all the mutations on the forward strand of the genome further confirms the involvement of a hyper-mutagenesis event (Figure 13).

**Figure 4. Challenges in identifying recent mutations.** While old mutations can easily be studied from bulk sequencing data of the tumor, the study of recent mutations from such data is hampered because of the low variant allele fraction (VAF) of the involved mutations. Therefore, heuristic filtering criteria are not sufficient in identifying recent mutations. Reliable study of recent mutations requires the study of single cancer cells or tumor islets isolated via laser-capture micro-dissection (LCM) **(Figure 1).** However, WGA of the limited amount of template in such samples results in a large number of false positive variant calls that obstruct the identification of islet-specific variants. Our analysis pipeline, MutLX, can overcome this issue by eliminating FP variant calls from DigiPico sequencing data.

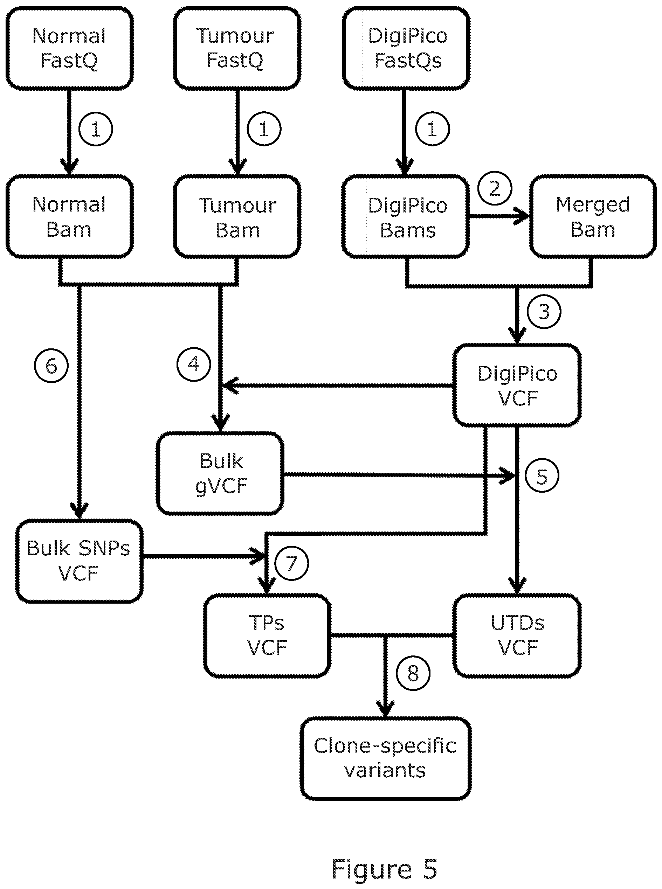

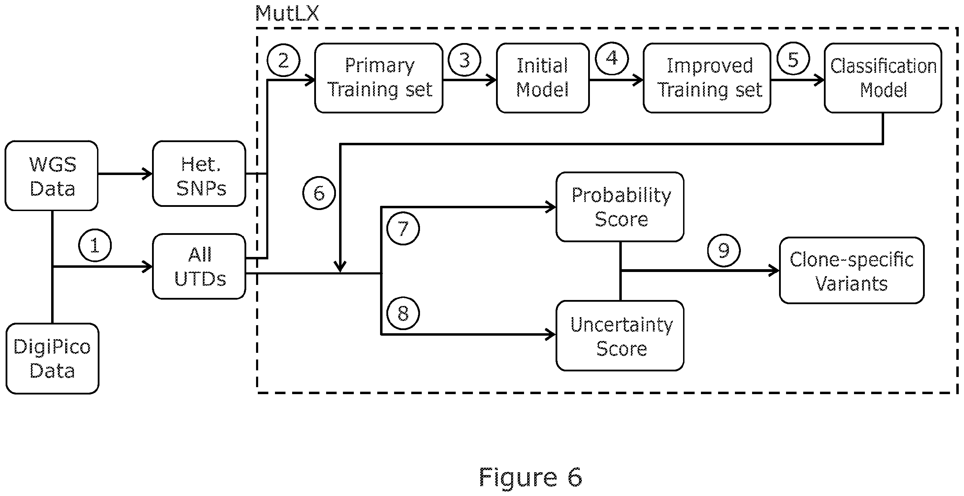

**Figure 5. Analysis workflow for DigiPico data. (1)** Next generation sequencing reads from normal tissue, bulk of the tumor, and DigiPico library are first mapped to human genome to generate bam files. DigiPico reads are divided into 384 FastQ files, one for each well of the 384-well plate. **(2)** The 384 individual bam files from DigiPico are merged into a single bam file. **(3)** *De novo* joint variant calling is performed on 384 individual bam files as well as the merged bam file using Platypus variant caller. Addition of the merged bam file ensures that variants that have a low per-well coverage will not be missed during variant calling. **(4)** The resulting *de novo* DigiPico variants are then used as a reference for variant re-calling from standard WGS data of the normal tissue and the bulk of the tumor. **(5)** The variant re-calling data can then be used to extract unique to DigiPico (UTD) variants by eliminating any variant that has supporting reads in the standard WGS data. **(6)** Standard WGS data are also used for variant calling using GATK to obtain a list of high-confidence germline SNPs. **(7)** This list will then be used as a guide to extract TP variant calls from DigiPico data. For this, any variant that was identified using GATK in the bulk blood sample and was also identified in the DigiPico data using Platypus was assumed real. **(8)** UTDs and germline SNPs are then used by MutLX to train a binary classification model for extraction of clone-specific variants from UTDs **(Figure 6).**

**Figure 6. MutLX analysis algorithm. (1)** UTD variants are identified by subtracting WGS data from DigiPico data. **(2)** UTDs and SNPs are used as training sets to train a primary binary classification model. **(3)** This model is used for primary analysis of the training set which allows for **(4)** generation of an improved training set. **(5)** The improved training set is then used to generate a classification model, which **(6)** can be used for analysis of UTDs. **(7)** A "probability score" indicating the likelihood for a mutation to be real, and **(8)** an "uncertainty score", as a measure of unreliability of the calculated probability scores, is calculated for each mutation using the model. **(9)** True variants are identified by a high probability score with high certainty (low uncertainty score).

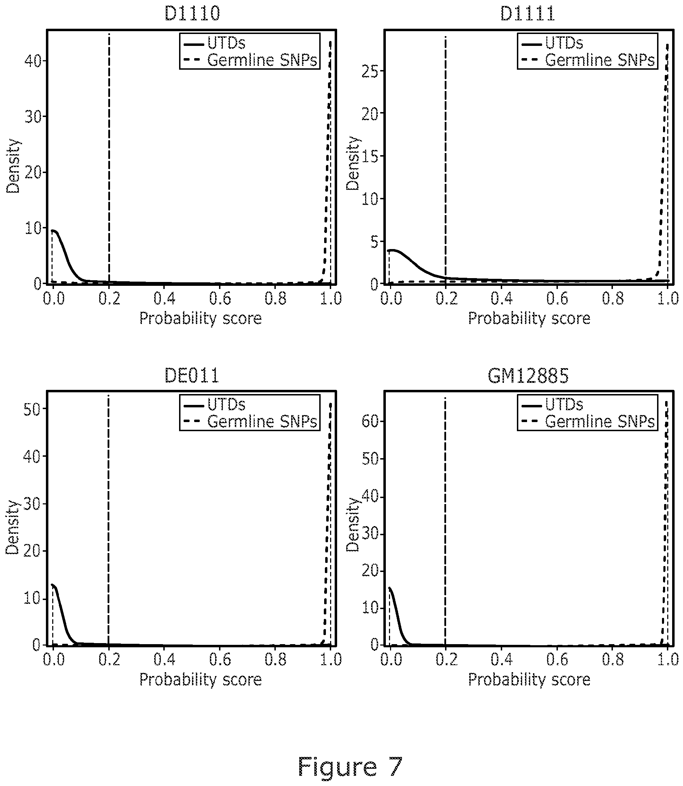

**Figure 7. Probability score values for runs D1110, D1111, DE011, and GM12885.** A cut-off value of 0.2 removes the majority of FP variant calls from UTDs while retaining nearly all germline SNPs in all samples.

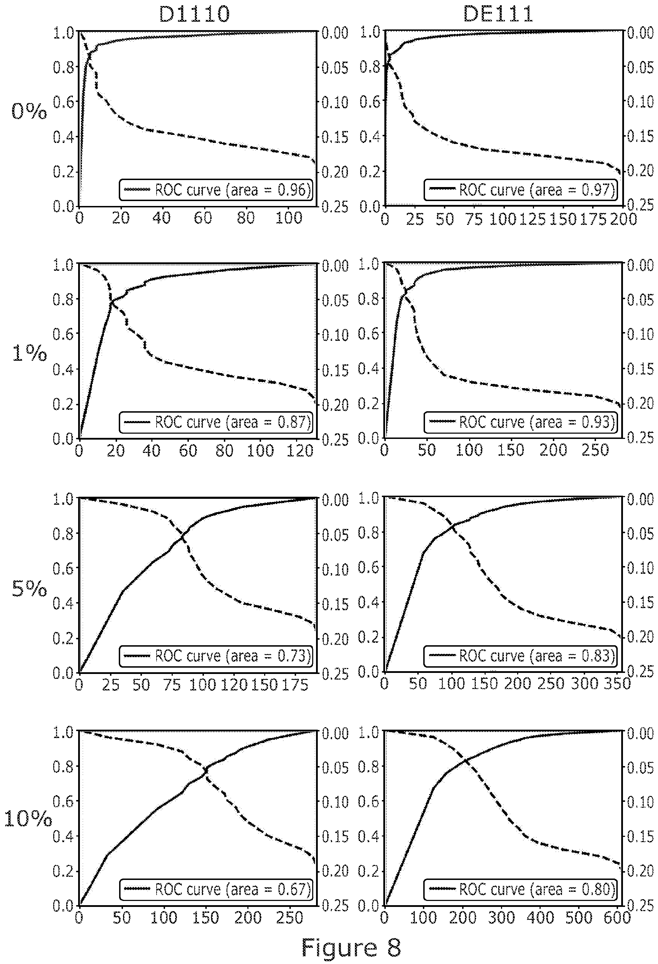

**Figure 8. Data simulation confirmed that the AUC negatively correlates with the number of true UTD variants.** Various number of somatic mutations were artificially mislabeled as UTDs (UTD*s) to achieve 1%, 5%, and 10% UTD*/UTD ratios in runs D1110 and DE111. Since both of these runs had been performed on 200 pg of purified DNA from bulk tumor samples, neither of them is expected to have true UTD variants. The independent analysis of each of these simulated datasets using MutLX confirmed the negative correlation between the number of true UTDs in the dataset and the AUC. Notably, it appears that a UTD*/UTD ratio of as little as 1% (16 and 36 variants in runs D1110 and DE11, respectively) can reduce the AUC suggesting that the presence of even a minor proportion of true clone-specific variants perturbs the ROC curve.

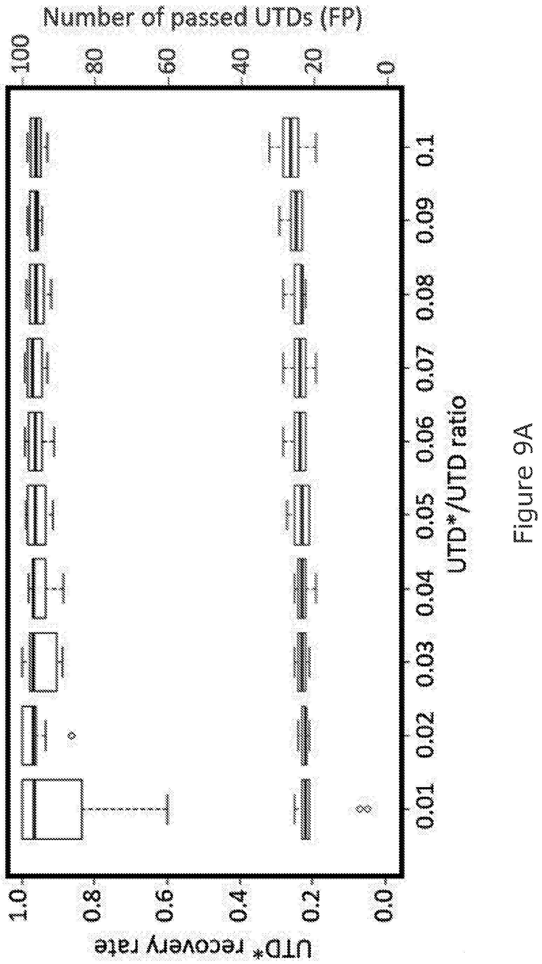

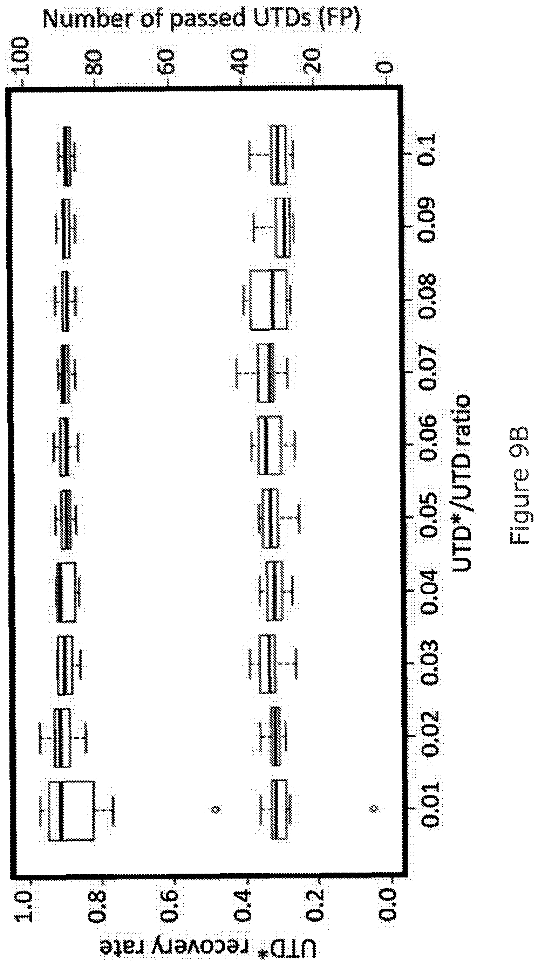

**Figure 9. Analysis of the synthetic DigiPico datasets.** Various numbers of high-confidence somatic mutations were artificially mislabeled as UTDs (UTD*s) to synthetically inflate the number of true UTDs at various UTD*/UTD ratios in runs **(A)** D1110 and **(B)** DE111. Since both of these runs had been performed on 200 pg of purified DNA from bulk tumor samples, neither of them is expected to have true UTD variants. Results indicate that presence of true UTD variants amongst a majority of artefactual UTDs does not appear to compromise the integrity of the classification models generated by MutLX. Each box plot shows the results from 10 different UTD* subsets used for the analysis. To achieve comparable FP rates between runs, cutoff values for analysis were used to achieve a TPR of 90% and 95% in germline variants for all synthetic datasets of runs D1110 **(A)** and DE111 **(B),** respectively. Boxplots show the median, interquartile range, and the range excluding outliers. Outliers are defined as being above or below 1.5 times the interquartile range.

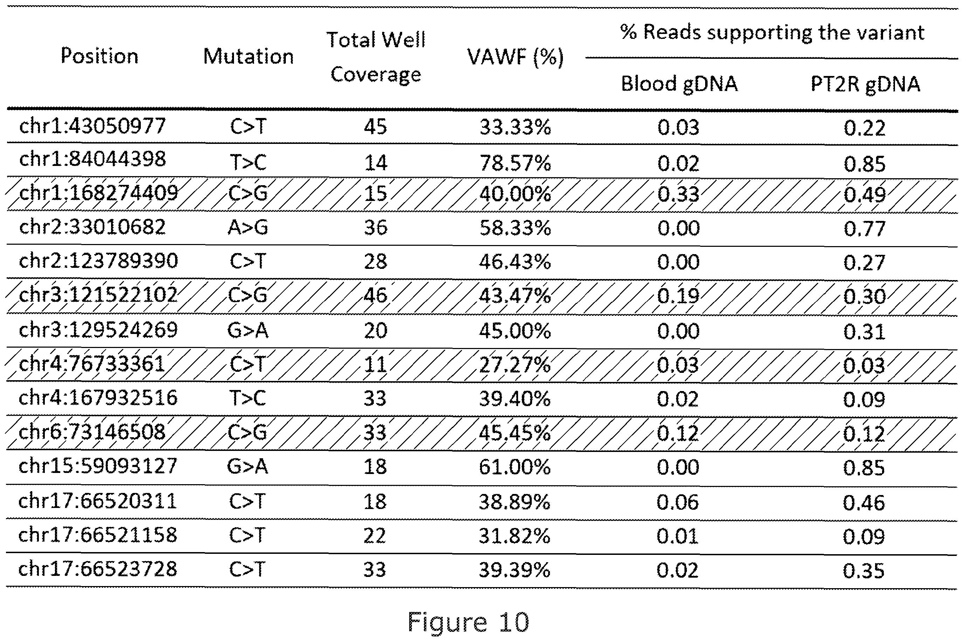

**Figure 10. Targeted sequencing of some of the clone-specific variants identified in run D1111.** Amplicon sequencing of the target sites was performed on the MiSeq platform. 3 out of the 14 targets appeared to have a high noise levels in the blood sample (highlighted in orange) and were therefore deemed inconclusive. Of the remaining 11 mutations only 1 did not seem to have any evidence in the bulk DNA sample of the PT2R tumour (highlighted in blue). VAWF: Variant Allele Well Fraction.

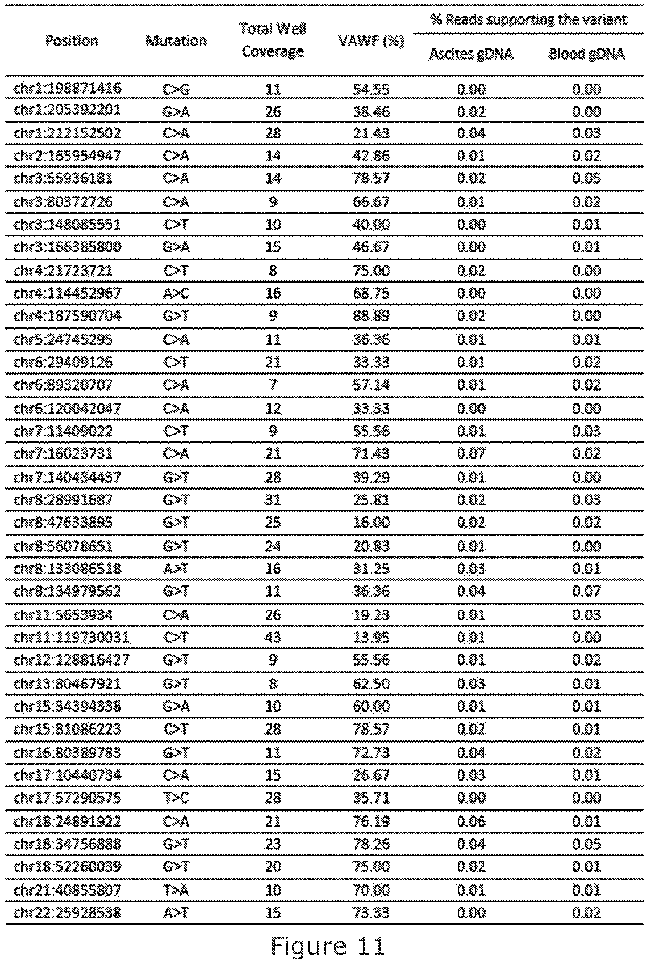

**Figure 11. Targeted sequencing of some of the artefactual variants identified in run DE111.**

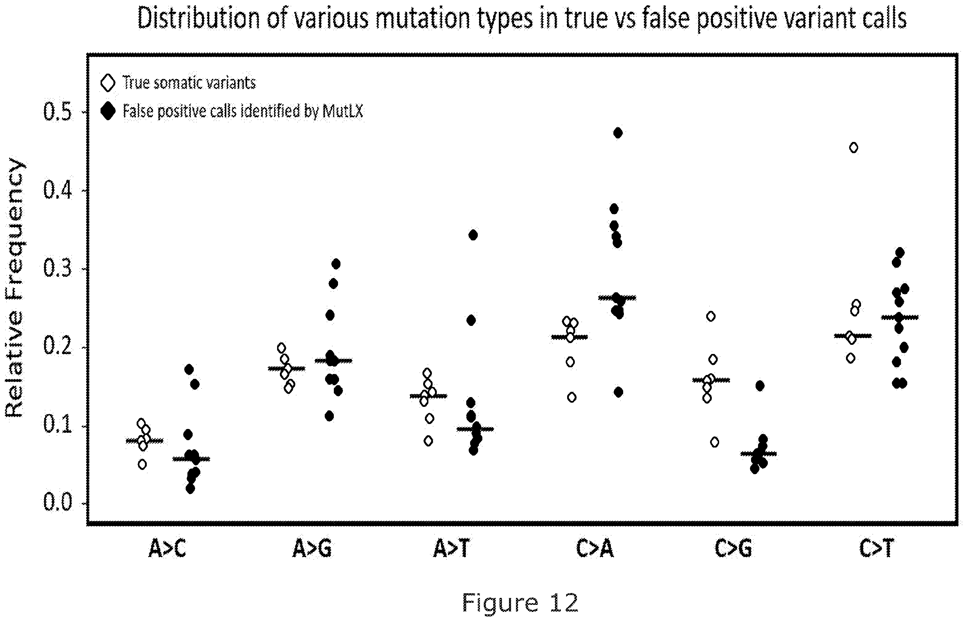

**Figure 12. Frequency of various mutation types among FP calls identified by MutLX in DigiPico data.** Green dots (on the left) are obtained from the analysis of somatic variants in standard WGS data from patients #11152, #11513, and OP 1036. Red dots (on the right) are obtained from all of artefactual mutations that were identified by MutLX from DigiPico data of the same patients. Black lines represent the median of the values in each set. The higher ratio of C>A mutations among the mutations that are eliminated by MutLX is in agreement with previous studies showing that oxidative DNA damage during library preparation results in formation of artefactual C>A mutations.

**Figure 13. IGV images of SNVs that identified in the sub-clonal kataegis in PT2R sample.** Note that nearly all mutations are in the form of C>T or C>G mutations on the forward strand of the genome.

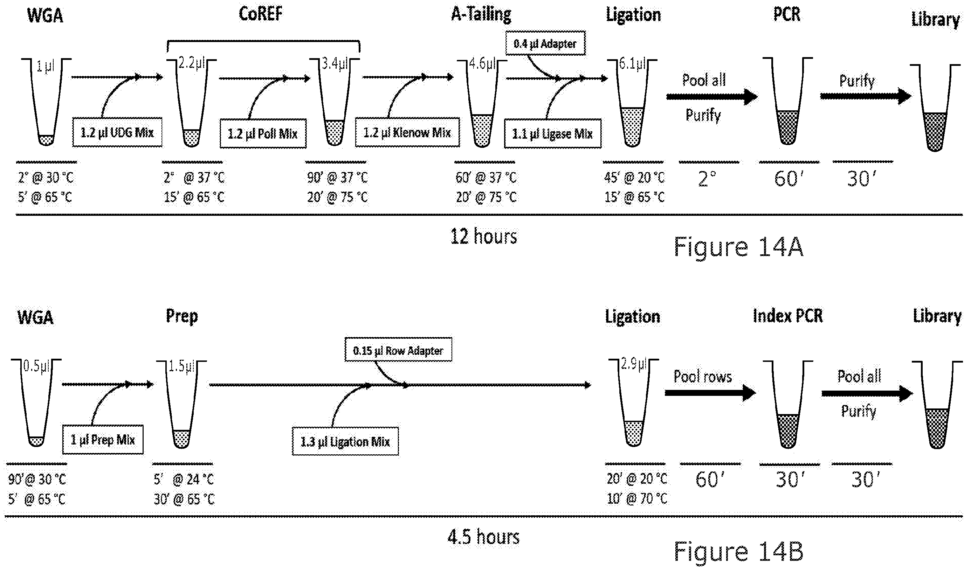

**Figure 14. Comparison of DigiPico and DigiPico2 workflows. (A)** DigiPico workflow takes nearly 12 hours and is composed of 7 steps, 5 of which occur in a 384 well plate. **(B)** DigiPico2 workflow takes only 4.5 hours and is composed of 5 steps, only 3 of which occur in a 384 well plate. Reactions in blue occur in 384 well plate format, green in 16 wells, and orange in 1 tube.

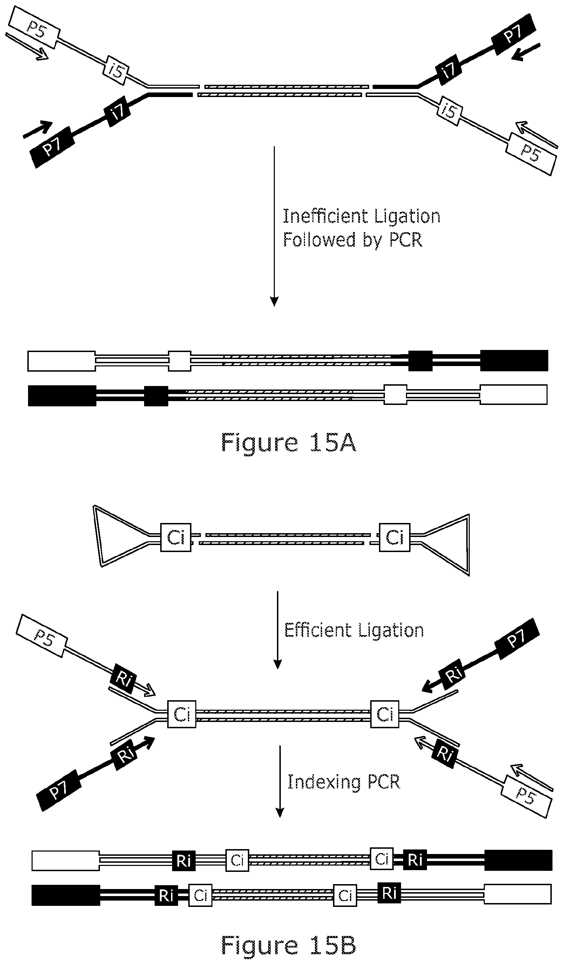

**Figure 15. Comparison of DigiPico and DigiPico2 indexing strategy. (A)** Asymmetric ligation of 2 annealed indexing oligos introduces one i5 index and one i7 index. Combination of these indices can produce 384 different indices in DigiPico. Not that each strand receives one i5 index and one i7 index so no redundancy is present. **(B)** In DigiPico2 initially column indices (Ci) are introduced through efficient ligation of looped adapters. Next, Indexing PCR is performed using row indexing primers (Ri). Note that each strand receives the Ci and Ri index twice each which introduces redundancy needed for removal of index cross-contaminants.

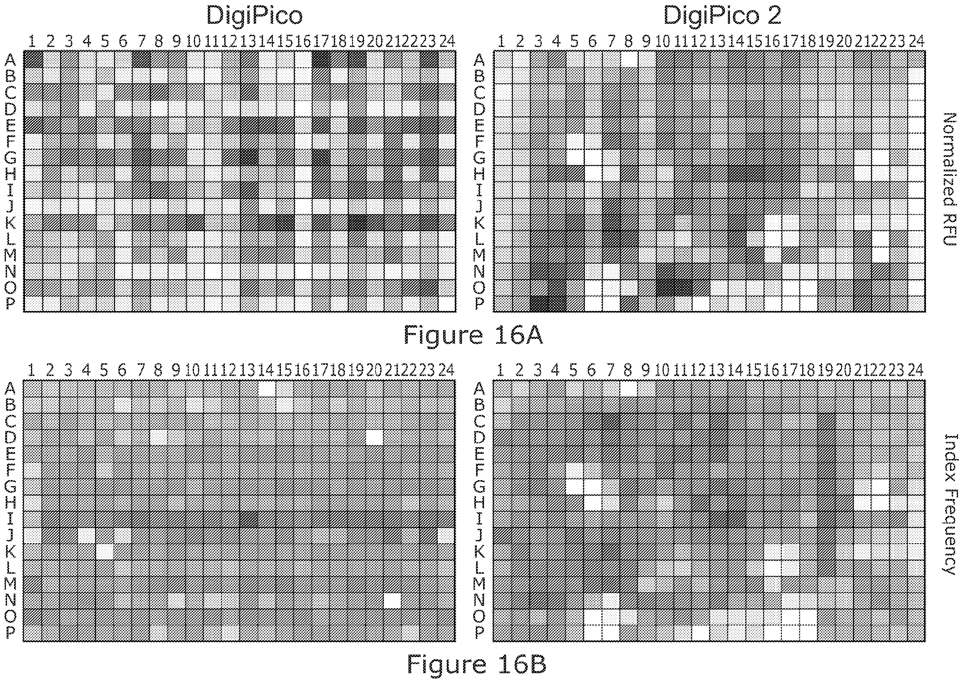

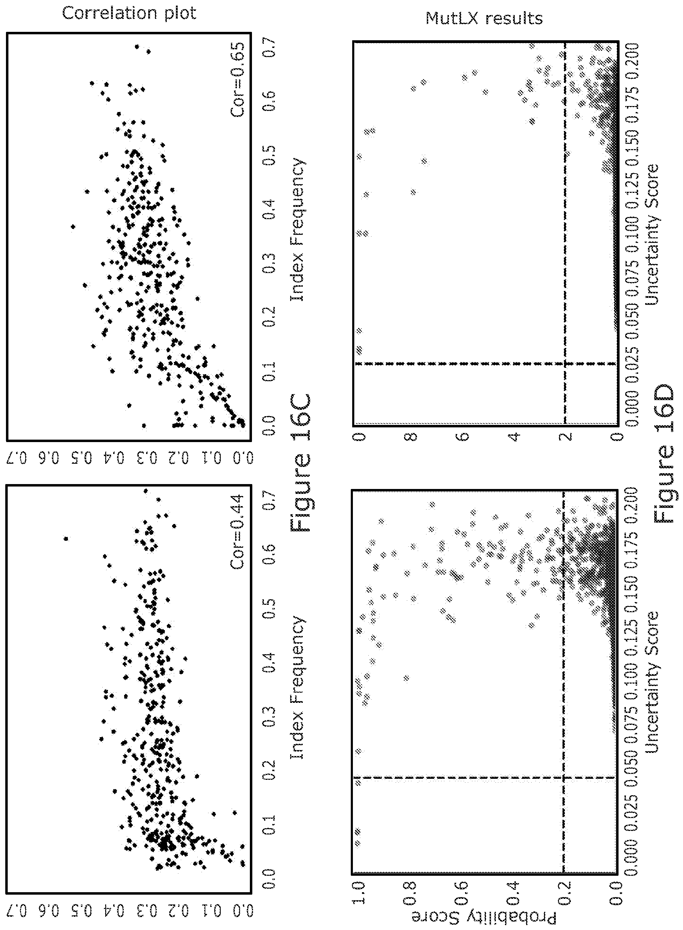

**Figure 16. Comparison of DigiPico and DigiPico2 results. (A)** Both WGA products appear to be relatively homogenous across the plates. Values represent the relative fluorescence from measuring EvaGreen (RFU). **(B)** The frequency of indices from each well across the plates appears to correlate better in DigiPico2 with the RFU values of WGA products. **(C)** This fact can be quantified using a correlation plot. **(D)** MutLX analysis of DigiPico2 data appear to result in a better distinction between true and artefactual mutations. Note the lower presence of mutations in the upper right section of plot. This area marks the artefactual mutations that have received a high probability score in the analysis.

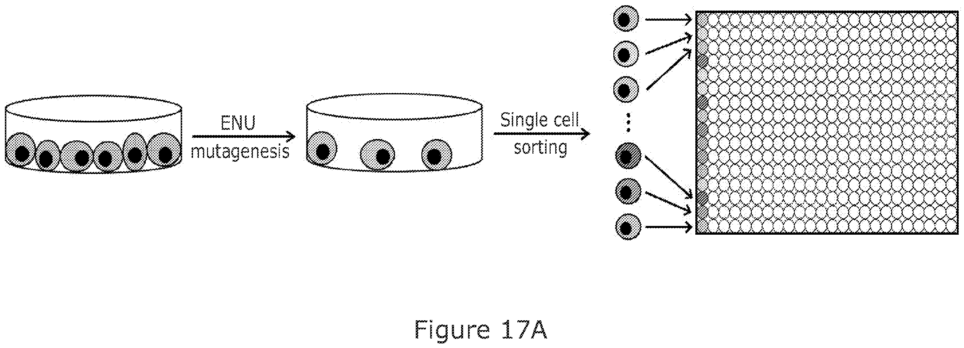

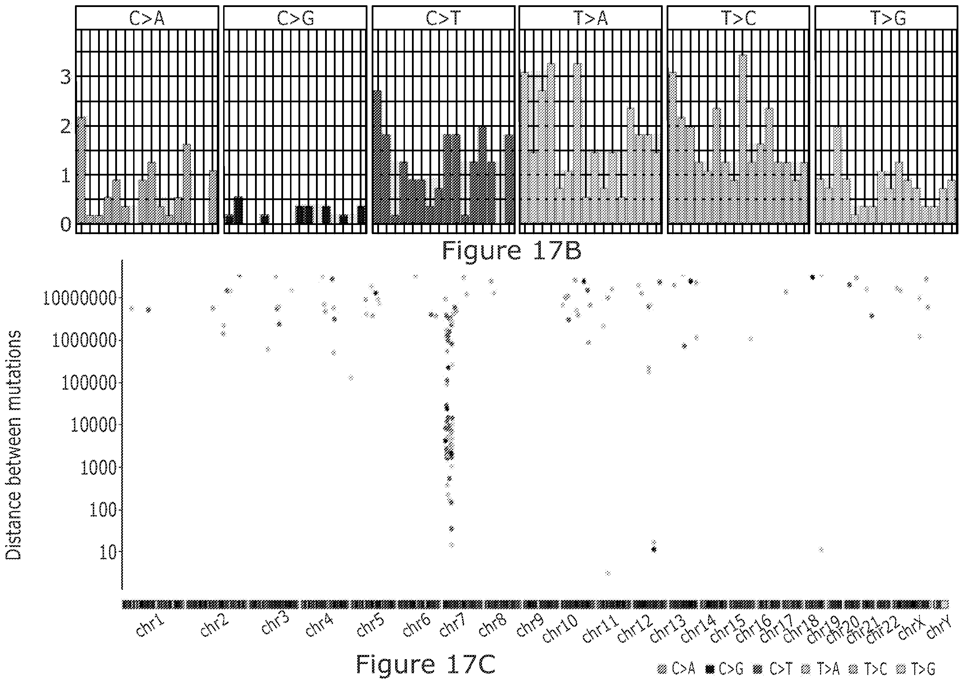

**Figure 17. Evaluation of Single Cell DigiPico (ScDigiPico) sequencing method.** To evaluate the efficacy of ScDigiPico method at identifying active mutational process, we simulated a such process by mutagenizing cultured Kuramochi cells using N-ethyl-N-nitrosourea (ENU). ENU is an alkylating agent and a very potent mutagen and preferentially causes T>C, T>A, and C>T mutations. In this setting, each cell is expected to obtain a different set of mutations but because the underlying mechanism of mutagenesis is the same the mutations are expected to be of the same type. **(A)** Cultured Kuramochi cells were exposed to 0.1 g/L ENU for 48 hours. Majority of cells die in the presence of ENU, but those that survive accumulate a large number of mutations. Single mutated Kuramochi cells are then sorted into every well of the first column in a 384-well plate. During ScDigiPico the DNA content from each cell is evenly distributed into all wells of the row before WGA and library preparation. **(B)** Analysing the results of ScDigiPico of mutated cells the frequency of novel mutations clearly matches with that of ENU, as expected. **(C)** In only one of the analysed cells following UV irradiation, we identified a clear Kataegis event on chromosome 7.

**Figure 18** - DigiPico/MutLX eliminate false positives from whole genome amplified DNA. Dots represent individual samples (~20 cancer cells) from a cancer patients or sequencing of whole genome amplified blood DNA (starting from picograms of blood DNA). Germline (Blood) DNA should not have thousands of unique variants when compared to standard DNA sequencing. However, existing methods find tens of thousands of such false positive mutations. In comparison, DigiPico/MutLX eliminates these false positives

**Example 1 - Revealing active mutational processes in tumours using DigiPico/MutLX at unprecedented accuracy**

**Summary**

Bulk whole genome sequencing (WGS) enables the analysis of tumour evolution but, because of depth limitations, can only identify old mutational events. The discovery of current mutational processes for predicting the tumour's evolutionary trajectory requires dense sequencing of individual clones or single cells. Such studies, however, are inherently problematic because of the discovery of excessive false positive mutations when sequencing picogram quantities of DNA. Data pooling to increase the confidence in the discovered mutations, moves the discovery back in the past to a common ancestor. Here we report a robust whole genome sequencing and analysis pipeline (DigiPico/MutLX) that virtually eliminates all false positive results while retaining an excellent proportion of true positives. Using our method, we identified, for the first time, a hyper-mutation (kataegis) event in a group of ~30 cancer cells from a recurrent ovarian carcinoma. This was unidentifiable from the bulk WGS data. Overall, we propose DigiPico/MutLX method as a powerful framework for the identification of clone-specific variants at an unprecedented accuracy.

## Introduction

In this work we developed a single DNA molecule WGA and sequencing approach to obtain high-quality and data-rich sequencing results from picogram quantities of DNA obtained from clinical samples (we termed DigiPico; for Digital sequencing of Picograms of DNA). Moreover, we implemented a complementary analysis workflow for DigiPico data using an artificial neural network (ANN)-based algorithm (MutLX, for Mutation Learn) to eliminate false positive results while maintaining excellent sensitivity for true positive mutations on a whole genome scale. We validate our approach using data from an extensively sequenced tumor from a single patient with a cumulative depth of ~4200x obtained from 45 whole genome sequencing runs on DNA from three different time points. We show the versatility of the methods by sequencing samples from 4 additional cancer patients and a lymphoblastoid cell line.

## MATERIAL AND METHODS

### Patient samples and consent

Patients #11152, #11502 and #11513 provided written consent for participation in the prospective biomarker validation study Gynaecological Oncology Targeted Therapy Study 01 (GO-Target-01) under research ethics approval number 11/SC/0014. Patient OP1036 participated in the prospective Oxford Ovarian Cancer Predict Chemotherapy Response Trial (OXO-PCR-01), under research ethics approval number 12/SC/0404. Necessary informed consents from study participants were obtained as appropriate. Blood samples were obtained on the day of surgery. Tumour samples were biopsied during laparoscopy or debulking surgery and were immediately frozen on dry ice. All samples were stored in clearly labelled cryovials in -80 °C freezers.

### Cell lines

GM12885 lymphoblastoid cell line (RRID:CVCL_5F01) was obtained from Coriell institute and cells were kept in culture as recommended by the provider.

### Sectioning and LCM

Frozen tumour samples were embedded in OCT (NEG-50, Richard-Allan Scientific) and 10 - 15 μm sections were taken using MB DynaSharp microtome blades (ThermoFisher Scientific) in a CryoStar cryostat microtome (ThermoFisher Scientific). Tumour sections were then transferred to PEN membrane glass slides (Zeiss) and were immediately stained on ice (2 minutes in 70% ethanol, 2 minutes in 1% Cresyl violet (Sigma-Aldrich) in 50% ethanol, followed by rinse in 100% ethanol. A PALM Laser Microdissection System (Zeiss) was used to catapult individual tumour islets into a 200 μl opaque AdhesiveCap (Zeiss).

### Standard WGS and data analysis

DNA was extracted using DNeasy blood and tissue kit (Qiagen). Up to 1 μg DNA was diluted in 50 μl of water for fragmentation using a Covaris S220 focused-ultrasonicator instrument to achieve 250 - 300 bp fragments. The resulting DNA fragments were then used for library preparation using NEBNext Ultra II library preparation kit (NEB), following the manufacturer's protocol. The resulting libraries were sequenced on Illumina NextSeq or HiSeq platforms at a depth of 30 - 40x over human genome. Sequencing reads in the FastQ format were initially trimmed using TrimGalore (14) and were then mapped to human hg19 genome using Bowtie2 (15). Germline variant calling was performed using GATK's HaplotypeCaller (16). Somatic variants were called using Strelka2 with a variant allele fraction cut-off of 0.2 (17).

### DigiPico Sequencing

200 pg of purified DNA, 20 - 30 resuspended nuclei, or laser-capture micro-dissected tumour islets, were first denatured using 5 μl of D2 buffer from Repli-g single cell kit (Qiagen). After 5 minutes incubation at room temperature, 95 μl of water was added to the sample and then using Mosquito HTS liquid handler (TTP Labtech) 200 nl of the denatured template was added to each well of a 384-well reaction plate already containing 800 nl of WGA mix (0.58 $\mu$l Sc Reaction Buffer, 0.04 $\mu$l Sc Polymerase (REPLI-g Single Cell kit, Qiagen), 0.075 $\mu$l 1 mM dUTP (Invitrogen), 0.04 $\mu$l EvaGreen 20x (Biotium), and 0.065 $\mu$l water). The plate was incubated at 30 °C for 2 hours followed by heat inactivation at 65 °C for 15 minutes. Addition of EvaGreen in the reaction allows for monitoring of the WGA reaction using a real-time PCR machine if required (18). Next, controlled enzymatic fragmentation (19) reaction steps were sequentially performed on the whole genome amplified DNA without any purification steps. Briefly, **(A)** 1200 nl of UDG mix (0.08 U/$\mu$l rSAP (NEB), 0.2 U/$\mu$l UDG (NEB), 0.4 U/$\mu$l EndoIV (NEB) in 1.8x NEBuffer 3) was added with 2 hours incubation at 37 °C and heat inactivation at 65 °C for 15 minutes. **(B)** 1200 nl of PolI mix (0.4 U/$\mu$l DNA Polymerase I (NEB) 0.25 mM dNTP, 8 mM MgC12, and 0.8 mM DTT) was added with 1.5 hours incubation at 37 °C and heat inactivation at 70 °C for 20 minutes. **(C)** 1200 nl of Klenow mix (0.5 U/$\mu$l Klenow exo- (NEB), 0.5 mM dATP, 8 mM MgC12, and 0.8 mM DTT) was added with 45 minutes incubation at 37 °C and heat inactivation at 70 °C for 20 minutes. **(D)** 400 nl of 20 $\mu$M full-length Illumina adapter oligos (Table S1) with well specific indices were added to each well followed by the addition of 1100 nl of Ligation mix (40 U/$\mu$l T4 DNA Ligase (NEB), 5 mM ATP, 11.5% PEG 8000 (Qiagen), and 6.8 mM MgC12) and with 30 minutes incubation at 20 °C and heat inactivation at 65 °C for 15 minutes.

The resulting products were then pooled and the DNA was precipitated using an equal volume of isopropanol. DNA was then resuspended in water and the products were dual-size selected using Agencourt AMPure XP SPRI magnetic beads (Beckmann coulter) with 0.45x bead ratio for the left selection and an additional 0.32x for the right selection. The purified DNA was then resuspended in water and was immediately used for limited-cycle PCR amplification using the P5 and P7 primer mix (Table S1). PCR was performed for 12 cycles with 10 seconds annealing at 55 °C and 45 seconds extension at 72 °C. Final products were bead purified at 0.9x ratio. The resulting libraries were then sequenced on Illumina sequencing platforms in 2x150 paired-end sequencing mode to achieve a depth of coverage of 30 - 40x over human genome. The additional processing steps required for the DigiPico library preparation, at present, adds nearly £250 to the total reagent costs.

**Analysis of DigiPico sequencing data**

The analysis pipeline of DigiPico sequencing data is presented in Supplementary Figure 5. Briefly, 384 paired-read FastQ files for each well were obtained after demultiplexing the Illumina sequencing data. FastQ files were trimmed for adapter sequences and quality (14). The first 12 nucleotides of each read were also removed. Trimmed reads were mapped to human hg19 reference genome using Bowtie2 (15) with the ignore-quals parameter activated and duplicate reads were marked using Picard Tools (20). Joint variant calling was then performed on all 384 individual bam files together with a merged bam file from all wells using Platypus variant caller (21). Next, all low-quality variants were removed by applying the quality filters (QUAL > 60, FR > 0.1, HP $\leq$ 4, QD > 10, and SbPval $\leq$ 0.95). Moreover, the total number of wells covering each locus (Tw) and the number of wells supporting each variant (Vw) were determined and the well count filters (Tw > 5, Vw > 2, and Vw/Tw > 0.1) were applied to only retain the high confidence loci for analysis. Lastly, all regions of the genome with bad mappability (22) were removed from the analysis using VCFtools (23). The resulting list of high confidence *de novo* DigiPico variants were then used to perform variant re-calling (genotyping) on WGS data from blood and bulk of the tumour using Platypus with minPosterior parameter set to 0 and minMapQual parameter set to 5. Any variant that was confidently unsupported in both of the standard WGS data was extracted as a UTD (Unique to DigiPico) variant. Any variant that was confidently also present in the bulk sequencing data of the blood sample (based on GATK analysis) was extracted as a TP (True Positive) variant (Figure 5).

**MutLX algorithm**

The MutLX analysis pipeline is summarised in Figure 6.

*Artificial neural network architecture*

The neural network model used in this study is a multilayer perceptron with an input layer consisting of N neurons (N=41) where N is the number of features used in each experiment and was implemented in Python3 using Keras (24). The model has two hidden layers with ReLU activations. We varied these numbers but did not see any significant improvement when using larger numbers of neurons. The last layer is a single output neuron with a sigmoid activation. The loss function is binary cross-entropy. For training, we applied a stochastic gradient descent optimization with momentum (Adam (25)) with a learning rate of 0.001, a batch-size of 8 and for 10 epochs. After 10 epochs we did not observe any additional improvement in performance.

*Features used for training*

The following features, extracted from the Platypus output of the DigiPico data, were used as the input of neural network model:

Platypus quality parameters: QUAL, BRF, FR, HP, HapScore, MGOF, MMLQ, MQ, QD, SbPval, NF, NR, TCF, and TCR (21).

Sequence context complexity: $F_{20}[1]$, $F_{20}[2]$, $F_{20}[3]$. Where $F_{20}[i]$ is the sum of the frequency of the $i$ most abundant nucleotides in the 10 bp sequence on either side of the variant position.

Read distribution data: $R_{merge}[ref+var]$, $R_{merge}[var]$, W[R[ref]>0s & R[var]=0], W[R[ref]>0][0/0], W[R[ref]>0][0/1], W[R[var]>0], W[R[var]>0][1/1], W[R[var]>0][0/1], W[R[var]>0 & R[ref]=0][0/1], W[R[ref]>0 & R[var]>0], W[R[ref]=0 & R[var]>0], W[R[ref]=0 & R[var]>1], W[R[ref]=0 & R[var]>2], W[R[ref]=0 & R[var]>3], W[R[ref]=0 & R[var]>4], W[R[ref]=0 & R[var]>5], $R_{max}[1][var]$, $R_{max}[2][var]$, $R_{max}[3][var]$, $R_{max}[1][ref+var]$, $R_{max}[2][ref+var]$, $R_{max}[3][ref+var]$, $Max_c + Max_r$, W[R[var]>0] - ($Max_c + Max_r$). Where $R_{merge}[x]$ indicates the total number of reads in the merged bam file supporting the allele x (ref indicates reference allele, var indicates variant allele). $W[i][j]$ indicates the number of wells matching criteria $i$ with reported genotype $j$, where indicated. Where in criteria $i$ the $R[x]$ indicates the number of reads in the specific well supporting allele $x$. $R_{max}[y][x]$ shows the number of reads in the well with the $y^{th}$ highest number of reads supporting allele x. Lastly, $Max_c$ is the number of variant supporting wells in the column with the highest number of wells supporting the variant allele and $Max_r$ is the number of variant supporting wells in the row with the highest number of wells supporting the variant allele.

*Training using MutLX*

For each DigiPico run, we consider a full training set as the collection of all UTD variants (labelled as 0) and heterozygous germline SNPs (labelled as 1). The number of UTD variants in this set is much smaller than heterozygous germline SNPs, making the set imbalanced. Therefore, in order to avoid bias towards a specific label in the training we create 25 different balanced training subsets for each DigiPico run. This is done so that each training subset is composed of all UTD variants and a randomly selected subset of heterozygous germline SNPs with a size equal to the number of UTD variants. As explained previously, the majority of UTD variants are FP variant calls with an unknown ratio of true clone-specific variants among them, hence making the 0 labels noisy. To perform two-step training considering these noisy labels, we employ the following strategy. After training an initial model on each balanced training subset, the resulting model is applied to the mutations in the full training set to obtain an initial probability value for each mutation. These probability values indicate the predicted probability of a mutation belonging to label 1 category. Hence, any 0 labelled mutation that attains a predicted probability value close to 1, is likely to be a mislabelled mutation. Therefore, to reduce the level of mislabelled data in the training set, all UTD variants with a probability value of more than 0.7 and all germline SNPs with a probability value of less than 0.3 are considered mislabelled and are removed from the training set. The cut-off values in this step were empirically determined by the analysis of various simulated datasets. In the end, following a similar sub-sampling strategy as in the initial training, a new model is trained on the remaining mutations of the training set. This model is then used for the analysis of all UTD variants.

*Calculation of probability and uncertainty scores*

As explained earlier, in MutLX the training process is repeated 25 times with different randomly selected germline SNP subsets, resulting in different models each time and hence 25 different predicted probability values for each mutation. We therefore defined the "probability score" for each mutation as the average of all of its predicted probability values:

$$\text{Probability score} = \frac{\sum_{i=1}^{n} P_i}{n}$$

Where $P_i$ is the probability value obtained from the $i^{th}$ training subset and n represents the number of subsets (n=25).

Moreover, to obtain an uncertainty estimation for each probability value we performed a test-time drop-out analysis (26). The trained model was applied to each mutation for 100 iterations during which, different neurons were dropped out with a rate of 0.8 and 0.7 for the first and the second hidden layers of the neural network, respectively. This process resulted in 100 probability values for each mutation. Based on these values, we defined the "uncertainty score" for each mutation as the average of the dropout variances from the 25 different subsets:

$$\text{Uncertainty score} = \frac{\sum_{i=1}^{n} \sigma_i^2}{n}$$

Where $\sigma_i^2$ is the variance of 100 probability values obtained from the dropout analysis of the $i^{th}$ training subset and n represents the number of subsets (n=25).

The uncertainty scores of all variants with a probability score above 0.2 (Figure 7) was used to generate a putative receiver operating characteristic (ROC) curve. The curve was generated by considering a range of cut-off values between 0.0 and 0.25 for the uncertainty score. At each cut-off value the ratio of germline SNPs that have an uncertainty score below the cut-off value was plotted against the corresponding number of UTDs. The area under the curve (AUC) was then calculated after normalizing the number of UTDs between 0 and 1. Note that in cases where true clone-specific variants are not expected (all UTDs are FP calls), this plot represents a ROC curve and the AUC of this plot should be close to 1, assuming a perfect model. In contrast, in when the AUC is significantly lower, it indicates the presence of true clone-specific variants in the sample. This negative correlation between the number of true UTDs and AUC was validated using simulated datasets (Figure 8). Based on these observations, for samples where the ROC curve suggests the presence of true clone-specific variants (AUC<0.9) MutLX uses "uncertainty score" cut-off values that result in a TPR of 95% to improve the recovery rate of clone-specific variants. For datasets with an AUC≥0.9 the cut-off value for filtering the data was determined based on the intersection of the threshold curve and the ROC curve.

**Generation and analysis of simulated DigiPico datasets**

Simulated data were used to: (a) validate the negative correlation between the number of true UTDs and AUC (Figure 8) and (b) ensure that over-fitting to potentially true clone-specific variants does not occur (Figure 9).

To generate simulated datasets, we first identified somatic mutations in the bulk WGS data of the tumour sample PT2R from patient #11152 using Strelka2 somatic variant caller. These somatic variants were then identified in the de novo variant calling data of run D1110 and any somatic variant with a Tw>6 and Vw/Tw>0.45 was selected as a high-confidence somatic variant. Next, various numbers of randomly selected high-confidence somatic variants were artificially mislabelled as UTDs (UTD*) to achieve 0.01, 0.02, 0.03, 0.04, 0.05, 0.06, 0.07, 0.08, 0.09, and 0.1 UTD*/UTD ratios. The resulting synthetic list of variants were then independently used for MutLX analysis and the number of UTDs and UTD*s that passed the MutLX filtering were calculated for each run. To ensure a robust analysis, for each ratio 10 different subsets of the somatic variants were analysed. A similar analysis was also performed on the DigiPico data DE111, obtained from the bulk DNA extraction from an ascites sample of patient # 11513.

**Validation of MutLX algorithm**

Tumour sample PT2R from patient #11152 was used for the validation of the MutLX algorithm. A small piece of the tumour was macro-dissected from a frozen specimen and was embedded in OCT medium for sectioning. The first section (15 $\mu$m) from the tumour was collected in a separate tube and the nuclei were resuspended in 50 $\mu$l of sterile PBS solution. Total number of nuclei in the suspension was measured and a volume containing 30 nuclei was used for direct denaturation with an equal volume of D2 buffer from Repli-g mini WGA kit (Qiagen). The resulting crude lysate was directly used for DigiPico library preparation for run D1111. The remainder of the tumour sample was then used for bulk DNA extraction using DNeasy blood and tissue kit (Qiagen). 200 pg of the resulting DNA was directly used to prepare DigiPico library D1110. 1 $\mu$g of the DNA was used for standard library preparation using NEBNext Ultra DNA library preparation kit (NEB). In this setting only run D1111 is expected to have true clone-specific variants. Since the template for run D1110 is a subset of the template used in the bulk WGS analysis, nearly all real variants in run D1110 will also be present in the WGS data at similar frequencies and therefore will not be identified as UTDs. A similar logic is also applicable to the results of DigiPico runs DE011 and GM12885. Since both of these DigiPico runs had been performed on 200 pg of DNA from bulk DNA extractions, no true UTD variants are expected to be present in these samples. It is also worth noting that because of the digitized nature of the data, variants with very low frequencies (<0.05%) will show an inflated variant allele fraction in runs D1110, DE011, and GM12885, however, since such variants are unlikely to appear in more than one well, they will be eliminated from the data based on the Vw filter. Therefore, it is safe to assume that nearly all UTD variants in these runs are FP calls.

**Application of SCcaller on DigiPico data**

SCcaller has originally been developed for the analysis of multiple displacement amplified single cell sequencing data (11). Since DigiPico library preparation also requires multiple displacement amplification on limiting amounts of template DNA, the resulting data are fundamentally similar to the natural input for SCcaller. Therefore, we used the merged bam file of DigiPico data as an input for SCcaller. For the analysis, the list of heterozygous SNPs was obtained from the respective bulk WGS data using GATK HaplotypeCaller and cut-off values were used for alpha=0.01. Next, all filtered SNVs were used for variant re-calling on respective standard WGS data and all variants that were confidently unsupported by WGS data were extracted as UTD variants.

**Mutation validation**

Variants that pass the MutLX analysis were validated by comparison with deep sequencing data of the bulk tumour from an independent sequencing platform. All DigiPico data from patient #11152 were validated through comparison with 39 deep sequencing datasets obtained from the same tumour masses sequenced on Complete Genomics sequencing platform (27). This included three Complete Genomics bulk sequencing and 36 LFR (Long-Fragment Read) sequencing data. Since the independent sequencing data for the omental mass were not obtained from exactly the same tumour mass as the ones that were used for DigiPico sequencing the validation rate by such a comparison for these runs is not expected to be high.

For targeted validation, primers were designed to obtain amplicons containing the variants using the primer3 tool (Table S1). Amplicons were obtained by performing a 2-step PCR using Phusion® High-Fidelity PCR Master Mix with GC Buffer for 16 cycles on 1 ng of template. All amplicons from each sample wer then pooled and purified before adapter ligation and indexing using NEBNext Ultra II kit. The resulting libraries were sequenced on a MiSeq platform. Sequencing results were mapped to human hg19 genome using Bowtie2 and the number of reads supporting each variant was counted using Platypus variant caller.

**Local hyper-mutation (kataegis) analysis**

To generate the rainfall plots, the distances between pairs of consecutive somatic mutations on chromosome 17 were plotted against their genomic position of the second mutation in each pair using a custom script in R. The presence of clusters of localized mutations indicates kataegis events. In these plots, each dot is coloured based on the mutation type of the second mutation in the pair in respect to the hg19 human reference genome.

**RESULTS**

**Implementation of DigiPico sequencing approach**

A key feature of amplification errors with or without prior DNA damage is that they are introduced at random during the amplification process (6, 7, 28). We, therefore, hypothesized that when amplifying and sequencing a single DNA molecule an artefactual mutation would be present in only a fraction of the reads that have resulted from sequencing the original single DNA molecule. In contrast, genuine variants would be expected to be present in all such reads. Partitioning of the template DNA into individual compartments prior to WGA, such that each compartment receives no more than one DNA molecule from every locus would result in such single DNA molecule sequencing data (Figure 1B). Since the artefactual mutations would result in compartments with reads supporting multiple alleles this approach enables the identification of these artefacts and allows for the elimination of FP variant calls. In addition, such partitioning approach also results in the independent progression of WGA reactions for each locus. Thus, providing multiple internal replication data for the WGA process. While genuine variants are expected to be regularly present across replicates, the artefactual mutations, because of their stochastic nature, will likely have a limited presence in a small number of compartments. Consequently, taking both of these points into account, such a WGA and sequencing approach can result in distinctive distribution patterns across the compartments for artefactual mutations compared to real mutations. Since ANNs have shown to be capable of extracting complex patterns from high-dimensional inputs, they make a good candidate for identifying and eliminating false positive mutations from this type of data. While previous partitioning and sequencing methods have been described to obtain haplotype information, there is no such method for distinguishing true mutations from artefactual mutations (19, 29, 30).

To fully benefit from the data-richness of a partitioning and sequencing approach for accurate genomics study of clinical samples, we developed DigiPico sequencing (Figure 1C). To perform DigiPico sequencing, first, we uniformly distribute nearly 200 pg of DNA (obtained from 20 - 30 human cells) into individual wells of a 384-well plate. This ensures that the likelihood for the co-presence of two different DNA molecules from the same locus in the same well is less than 10% (19). Following WGA, each well is then processed independently into indexed libraries, each receiving a unique barcode sequence, prior to pooling and sequencing (Figure 1C). Since, in our approach, the key distinguishing factor for artefactual mutations lies in their peculiar distribution pattern, homogeneous distribution and amplification of DNA molecules as well as consistent depth of sequencing coverages across the wells would be critical. Achieving this homogeneity ensures that the differences in the distribution pattern of true and artefactual mutations are maximized. To ensure that the required homogeneity is achieved, during every DigiPico library preparation we monitored the WGA reactions' progress and quantified the final outcome for all the wells. The former was achieved by adding EvaGreen dye to the WGA reactions and monitoring the fluorescent intensity every 5 minutes in real-time. EvaGreen is an intercalating dye that binds to the minor groove of the DNA and therefore, does not interfere with the isothermal WGA reactions (18). For the latter, we introduced a per-well qPCR step to measure the relative number of adapter-ligated fragments in each well using adapter specific primers prior to pooling. Only libraries that passed both of these homogeneity tests were used for sequencing (Figs. 1D and 1E). Importantly, we also miniaturized the WGA reaction volumes to 1 μl. This could only be achieved after the identification of a compatible multiple displacement amplification (MDA) approach. Comparing six different MDA strategies, REPLI-g Single Cell amplification was the only method that met the required sensitivity and selectivity for our purpose (Figure 1F). Reaction miniaturization allowed us to streamline the library preparation process in a single 384-well plate without the need for intermediate purification steps using readily available automated pipetting instruments. Finally, we aimed to optimize the DigiPico library preparation process for frozen clinical samples. This was achieved by performing the WGA reaction directly on the crude lysate of small groups of neighbouring cells (tumour islets) isolated via LCM (laser-capture micro-dissection). This strategy ensured the minimal loss of genomic material while minimizing the manipulation time and thereby, reducing the chance of template oxidation.

**DigiPico sequencing platform generates high quality libraries from limited clinical samples**

Having optimized all the necessary aspects of DigiPico library preparation process, we decided to assess the quality of DigiPico libraries obtained from clinical samples. For this purpose, we prepared DigiPico libraries D1110 and D1111 from a frozen recurrent tumour sample (PT2R) obtained from a high-grade serous ovarian cancer patient (#11152). In this experiment, while D1110 library was prepared from 200 pg of template taken from a bulk DNA extraction of the PT2R sample, the D1111 library preparation was directly performed on a small frozen section of the remainder of this tumour sample (containing nearly 30 cancer cells). Each library was sequenced on an Illumina NextSeq platform to obtain nearly 400,000,000 reads in 150 x 2 paired-end format. The initial assessment of the obtained sequencing data revealed that both the D1110 and D1111 libraries have resulted in high quality sequencing data with an overall mapping rate of 91.35% and 94.27% on human hg19 genome, respectively (Figure 1G). Analysing the homogeneity of distribution across the plates indicated that in runs D1110 and D1111, on average, each well covers nearly 4.4% and 4.6% of the genome with an average depth of 1.7x and 2.1x in each well, respectively, with an outstanding homogeneity across the plates (Figs. 1H and 1I). This cumulatively resulted in a breadth of coverage of 92.1% and 91.1% with a depth of 30x and 43x for each run, respectively. These results confirmed that DigiPico sequencing can be used to produce high-quality sequencing data with excellent coverage from limited amount of frozen clinical samples.

Lastly, we assessed whether our initial hypotheses regarding the distinctive distribution pattern of different mutation types hold true in actual DigiPico datasets. For this purpose, we assumed that any variant that is shared between a DigiPico dataset and the standard bulk sequencing data of the same tumour sample must be a true variant. These should mainly consist of germline SNPs and clonal somatic variants. As a result, by definition, all FP variant calls and the majority of clone-specific mutations (had they existed in the sample under study) will be among variants that are only present in the DigiPico data and not in the bulk WGS data. These variants are referred to as UTD (Unique to DigiPico) for simplicity, hereafter. Consequently, given that the standard bulk sequencing data of the PT2R sample had been obtained from the same DNA extract that was used for D1110 library preparation, nearly all the UTD variants in D1110 DigiPico run ought to be artefacts (Figure 1J). To the contrary, the UTDs in run D1111 are likely to contain some clone-specific mutations alongside the artefactual mutations (Figure 1J). Therefore, we used the UTD variants in run D1110 as a representative of artefactual mutations in our analysis. Comparing the frequency of wells with co-presence of two allele for the same locus (Figure 2A) as well as the number of wells supporting each variant (Figure 2B) in run D1110 showed that UTDs had significantly higher proportion of the former and lower number of the latter compared to any other category of mutations (p < 2e-16 for both analyses, One-way ANOVA followed by Tukey HSD testing). This clearly supported the distinct distribution pattern of artefactual mutations in this DigiPico dataset as hypothesized.

**MutLX analysis pipeline for DigiPico data**

Having obtained high-quality data using DigiPico sequencing, we decided to implement an analysis pipeline to eliminate FP variant calls based on the distribution pattern of mutations. As mentioned earlier, ANN algorithms are ideally suited for problems with such complex patterns. Given a representative set of correctly labelled examples (training set), an ANN can learn to classify mutations without the need for any class-specific information. However, there are two main issues in implementing ANN algorithms for the problem of eliminating FP mutations from sequencing data; (a) the difficulty in obtaining a generalizable model and (b) unavailability of representative accurately labelled training sets. First, it is not possible to generate a model that is generalizable for the analysis of every DigiPico dataset because the distribution pattern of mutations depends on various run-specific initial conditions that cannot easily be accounted for (e.g. the copy number state of the genome). Therefore, run-specific models tailored to each DigiPico run will be required. This means that subsets of run-specific mutations need to be selected as a training sets for each DigiPico run. Second, while correctly labelled examples of true mutations can easily be extracted from known SNPs in the genome, identifying a representative and accurate set of examples for artefactual mutations is not possible. To address this issue, we considered UTDs as a reasonable approximation for a representative set of artefactual mutations, assuming that UTDs are predominantly composed of such mutations. This assumption, however, can result in a key challenge. UTDs by definition are composed of artefactual mutations as well as true clone-specific mutations. While artefactual mutations are expected to be abundantly present in all DigiPico runs, true clone-specific mutations may be present at different frequencies depending on the sample (Figure 1J). Therefore, when UTDs are all considered as examples for artefactual mutations, samples with more clone-specific variants will have a noisier training set. If this is not taken into account, it can put the samples with true-clone specific variants at an analytical disadvantage, because noisier training sets can result in worse classification models (Figure 2C). Specifically, the presence of real mutations among the examples of artefactual mutations in samples with true clone-specific variants may result in over-fitting of the model against these variants (Figure 2C). This can undermine the ability of the model to accurately identify true clone-specific variants in such samples. Given that the main objective of DigiPico sequencing is to identify clone-specific variants it is essential to ensure that such over-fitting does not occur when analysing DigiPico datasets.

Considering all the aforementioned limitations and issues, we designed and implemented an ANN-based binary classifier, MutLX, for the analysis of DigiPico datasets. The focus of the DigiPico analysis pipeline was set for effective elimination of FP calls and accurate identification of true clone-specific variants from UTDs. To address the issue of training with imperfect training sets, we employed the following approach in training MutLX. Initially we considered all UTD variants as examples for artefactual mutations (labelled as 0s) and a similar number of randomly selected heterozygous germline SNPs as example for true variants (labelled as 1s). Since the majority of UTD variants are FP calls with an unknown ratio of true clone-specific variants, the 0 labels are considered to be "noisy" at this stage. In other words, while true-clone specific variants, must have been labelled as 1, because of their anonymity at this stage, they are labelled as 0 among others. To accommodate for this type of noise in the training dataset we employed a two-step training process (Figure 2D). In the first step, a model is trained given all labelled example data. This initial model is then used to compute the probability of each mutation belonging to its label's category. Any mutation that appears to have been mislabelled based on these model predictions, is temporarily eliminated (pruned) from the dataset. The underlying assumption here is that even though a model trained on noisy samples might not be as robust as a model trained on a hypothetically clean dataset, still, it will be biased towards better predictions of correct examples because of their higher ratio compared to noise in the dataset. Therefore, the examples that the model predict against their original label, are likely to have been mislabelled in the first place. In the second step, a new classification model is trained on this pruned training set. Since the second training set is likely to contain less mislabelled data points, the final model is expected to be more effective at identifying true mutations regardless of whether or not all UTD variants had indeed been artefacts (31, 32). We next employ this model to assign a "probability score" to each putative mutation. This score indicates the likelihood that a certain mutation belongs to the true variants' category. While this two-step training process is expected to significantly improve the classification model, the final model will still be prone to errors due to imperfections in the training sets. Therefore, we added another level of analysis to further improve the accuracy of our pipeline. This was achieved by assigning an uncertainty estimate to the "probability score" of each mutation. This uncertainty estimation is based on the assumption that a robust prediction is supported by most of the activated neurons in the hidden layers of the ANN. Therefore, any subset of these neurons would also consistently result in a similar probability score and hence, there will be a low variance between various "probability scores" obtained from different neuronal subsets (Figure 2E). In contrast, the seemingly high "probability score" of an artefactual mutation is most likely only supported by some of the neurons in the hidden layer of the ANN. Therefore, different neuronal subsets will result in varying "probability scores" which will lead to a high variance in the scores obtained from different neuronal subsets for an artefactual mutation (Figure 2E). As a result, an "uncertainty score" can be calculated as the variance of "probability scores" obtained from multiple different randomly selected neuronal subsets of the trained ANN in MutLX (26). The combination of "probability score" and "uncertainty score" for each mutation should therefore allow us to accurately determine whether a called variant is a real mutation or a result of artefactual changes in the template (Figure 6).

**Validation of the MutLX algorithm**

To validate our strategy, we chose to test the MutLX analysis pipeline on runs D1110 and D1111. This is because these DigiPico runs had been obtained from a HGSOC that was previously extensively sequenced with data available from 48 independent whole genome sequencing data sets across three different time points (patient #11152) at a total depth of approximately 4200x from two independent sequencing platforms (33). To our knowledge, this comprises the most extensively whole genome sequenced tumour to date. This exceptionally large dataset allows for reliable cross-validation of mutations in this tumour. For this purpose, we used the MutLX algorithm to analyse the sequencing data from runs D1110 and D1111. As explained previously, when using the bulk sequencing data from the PT2R site for comparison with these DigiPico datasets, true UTD variants (clone-specific variants) are only expected to be present in run D1111, while nearly all UTDs in run D1110 are expected to be artefacts (Figure 1J). In addition, we also analysed DigiPico sequencing data prepared from purified DNA of a blood sample (run DE011), as well as cultured GM12885 lymphoblastoid cells, both of which are also expected to have no true UTD mutations. The *de novo* variant calling on these DigiPico runs followed by an initial filtering based on the well counts resulted in the identification of thousands of UTD variants in each sample, nearly all of which were expected to be FP calls. However, the application of MutLX algorithm on the UTD variants of runs D1110, DE011, and GM12885 resulted in the effective elimination of over 99% of the FP variant calls in these runs to only 4, 7, and 3 genome-wide FP mutations, respectively, while maintaining a sensitivity of ~85% for detecting true mutations (Figures 2F and 2G). In comparison, SCcaller (11) analysis of the same data resulted in 713, 712, and 13,280 FP variant calls, respectively (Figure 2G). On the other hand, MutLX identified 264 putative clone-specific variants in run D1111, 238 of which (90%) were validated through comparison with independent high-depth datasets of this tumour sample (Figure 2G). Moreover, these observations were further validated by performing targeted sequencing on the bulk DNA of the tumour. Whereby, out of the 11 analysed amplicons harbouring clone-specific variants from run D1111, 10 were found to conclusively be present at low frequencies in the bulk DNA of the PT2R sample (Figure S7). Furthermore, amplicon sequencing of 37 seemingly high-quality UTD variants from run DE111 that were labelled as artefactual by MutLX algorithm indicated no evidence for their presence in the bulk DNA sample (Figure 11). These results clearly confirm that MutLX can learn accurate classification models that distinguish artefactual mutations from real variants and is able to effectively identify true clone-specific variants in DigiPico data.

Additionally, we investigated whether the presence of true clone-specific mutations could compromise the sensitivity of the model due to over-fitting. For this purpose, we artificially mislabelled varying numbers of somatic mutations in runs D1110 and DE111 as artificial UTD variants (UTD*) to generate synthetic datasets with various ratios of true UTDs. These synthetic datasets were then independently analysed by MutLX and the FP rate as well as the recovery rate of UTD*s at varying UTD*/UTD ratios were examined in all the synthetic datasets. The results showed that a UTD*/UTD ratio as high as 10% does not significantly affect the recovery rate of UTD* variants, indicating that overfitting does not occur in MutLX (Figure 9).

**Versatility of DigiPico/MutLX sequencing and analysis approach**

Finally, to ensure the versatility of our proposed method, DigiPico sequencing was performed on various sources of template DNA from four different HGSOC patients and the resulting UTDs were analysed using MutLX algorithm. The results clearly indicate that MutLX can reliably identify and eliminate the artefactual variant calls from a diverse set of DigiPico libraries (Table 1). This strongly suggests that DigiPico/MutLX can effectively enable the study of recently acquired mutations in solid tumours. Importantly, analysing the frequency of different mutation types in these data indicated the presence of a higher level of C>A mutations among the identified artefactual mutations, consistent with the notion that such FP calls are a result of oxidative damage to the template DNA (Figure 12).

**The study of active mutational processes using DigiPico/MutLX**

We next tested the feasibility of studying mutational processes in a patient with HGSOC (#11152). For this patient, various sequencing data from a pre-chemotherapy omental mass were available (standard bulk sequencing at 30x as well as five tumour islet DigiPico runs). The patient subsequently had a recurrence and tumour samples were collected from the pelvis (pelvic recurrent tumour; PT2R) and from the para-aortic lymph node (PALNR) for standard bulk sequencing as well as DigiPico sequencing of tumour islets. The analysis of the bulk pre-chemotherapy sequencing data identified 13,721 somatic mutations. 84.6% of these mutations were present in at least three tumour islets from DigiPico data, 91.4% of which were also present in at least three additional islets from previously published LFR data (33). The high occurrence of the mutations indicates that they were early mutations that became fixed in the tumour. The analysis of DigiPico data from tumour islets revealed that there was a limited number of clone-specific mutations that were absent in the bulk tumour. Each of the five pre-chemotherapy islets harboured a number of truly unique mutations (2, 6, 8, 8, and 36), compared to other islets, indicating that they were recent occurrences (Figure 3A). The bulk WGS data of the PT2R recurrence indicated the emergence of 3,009 new somatic mutations that were absent in the pre-chemotherapy bulk sequencing data, DigiPico data or LFR data. These mutations may have occurred at any point since the common ancestors of the omental mass and the PT2R recurrence diverged from each other (Figure 3A). The analysis of tumour islets in the recurrence samples from patient #11152 showed that the pelvic recurrent tumour (PT2R) has a high load of clone-specific mutations compared to the para-aortic lymph node recurrence (PALNR) or the pre-chemotherapy tumour. This observation suggests that, in this patient, molecular mechanisms underlying the SNV mutagenesis may have been recently activated (Figure 3A). Moreover, analysing the clone-specific mutations of the PT2R sample with a rainfall plot revealed the presence of a strong sub-clonal local hyper-mutagenesis (kataegis) event (8) on chromosome 17 (Figures 3B, 3C, and 11). Comparing the mutations that made up this kataegis events to bulk sequencing data, DigiPico data and LFR data of the pre-chemotherapy omental mass revealed that they were only found in the DigiPico PT2R data indicating that they were genuine clone-specific mutations.

**Discussion**

In this work we presented DigiPico/MutLX as an integrated platform for the identification of mutations from small groups of cells with unprecedented accuracy on a whole genome scale. We believe that this work provides an important stepping stone for the discovery of current or recent somatic mutational processes that occur in cancer and normal tissue. Understanding current mutational processes is key for predicting the evolutionary trajectory of a tumour and, potentially, for interfering with such trajectories therapeutically. A mutation that is identified in bulk sequencing of a tumour must have occurred at a point during the extended history of a tumour from the initiation till presentation. In contrast, a cell-specific mutation must have occurred during the limited lifetime of that cell. Similarly, a mutation in a small clone that has been derived from a single cell is also recent. The age of such a mutation can't be more than the age of the clone which is defined by the number of cell divisions it took to generate that clone. Studying patterns in cell-specific or small-clone-specific mutations can allow for the identification of recent or current mutational processes (1). Defining such processes is highly desirable since they can be causally linked to biological or chemical phenomena and, therefore, yield significant mechanistic insights. Identifying these mechanisms have important practical implications since they are potentially amenable for therapeutic intervention or for predicting future tumour behaviour. The current state of the art does not allow the direct accurate identification of mutations from individual cells or individual small clones from tumours. DigiPico/MutLX enables this endeavour for the first time.

To overcome significant technical pitfalls predominantly related to the discovery of false positive mutations, current methods for single cell WGS analysis either require extensive validation studies (11) or rely on combining data from multiple cells to obtain reliable mutations that are shared between cells (12, 34). These cells are then grouped into clones that have been derived from a common ancestor. While such techniques go to a more recent common ancestor compared to bulk sequencing, they are still not ideal as data derived from these approaches do not reflect mutational processes that are taking place in existing cells. Furthermore, reducing the depth of sequencing per cell to enable sequencing large numbers of cells, reduces the breadth of coverage, which is already compromised by loss of genetic materials during preparation steps. This increases the number of cells that are needed to be analysed for inferring and identifying clones which in turn moves the ancestor further back into the past. In addition, the lack of information about physical relatedness in single cell analysis methods, results in loss of an opportunity to group cells that are likely to be from a single clone. This increases the gap between the ancestor of an inferred clone and the present time, making it difficult to define processes that are active within currently existing cells in a tumour.

DigiPico/MutLX has the distinct advantage of enabling the preservation of spatial information. Analysing spatially-related cells, preserves physical relatedness and enables the assumption that physically related cells belong to an individual clone (9). Defining distinct structures that may have arisen from a tissue resident stem cell has also been suggested to identify and analyse clones. For example, cells from a single small intestinal crypt or a single endometrial gland could be reasonable expected to come from a single tissue-resident stem cell (35, 36). Under these circumstances, each anatomical unit defines a clone that may or may not have clone specific mutations that can be related to a mutational driver. Furthermore, sequencing data from a clone can be computationally used to infer subclones and predict more recent events that may have arisen within a clone. This is akin to what bulk sequencing and analysis achieves but at the level of a single clone that is composed of a limited number of cells. Preserving spatial information is also particularly interesting because of the recent developments in enabling spatial transcriptomics technologies (37). It is conceivable that combining highly accurate DNA sequencing with spatial transcriptomics would allow the dissection of genetic and non-genetic heterogeneity in tissues. In short, current technologies, for the analysis of small clones yield large number of false positive results making it impossible to obtain direct accurate clone specific information on a genome scale without exhausting validation. Combing data from multiple clones, is a common solution but moves the ancestor further back into the past. We have previously used this approach for the analysis of small collection of tumour cells (tumour islets) (33). Because of the uncertainty associated with the mutation calls from individual islets, it was necessary to only call mutations that were shared between all tumour islets and effectively identify only truncal mutations. This was then followed by independent validation of some 700 mutations using targeted sequencing. While this still yielded important biological insights, we were unable to study islet-specific mutations. DigiPico/MutLX is now enabling the study of such mutations. We demonstrated how the direct analysis of DNA from ~30 cancer cells, resulted in the confident identification of a sub-clonal kataegis event.

Overall, here we showed that DigiPico and MutLX can enable hyper-accurate identification of somatic mutations from limiting numbers of cells obtained from clinical samples, as an important improvement over the existing methodologies. Moreover, unlike other computational methods that rely on diploid regions of the genome to calculate amplification biases, our method is also compatible with genomes that suffer from extensive copy number alterations, such as in HGSOC. We believe that the versatility of the DigiPico/MutLX method enables the study of active mutational processes in tumours as well as in normal tissues.

**AVAILABILITY**

Source code for MutLX is available on Github (https://github.com/mmdknr/DigiPico).

**ACCESSION NUMBERS**

All sequencing data used in this study are available on EGA (EGAD00001005118).

**REFERENCES**

1. Turajlic,S., Sottoriva,A., Graham,T. and Swanton,C. (2019) Resolving genetic heterogeneity in cancer. Nat. Rev. Genet., 10.1038/s41576-019-0114-6.

2. Zhang,J., Späth,S.S., Marjani,S.L., Zhang,W. and Pan,X. (2018) Characterization of cancer genomic heterogeneity by next-generation sequencing advances precision medicine in cancer treatment. Precis. Clin. Med., 1, 29-48.

3. Gerstung,M., Jolly,C., Leshchiner,I., Dentro,S.C., Gonzalez,S., Mitchell,T.J., Rubanova,Y., Anur,P., Rosebrock,D., Yu,K., et al. (2017) The evolutionary history of 2,658 cancers. bioRxiv, 10.1101/161562.

4. Barber,L.J., Davies,M.N. and Gerlinger,M. (2015) Dissecting cancer evolution at the macro-heterogeneity and micro-heterogeneity scale. Curr. Opin. Genet. Dev., 30, 1-6.

5. Bohrson,C.L., Barton,A.R., Lodato,M.A., Rodin,R.E., Luquette,L.J., Viswanadham,V. V, Gulhan,D.C., Cortes-Ciriano,I., Sherman,M.A., Kwon,M., et al. (2019) Linked-read analysis identifies mutations in single-cell DNA-sequencing data. Nat. Genet., 10.1038/s41588-019-0366-2.

6. Chen,L., Liu,P., Evans,T.C.J. and Ettwiller,L.M. (2017) DNA damage is a pervasive cause of sequencing errors, directly confounding variant identification. Science, 355, 752-756.

7. Costello,M., Pugh,T.J., Fennell,T.J., Stewart,C., Lichtenstein,L., Meldrim,J.C., Fostel,J.L., Friedrich,D.C., Perrin,D., Dionne,D., et al. (2013) Discovery and characterization of artifactual mutations in deep coverage targeted capture sequencing data due to oxidative DNA damage during sample preparation. Nucleic Acids Res., 41, e67.

8. Nik-Zainal,S., Alexandrov,L.B., Wedge,D.C., Van Loo,P., Greenman,C.D., Raine,K., Jones,D., Hinton,J., Marshall,J., Stebbings,L.A., et al. (2012) Mutational processes molding the genomes of 21 breast cancers. Cell, 149, 979-993.

9. Martincorena,I., Fowler,J.C., Wabik,A., Lawson,A.R.J., Abascal,F., Hall,M.W.J., Cagan,A., Murai,K., Mahbubani,K., Stratton,M.R., et al. (2018) Somatic mutant clones colonize the human esophagus with age. Science, 362, 911-917.

10. Tubbs,A. and Nussenzweig,A. (2017) Endogenous DNA Damage as a Source of Genomic Instability in Cancer. Cell, 168, 644-656.

11. Dong,X., Zhang,L., Milholland,B., Lee,M., Maslov,A.Y., Wang,T. and Vijg,J. (2017) Accurate identification of single-nucleotide variants in whole-genome-amplified single cells. Nat. Methods, 14, 491-493.

12. Zafar,H., Wang,Y., Nakhleh,L., Navin,N. and Chen,K. (2016) Monovar: single-nucleotide variant detection in single cells. Nat. Methods, 13, 505-507.

13. Chen,C., Xing,D., Tan,L., Li,H., Zhou,G., Huang,L. and Xie,X.S. (2017) Single-cell whole-genome analyses by Linear Amplification via Transposon Insertion (LIANTI). Science, 356, 189-194.

14. Krueger F. (2016) Trim Galore!

15. Langmead,B. and Salzberg,S.L. (2012) Fast gapped-read alignment with Bowtie 2. Nat Meth, 9, 357-359.

16. McKenna,A., Hanna,M., Banks,E., Sivachenko,A., Cibulskis,K., Kernytsky,A., Garimella,K., Altshuler,D., Gabriel,S., Daly,M., et al. (2010) The Genome Analysis Toolkit: a MapReduce framework for analyzing next-generation DNA sequencing data. Genome Res., 20, 1297-1303.

17. Kim,S., Scheffler,K., Halpern,A.L., Bekritsky,M.A., Noh,E., Källberg,M., Chen,X., Kim,Y., Beyter,D., Krusche,P., et al. (2018) Strelka2: fast and accurate calling of germline and somatic variants. Nat. Methods, 15, 591-594.

18. Hosokawa,M., Nishikawa,Y., Kogawa,M. and Takeyama,H. (2017) Massively parallel whole genome amplification for single-cell sequencing using droplet microfluidics. Sci. Rep., 7, 5199.

19. Peters,B.A., Kermani,B.G., Sparks,A.B., Alferov,O., Hong,P., Alexeev,A., Jiang,Y., Dahl,F., Tang,Y.T., Haas,J., et al. (2012) Accurate whole-genome sequencing and haplotyping from 10 to 20 human cells. Nature, 487, 190-195.

20. Picard Tools (2018).

21. Rimmer,A., Phan,H., Mathieson,I., Iqbal,Z., Twigg,S.R.F., Wilkie,A.O.M., McVean,G. and Lunter,G. (2014) Integrating mapping-, assembly- and haplotype-based approaches for calling variants in clinical sequencing applications. Nat. Genet., 46, 912-918.

22. Derrien,T., Estellé,J., Marco Sola,S., Knowles,D.G., Raineri,E., Guig6,R. and Ribeca,P. (2012) Fast computation and applications of genome mappability. PLoS One, 7, e30377-e30377.

23. Danecek,P., Auton,A., Abecasis,G., Albers,C.A., Banks,E., DePristo,M.A., Handsaker,R.E., Lunter,G., Marth,G.T., Sherry,S.T., et al. (2011) The variant call format and VCFtools. Bioinformatics, 27, 2156-2158.

24. Chollet,F. and others (2015) Keras.

25. Kingma,D.P. and Ba,J. (2014) Adam: A Method for Stochastic Optimization. CoRR, abs/1412.6**.**

26. Gal,Y. and Ghahramani,Z. (2015) Dropout as a Bayesian Approximation: Representing Model Uncertainty in Deep Learning. arXiv e-prints*.*

27. Drmanac,R., Sparks,A.B., Callow,M.J., Halpern,A.L., Burns,N.L., Kermani,B.G., Carnevali,P., Nazarenko,I., Nilsen,G.B., Yeung,G., et al. (2010) Human Genome Sequencing Using Unchained Base Reads on Self-Assembling DNA Nanoarrays. Science (80-. )., 327**.**

28. Arbeithuber,B., Makova,K.D. and Tiemann-Boege,I. (2016) Artifactual mutations resulting from DNA lesions limit detection levels in ultrasensitive sequencing applications. DNA Res., 23, 547-559.

29. Amini,S., Pushkarev,D., Christiansen,L., Kostem,E., Royce,T., Turk,C., Pignatelli,N., Adey,A., Kitzman,J.O., Vijayan,K., et al. (2014) Haplotype-resolved whole-genome sequencing by contiguity-preserving transposition and combinatorial indexing. Nat. Genet., 46, 1343-1349.

30. Zheng,G.X.Y., Lau,B.T., Schnall-Levin,M., Jarosz,M., Bell,J.M., Hindson,C.M., Kyriazopoulou-Panagiotopoulou,S., Masquelier,D.A., Merrill,L., Terry,J.M., et al. (2016) Haplotyping germline and cancer genomes with high-throughput linked-read sequencing. Nat. Biotechnol., 34, 303-311.

31. Northcutt,C.G., Wu,T. and Chuang,I.L. (2017) Learning with Confident Examples: Rank Pruning for Robust Classification with Noisy Labels. In Proceedings of the Thirty-Third Conference on Uncertainty in Artificial Intelligence, UAI' 17. AUAI Press.

32. Natarajan,N., Dhillon,I.S., Ravikumar,P.K. and Tewari,A. (2013) Learning with noisy labels. In Advances in neural information processing systems.pp. 1196-1204.

33. Hellner,K., Miranda,F., Fotso Chedom,D., Herrero-Gonzalez,S., Hayden,D.M., Tearle,R., Artibani,M., KaramiNejadRanjbar,M., Williams,R., Gaitskell,K., et al. (2016) Premalignant SOX2 overexpression in the fallopian tubes of ovarian cancer patients: Discovery and validation studies. EBioMedicine, 10, 137-149.

34. Laks,E., Zahn,H., Lai,D., McPherson,A., Steif,A., Brimhall,J., Biele,J., Wang,B., Masud,T., Grewal,D., et al. (2018) Resource: Scalable whole genome sequencing of 40,000 single cells identifies stochastic aneuploidies, genome replication states and clonal repertoires. bioRxiv, 10.1101/411058.

35. Moore,L., Leongamornlert,D., Coorens,T.H.H., Sanders,M.A., Ellis,P., Dawson,K., Maura,F., Nangalia,J., Tarpey,P.S., Brunner,S.F., et al. (2018) The mutational landscape of normal human endometrial epithelium. bioRxiv, 10.1101/505685.

36. Lee-Six,H., Ellis,P., Osborne,R.J., Sanders,M.A., Moore,L., Georgakopoulos,N., Torrente,F., Noorani,A., Goddard,M., Robinson,P., et al. (2018) The landscape of somatic mutation in normal colorectal epithelial cells. bioRxiv, 10.1101/416800.

37. Burgess,D.J. (2019) Spatial transcriptomics coming of age. Nat. Rev. Genet., 20, 317.

**DigiPico2**

**Example 2 - DigiPico2, a novel method for whole genome sequencing of picogram quantities of DNA with unprecedented accuracy**

**Introduction**

Previously we described DigiPico library preparation pipeline and MutLX analysis platform as a method for accurate identification of single nucleotide variants (SNV) from limited amount of clinical material. This was an important methodological advancement mainly due to the fact that the limited amount of genetic material obtained from clinical samples must be whole genome amplified (WGA) prior to sequencing. The process of WGA however introduces up to 100,000 artefactual mutations in the amplified DNA which inundates the final analysis results with false positive variant calls that hamper any meaningful genetic interpretation from the original sample. In DigiPico/MutLX strategy we overcome this obstacle by separating individual molecules of DNA into independent compartments before the WGA step and indexing them after the process. By doing so we digitize the information for real mutations, meaning each compartment will either carry the mutated allele or not. Artefactual mutations, however, because of the way that they are generated during the WGA process, will result in compartments that will contain both the mutated and the reference allele information (Figure 1B). Based on this information we then developed an artificial neural network (ANN) based algorithm, MutLX, to effectively identify and eliminate these artefactual mutations from our data (Figure 2). We extensively tested our strategy on simulated data as well as patient samples and showed that our method is indeed effective in eliminating the FP variant calls.

While producing high quality data, DigiPico library preparation method, however, suffers from few technical limitations. Firstly the fragmentation step of the library preparation (CoREF), which was borrowed from a previously described method, is extremely complex and time consuming. Moreover, CoREF requires the use of dUTP during the WGA process. Since dUTP is an unnatural nucleotide it is likely that it may introduce further artefactual mutations in the final products. Next, we found that the adapter ligation efficiency was very low in DigiPico which could sometimes compromise the library quality. Lastly, because of the high number of indices and lack of redundancy in the index information there is a chance for index cross-contamination which could adversely affect the final results. Therefore, we developed DigiPico2 library preparation method to address all these issues.

## Results

*Improving the DigiPico library preparation workflow*

As explained previously, the use of dUTP in the DigiPico method is because of the requirement of CoREF fragmentation procedure, which is a very complex fragmentation strategy (Figure 14A). Therefore using an alternative fragmentation method would solve the complexity and the dUTP issues at the same time. For this we decided to use an existing fragmentation and end-repair strategy offered by the Lotus DNA library preparation kit (IDT, USA). In Lotus DNA library preparation kit, a cocktail of enzymes is used to fragment the large DNA molecules into smaller pieces in a time dependant manner, and to prepare the termini of the fragments for the ligation step. To make this new strategy compatible with DigiPico we initially ensured that all compartments receive the enzyme cocktail at more or less the same time using the I-DOT (Dispendix, Germany) dispenser. Next we optimized the reaction conditions to achieve the desired fragment lengths for DigiPico sequencing. Doing so we were able to reduce the library preparation time to 4.5 hours from the original 12 hours and eliminate the need to use dUTP in the WGA reaction (Figure 14B).

Next, we aimed at addressing the low ligation efficiency in DigiPico. Originally our adapter ligation and indexing relied on using an asymmetric ligation approach. In this approach long indexing oligos with short complementary regions were used for ligation which is extremely inefficient (Figure 15A). A more efficient approach would require a looped common adapter that is ligated to both ends of the fragments. But because these adapters do not contain any indices in their standard form, the products need to be individually purified and then indices are introduced later through a PCR reaction using indexing primers. This however adds another challenge because purifying 384 independent products will be extremely complex, time consuming, and adds a significant risk of cross-contamination. To overcome these issues, we designed a novel indexing strategy for DigiPico2. In DigiPico2, one set of indices are first introduced in the stem loop of the common adapter (Figure 15B). At the ligation step, all wells in each column of the plate will receive a different indexing looped common adapter, therefore a total of 24 different oligos would be sufficient to index all columns of the plate with the first set of indices (Column indices). After the ligation step, all wells in each row will be pooled into a separate tube, resulting in 16 different pools. These 16 different pools can conveniently be purified to be used for the next step of indexing. At the next step the purified products from each pool will be indexed through a PCR reaction using 16 different indexing primers (Row indices). At the end of this stage each well of the plate will have received a different column-row index combination. Using this indexing strategy not only will significantly improve the ligation efficiency but also introduces 2 sets of redundancy that can be employed to eliminate index cross-contamination from the data. In the first set, the column indices will bind to both end of each fragment. Therefore any cross contamination will most likely result in fragments that have unidentical indices at their termini and so can easily be removed from the data. In the second set, the indexing oligos for each row can be dually indexed so that both the standard index 1 (i7) and standard index 2 (i5) sequences can uniquely identify a specific row. Combining these sets of redundancy the index cross-contamination rate can be reduced by at least 2 orders of magnitude.

*DigiPico2 workflow significantly improves the library quality*

To test the effect of these modification on the final quality of the data, DigiPico2 sequencing was performed on 120 pg of DNA from blood of patient 11152. This sample was used because we had previously extensively sequenced the tumour and normal cells from this patient. As expected the WGA, similar to the previous version, resulted in a very uniform distribution of products (Figure 16A). After library preparation and sequencing, however, it became clear that unlike with DigiPico, in DigiPico2 the representation of each well in the finally library appears to strongly correlate with the amount of WGA products (Figure 16A-C). This is likely a direct result of an improved ligation efficiency. This improved correlation can also allow for introduction of a QC measure, solely based on the uniformity of the WGA products which previously was not possible. Moreover, analysing the index redundancy information we found that nearly 5% of the reads were eliminated using the index cross-contamination filter. Without this new filter these contaminants could have negatively affect the results of the analysis. Lastly we analysed the data from DigiPico2 using the MutLX algorithm. The final results showed a clearer distinction between artefactual mutations and real mutations indicating that the DigiPico2 performs at least as good as DigiPico method when analysed using MutLX algorithm (Figure 16D).

*Extending DigiPico2 workflow to single cell whole genome sequencing*

Having established DigiPico2 workflow, we tested whether it can be applied to single cell whole genome sequencing. This is important, since an active mutational process is likely to start within an individual cell. We, therefore, introduced a work flow for single cell DigiPico (ScDigiPico) sequencing by partitioning DNA from individual cells to an entire row of a 384-well plate (Figure 17A). To evaluate the efficacy of ScDigiPico method at identifying active mutational process, we simulated a such process by mutagenizing cultured Kuramochi cells using N-ethyl-N-nitrosourea (ENU). ENU is an alkylating agent and a very potent mutagen and preferentially causes T>C, T>A, and C>T mutations. In this setting, each cell is expected to obtain a different set of mutations but because the underlying mechanism of mutagenesis is the same the mutations are expected to be of the same type. ScDigiPico enabled the identification of the enrichment of the aforementioned mutation types (Figure 17B). We also further investigated the potential of ScDigiPico by irradiating Kuramochi cells with UV light prior to single cell sorting and ScDigiPico library preparation (Figure 17C) and identified a Kataegis event in one of the cells. These results cumulatively indicate that ScDigiPico is an effective strategy for accurate identification of true mutations caused by active mutational processes in individual cancer cells.

## DigiPico2 protocol

200 pg of purified DNA, 20 - 30 resuspended nuclei, or laser-capture micro-dissected tumour islets, were first denatured using 5 $\mu$l of D2 buffer from Repli-g single cell kit (Qiagen). After 5 minutes incubation at room temperature, 95 $\mu$l of water was added to the sample and then using Mosquito HTS liquid handler (TTP Labtech) 200 nl of the denatured template was added to each well of a 384-well reaction plate already containing 800 nl of WGA mix (0.58 $\mu$l Sc Reaction Buffer, 0.04 $\mu$l Sc Polymerase (REPLI-g Single Cell kit, Qiagen), 0.04 $\mu$l EvaGreen 20x (Biotium), and 0.065 $\mu$l water). The plate was incubated at 30 °C for 1.5 hours followed by heat inactivation at 65 °C for 15 minutes. Addition of EvaGreen in the reaction allows for monitoring of the WGA reaction using a real-time PCR machine if required. Next, 250 nl of the WGA reactions was transferred to a new plate and 1.1 $\mu$l of NEBNext Ultra II FS Reaction mixture (753 nl water, 270 nl Ultra II FS Reaction Buffer, and 77 nl Ultra II FS Enzyme Mix) was added to each well using I-DOT dispenser (Dispendix). Plate was incubated at 37 °C for 6 minutes followed by incubation at 65 °C for 30 minutes. Next 150 nl of DigiPico indexed loop-adapters carrying column indices was added to all wells. Please note that all wells within the same column would receive the same indexing oligo at this stage. Next 1.2 $\mu$l of Ultra II Ligation mix (1150 nl Ultra II Ligation Master Mix, 38 nl Ligation Enhancer, and 12 nl water) was added to each well using Mosquito liquid handler with 5 cycles of mixing and the plate was incubated at 20 °C for 15 minutes followed by heat inactivation at 65 °C for 10 minutes. Next all wells within same row were pooled together using Mosquito liquid handler. Then 1.5 $\mu$l of USER enzyme (NEB) was added to 20 $\mu$l of pool from each row and the reaction was incubated at 37 °C for 15 minutes. USER enzyme cuts the looped adapters at the Uracil position. Next the products were size selected using SPRI beads to achieve a size range of 300-400 bp. Products from each row were then amplified using row indexing primers for 4 cycles. The final products were pooled together and the final library was purified using SPRI beads.

## ScDigiPico protocol

Individual cells were sorted to wells in the first column of a 384-well plate. Each well contained 4.5 $\mu$l of MyPK buffer (). Plate was incubated at 55 °C for 30 minutes. Then 900 nl of Stop Solution was added to each well and the plate was kept at 95 °C for 5 minutes to inactivate the proteinase K. Lysed cells were then distrusted across the rows using Mosquito liquid handler, 200 nl in each well. Next 800 nl of WGA reaction mixture was added to each well and the WGA and library preparation was followed similar to DigiPico2.

## Indexed Looped-adapter - column indices

(some parts of the sequence from the instruction manual of library preparation (NEBNext® Multiplex Oligos for Illumin® (Index Primers Set 1)) - https://international.neb.com/-    /media/nebus/files/manuals/manuale7335.pdf?rev=4bf1622b342b4d73a2b01443068ed 2c5&hash=B049D91A18CDB471AB388DC6E67E06B79263ESC5)

P-[index'] AGATCGGAAGAGCACACGTCTGAACTUCCCTACACGACGCTCTTCCGATCT [index]*T (SEQ ID NO: 1)
Where P is a 5' phosphate group and * shows a phosphorothioate bond. Index = a column index (Ci) or row index (Ri) sequence acting as a unique barcode for each column or row respectively.

P5:         AATGATACGGCGACCACCGAGATCTACAC        [r-index]
ACACTCTTTCCCTACACGACGCTCTTCCGATC*T (SEQ ID: NO: 2)

P7:         CAAGCAGAAGACGGCATACGAGAT            [r-index]
GTGACTGGAGTTCAGACGTGTGCTCTTCCGATC*T (SEQ ID: NO: 3)

\* shows a phosphorothioate bond.

**Claims**

1. A method of whole genome sequencing of a single cell or cell-group for identification of single nucleotide variants, determining chromosome structural variations, or determining phasing information in the genome of the single cell or cell-group, the method comprising:

    i) providing a multi-well array plate comprising rows and columns of reaction wells;
    ii) providing genomic DNA of single cells or cell-groups, wherein the genomic DNA is distributed into a plurality of reaction wells on the multi-well array plate, such that there is no more than one single-stranded genomic DNA molecule of any given locus per reaction well,
    iii) carrying out whole genome amplification (WGA) of each genomic DNA molecule to provide multiple copies of the genomic DNA molecule in each reaction well;
    iv) fragmenting the DNA molecules of each reaction well and ligating a pair of looped adapters at each end or tagmenting using transposase-delivered adapters to form adapted-DNA fragments, wherein the looped adapters or transposase-delivered adapters comprise either a Column Index (Ci) sequence or a Row Index (Ri) sequence, wherein the Ci sequence is common to each looped adapter or transposase-delivered adapter of every reaction well in a column of the multi-well array plate, or wherein each Ri sequence is common to each looped adapter or transposase-delivered adapter of every reaction well in a row of the multi-well array plate;
    vi) providing the indexed DNA library by performing indexing PCR on the adapted-DNA fragments, wherein the adapted-DNA fragments are amplified to form indexed PCR products using forward and reverse indexing primers, wherein either a Row Index (Ri) sequence or Column Index (Ci) sequence is introduced by each forward and reverse indexing primers onto each end of the adapted-DNA fragments, such that the resulting indexed PCR products comprise both a pair of flanking Column Index (Ci) sequences that are common to each well of a column and a pair of flanking Row Index (Ri) sequences that are common to each well of a row, and
    vii) sequencing the indexed DNA library to provide data for determining any single nucleotide variants, determining chromosome structural variations, or determining phasing information in the genome of the single cell or cell-group.

2. The method of whole genome sequencing of a single cell or cell-group according to claim 1, wherein looped adapters are provided, such that the method comprises a step of fragmenting the DNA molecules of each reaction well and a subsequent ligation reaction to ligate looped adapters to the fragmented DNA; or wherein the transposase-delivered adapters are provided such that the method comprises fragmenting the DNA molecules by the process of tagmentation.

3. The method of whole genome sequencing of a single cell or cell-group according to claim 1 or 2, wherein following fragmenting of the DNA to form DNA fragments, the DNA fragments are end-repaired and dA-tailed, such that they can be ligated to the looped adapters.

4. The method of whole genome sequencing of a single cell or cell-group according to any preceding claim, wherein the looped adapters comprise an oligonucleotide having a secondary stem-loop structure, and wherein the looped adapter comprises a pair of complementary sequence regions flanking a loop region, wherein the pair of complementary sequences are arranged to hybridise with each other to form the stem-loop structure of the looped adapter.

5. The method of whole genome sequencing of a single cell or cell-group according to any preceding claim, wherein Ci sequences are provided in the adapted-DNA fragments, and the method may additionally comprise the step of pooling the adapted-DNA fragments of each reaction well in a row prior to the indexing PCR; or wherein Ri sequences are provided in the adapted-DNA fragments, and the method additionally comprise the step of pooling the adapted-DNA fragments of each reaction well in a column prior to the indexing PCR.

6. The method of whole genome sequencing of a single cell or cell-group according to any preceding claim, wherein the adapted-DNA fragments comprise Ci sequences, and the forward and reverse indexing PCR primers each comprise Ri sequences, for providing a pair of Ri sequences in the indexed PCR product.

7. The method of whole genome sequencing of a single cell or cell-group according to any preceding claim, wherein the forward and reverse indexing PCR primers further comprise sequencing adapter sequences, such that sequencing adapters are incorporated into the indexed PCR product.

8. The method of whole genome sequencing of a single cell or cell-group according to any preceding claim, wherein the method comprises determining any real SNVs in the genome of the single cell or cell-group by determining if substantially all indexed DNA library sequences originating from a single well comprise the same SNV, or if only a fraction of the indexed DNA library sequences comprise the same SNV, wherein a SNV represented in substantially all indexed DNA library sequences originating from a single well is determined to be a real SNV in the genomic DNA, and a SNV found in only a fraction of the indexed DNA library sequences originating from a single well is determined to be a false positive (FP) SNV.

9. The method of whole genome sequencing of a single cell or cell-group according to any preceding claim, wherein the method further comprises matching indexed DNA library sequences originating from a single well representing one strand of the genomic DNA with indexed DNA library sequences originating from another well representing the complementary strand of genomic DNA, wherein a SNV substantially present in all indexed DNA library sequences of both complementary strands of the genomic DNA is determined to be a real SNV, and a SNV not substantially present in all indexed DNA library sequences of both complementary strands of genomic DNA is determined to be a false positive.

10. The method of whole genome sequencing of a single cell or cell-group according to claim 8 or 9, wherein the determination is carried out *in silico* using BAM file data that has been generated from mapping the sequence data to a reference genome, optionally wherein *in silico* determinations or matching of indexed DNA sequences, and/or the calculation of probability scores is carried out by an artificial neural network (ANN) model, optionally by a multilayer perceptron.

11. The method of whole genome sequencing of a single cell or cell-group according to any preceding claim wherein a probability score of a particular nucleotide variant being a real SNV or a false positive is calculated *in silico,* such that a given variant nucleotide is determined to have a statistically significant probability of being a real SNV or a false positive.

12. The method of whole genome sequencing of a single cell or cell-group according to any preceding claim wherein the sequence data is provided in the form of paired-read FastQ files, optionally wherein the sequence data of the Paired-read FastQ files is trimmed for removal of adapter sequences and for quality, to provide trimmed data.

13. A method of preparing an indexed DNA library for sequencing of nucleic acid molecules the method comprising:

 i) providing a multi-well array plate comprising rows and columns of reaction wells;
 ii) providing nucleic acid molecules, wherein the nucleic acid molecules are distributed into a plurality of reaction wells on the multi-well array plate, such that there is no more than one single-stranded nucleic acid molecule from any given locus per reaction well,
 iii) carrying out amplification of the nucleic acid molecule to provide multiple DNA copies of the nucleic acid molecule in each reaction well;
 iv) fragmenting the DNA molecules of each reaction well and ligating a pair of looped adapters at each end or tagmenting using transposase-delivered adapters to form adapted-DNA fragments, wherein the looped adapters or transposase-delivered adapters comprise either a Column Index (Ci) sequence or a Row Index (Ri) sequence, wherein the Ci sequence is common to each looped adapter or transposase-delivered adapter of every reaction well in a column of the multi-well array plate, or wherein each Ri sequence is common to each looped adapter or transposase-delivered adapter of every reaction well in a row of the multi-well array plate;
 vi) providing the indexed DNA library by performing indexing PCR on the adapted-DNA fragments, wherein the adapted-DNA fragments are amplified to form indexed PCR products using forward and reverse indexing primers, wherein either a Row Index (Ri) sequence or Column Index (Ci) sequence is introduced by each forward and reverse indexing primers onto each end of the adapted-DNA fragments, such that the resulting indexed PCR products comprise both a pair of flanking Column Index (Ci) sequences that are common to each well of a column and a pair of flanking Row Index (Ri) sequences that are common to each well of a row, and optionally wherein the forward and reverse indexing primers further provide respective 5' and 3' sequencing adapters onto the indexed PCR products that are suitable for use in a sequencing reaction.

14. A method of preparing an indexed DNA library for whole genome sequencing of single cells or cell-groups for the identification of single nucleotide variants, determining chromosome structural variations, or determining phasing information in the genome of the single cells or cell-groups, the method comprising:

i) providing a multi-well array plate comprising rows and columns of reaction wells;
ii) providing genomic DNA of single cells or cell-groups, wherein the genomic DNA is distributed into a plurality of reaction wells on the multi-well array plate, such that there is no more than one single-stranded genomic DNA molecule of any given locus per reaction well,
iii) carrying out whole genome amplification (WGA) of each genomic DNA molecule to provide multiple copies of the genomic DNA molecule in each reaction well;
iv) fragmenting the DNA molecules of each reaction well and ligating a pair of looped adapters at each end or tagmenting using transposase-delivered adapters to form adapted-DNA fragments, wherein the looped adapters or transposase-delivered adapters comprise either a Column Index (Ci) sequence or a Row Index (Ri) sequence, wherein the Ci sequence is common to each looped adapter or transposase-delivered adapter of every reaction well in a column of the multi-well array plate, or wherein each Ri sequence is common to each looped adapter or transposase-delivered adapter of every reaction well in a row of the multi-well array plate;
vi) providing the indexed DNA library by performing indexing PCR on the adapted-DNA fragments, wherein the adapted-DNA fragments are amplified to form indexed PCR products using forward and reverse indexing primers, wherein either a Row Index (Ri) sequence or Column Index (Ci) sequence is introduced by each forward and reverse indexing primers onto each end of the adapted-DNA fragments, such that the resulting indexed PCR products comprise both a pair of flanking Column Index (Ci) sequences that are common to each well of a column and a pair of flanking Row Index (Ri) sequences that are common to each well of a row, and optionally wherein the forward and reverse indexing primers further provide respective 5' and 3' sequencing adapters onto the indexed PCR products that are suitable for use in a sequencing reaction.

15. A method of whole genome sequencing of a single cell or cell-group to provide data for the identification of single nucleotide variants (SNVs), determining chromosome structural variations, or determining phasing information in the genome of the single cell or cell-group, the method comprising:

i) preparing an indexed DNA library by carrying out the method according to claim 13 or 14, or providing an indexed DNA library prepared in accordance with claim 13 or 14;
ii) sequencing the indexed DNA library to provide data for determining any single nucleotide variants (SNVs), determining chromosome structural variations, or determining phasing information in the genome of the single cell or cell-group.

**Patentansprüche**

1. Ein Verfahren zur Sequenzierung des gesamten Genoms einer einzelnen Zelle oder Zellgruppe zur Identifizierung einzelner Nukleotidvarianten, zur Bestimmung der Chromosomenstrukturvariationen oder zur Bestimmung der Phaseninformation im Genom der einzelnen Zelle oder Zellgruppe, wobei das Verfahren umfasst:

i) die Bereitstellung einer Multiwell-Testplatte, die Reihen und Spalten von Reaktionsvertiefungen umfasst;
ii) die Bereitstellen genomischer DNA von Einzelzellen oder Zellgruppen, wobei die genomische DNA in eine Vielzahl von Reaktionsvertiefungen auf der Multiwell-Testplatte verteilt wird, so dass nicht mehr als ein einzelsträngiges genomisches DNA-Molekül eines beliebigen Locus pro Reaktionsvertiefung vorhanden ist,
iii) die Durchführung der Ganzgenom-Amplifikation (WGA) jedes genomischen DNA-Moleküls, um mehrere Kopien des genomischen DNA-Moleküls in jedem Reaktionsvertiefung zu erhalten;
iv) die Fragmentierung der DNA-Moleküle jeder Reaktionsvertiefung und die Ligation eines Paares der eine Schleife bildenden Adaptern an jedem Ende oder die Tagmentierung unter Verwendung der Transposase-vermittelten Adaptern, um angepasste DNA-Fragmente zu bilden, wobei die geschlungenen Adapter oder die Transposase-vermittelten Adapter entweder eine Spaltenindex-Sequenz (Ci-Sequenz) oder eine Reihenindex-Sequenz (Ri-Sequenz) umfassen, wobei die Ci-Sequenz jedem Schleifenadapter oder Transposase-vermittelten Adapter jeder Reaktionsvertiefung in einer Spalte der Multiwell-Testplatte gemeinsam ist, oder wobei jede Ri-Sequenz jedem Schleifenadapter oder Transposase-vermittelten Adapter jeder Reaktionsvertiefung in einer Reihe der Multiwell-Testplatte gemeinsam ist;

vi) die Bereitstellung der indizierten DNA-Bibliothek durch die Durchführung der PCR-Indizierung an den adaptierten DNA-Fragmenten, wobei die adaptierten DNA-Fragmente amplifiziert werden, um indizierte PCR-Produkte unter Verwendung von indizierenden Vorwärts- und Rückwärtsprimern zu bilden, wobei entweder eine Reihenindex-Sequenz (Ri-Sequenz) oder eine Spaltenindex-Sequenz (Ci-Sequenz) durch jeden indizierenden Vorwärts- und Rückwärts-Primer an jedem Ende der adaptierten DNA-Fragmente eingeführt wird, so dass die resultierenden Produkte der PCR-Indizierung sowohl ein Paar flankierender Spaltenindex-Sequenzen (Ci-Sequenzen), die jeder Vertiefung einer Spalte gemeinsam sind, als auch ein Paar flankierender Reihenindex-Sequenzen (Ri-Sequenzen), die jeder Vertiefung einer Reihe gemeinsam sind, umfassen, und

vii) die Sequenzierung der indizierten DNA-Bibliothek, um Daten zur Bestimmung einzelner Nukleotidvarianten, zur Bestimmung der Chromosomenstrukturvariationen oder zur Bestimmung der Phaseninformation im Genom der einzelnen Zelle oder Zellgruppe zu erhalten.

2. Das Verfahren zur Sequenzierung des gesamten Genoms einer einzelnen Zelle oder Zellgruppe nach Anspruch 1, wobei geschlungene Adapter bereitgestellt werden, so dass das Verfahren einen Verfahrensschritt der Fragmentierung der DNA-Moleküle jeder Reaktionsvertiefung und eine anschließende Ligationsreaktion umfasst, um die eine Schleife bildenden Adapter an die fragmentierte DNA zu ligieren; oder wobei die Transposase-vermittelten Adapter so bereitgestellt werden, dass das Verfahren die Fragmentierung der DNA-Moleküle im Tagmentierungsprozess umfasst.

3. Das Verfahren zur Sequenzierung des gesamten Genoms einer einzelnen Zelle oder Zellgruppe nach Anspruch 1 oder 2, wobei nach der Fragmentierung der DNA zur Bildung von DNA-Fragmenten die Endreparatur und das dA-Tailing der DNA-Fragmente erfolgt, so dass sie an die eine Schleife bildenden Adapter ligiert werden können.

4. Das Verfahren zur Sequenzierung des gesamten Genoms einer einzelnen Zelle oder Zellgruppe nach einem der vorangegangenen Ansprüche, wobei die eine Schlaufe bildenden Adapter ein Oligonukleotid mit einer sekundären Stamm-Schleifen-Struktur umfassen, und wobei der eine Schlaufe bildende Adapter ein Paar komplementärer Sequenzregionen umfasst, die eine Schleifenregion flankieren, wobei das Paar komplementärer Sequenzen so angeordnet ist, dass sie miteinander hybridisieren, um die Stamm-Schleifen-Struktur des eine Schlaufe bildenden Adapters zu formen.

5. Das Verfahren zur Sequenzierung des gesamten Genoms einer einzelnen Zelle oder Zellgruppe nach einem der vorangegangenen Ansprüche, wobei die Ci-Sequenzen in den adaptierten DNA-Fragmenten bereitgestellt werden und das Verfahren zusätzlich den Verfahrensschritt der Poolbildung von adaptierten DNA-Fragmenten aus jeder Reaktionsvertiefung in einer Reihe vor der PCR-Indizierung umfassen kann; oder wobei die Ri-Sequenzen in den adaptierten DNA-Fragmenten vorgesehen sind, und das Verfahren zusätzlich den Verfahrensschritt der Poolbildung von adaptierten DNA-Fragmenten aus jeder Reaktionsvertiefung in einer Spalte vor der PCR-Indizierung umfasst.

6. Ein Verfahren zur Sequenzierung des gesamten Genoms einer einzelnen Zelle oder Zellgruppe nach einem der vorangegangenen Ansprüche, wobei die adaptierten DNA-Fragmente die Ci-Sequenzen umfassen und die Vorwärts- und Rückwärtsprimer für die PCR-Indizierung jeweils die Ri-Sequenzen umfassen, um ein Paar von Ri-Sequenzen im indizierten PCR-Produkt bereitzustellen.

7. Ein Verfahren zur Sequenzierung des gesamten Genoms einer einzelnen Zelle oder Zellgruppe nach einem der vorangegangenen Ansprüche, wobei die Vorwärts- und Rückwärtsprimer für die PCR-Indizierung ferner Sequenzen von Sequenzierungsadaptern umfassen, so dass die Sequenzierungsadapter in das indizierte PCR-Produkt eingebaut werden.

8. Ein Verfahren zur Sequenzierung des gesamten Genoms einer einzelnen Zelle oder Zellgruppe nach einem der vorangegangenen Ansprüche, wobei das Verfahren die Bestimmung aller realen SNVs im Genom der einzelnen Zelle oder Zellgruppe umfasst, indem bestimmt wird, ob substantiell alle indizierten DNA-Bibliothekssequenzen, die aus einer einzelnen Vertiefung stammen, die gleiche SNV umfassen, oder ob nur ein Bruchteil der indizierten DNA-Bibliothekssequenzen die gleiche SNV umfasst,

wobei eine SNV, die in substantiell allen indizierten DNA-Bibliothekssequenzen, die aus einer einzelnen Vertiefung stammen, vertreten ist, als eine echte SNV in der genomischen DNA bestimmt wird, und eine SNV, die nur in einem Bruchteil der indizierten DNA-Bibliothekssequenzen, die aus einer einzelnen Vertiefung stammen, gefunden wird, als eine falsch-positive (FP) SNV bestimmt wird.

9. Ein Verfahren zur Sequenzierung des gesamten Genoms einer einzelnen Zelle oder Zellgruppe nach einem der vorangegangenen Ansprüche, wobei das Verfahren ferner den Abgleich indizierter DNA-Bibliothekssequenzen, die aus einer einzelnen Vertiefung, die einen Strang der genomischen DNA repräsentiert, stammen, mit indizierten DNA-Bibliothekssequenzen, die aus einer anderen Vertiefung, die den komplementären Strang der genomischen DNA repräsentiert, stammen, umfasst

wobei eine SNV, die substantiell in allen indizierten DNA-Bibliothekssequenzen beider komplementärer Stränge der genomischen DNA vorhanden ist, als echte SNV bestimmt wird, und eine SNV, die nicht substantiell in allen indizierten DNA-Bibliothekssequenzen beider komplementärer Stränge der genomischen DNA vorhanden ist, als falsch positiv bestimmt wird.

10. Ein Verfahren zur Sequenzierung des gesamten Genoms einer einzelnen Zelle oder Zellgruppe nach Anspruch 8 oder 9, wobei die *in- silico*-Bestimmung unter Verwendung von BAM-Dateidaten durchgeführt wird, die aus dem Mapping der Sequenzdaten auf ein Referenzgenom erzeugt wurden, wobei die *in silico*-Bestimmungen oder die Anpassung indizierter DNA-Sequenzen und/oder die Berechnung von Wahrscheinlichkeitswerten durch ein Modell eines künstlichen neuronalen Netzes (ANN), gegebenenfalls durch ein mehrschichtiges Perzeptron, durchgeführt wird.

11. Ein Verfahren zur Sequenzierung des gesamten Genoms einer einzelnen Zelle oder Zellgruppe nach einem der vorangegangenen Ansprüche, wobei ein Wahrscheinlichkeitswert dafür, dass eine bestimmte Nukleotidvariante eine echte SNV oder eine falsch-positive Variante ist, *in silico* berechnet wird, so dass bestimmt wird, dass ein bestimmtes Nukleotid eine statistisch signifikante Wahrscheinlichkeit hat, eine echte SNV oder eine falsch-positive Variante zu sein.

12. Ein Verfahren zur Sequenzierung des gesamten Genoms einer einzelnen Zelle oder Zellgruppe nach einem der vorangegangenen Ansprüche, wobei die Sequenzdaten in Form von Paired-Read-FastQ-Dateien bereitgestellt werden, wobei die Sequenzdaten der Paired-Read-FastQ-Dateien optional auf die Entfernung von Adaptersequenzen und auf Qualität gekürzten werden, um gekürzte Daten bereitzustellen.

13. Ein Verfahren zur Herstellung einer indizierten DNA-Bibliothek zur Sequenzierung von Nukleinsäuremolekülen umfassend:

i) die Bereitstellung einer Multiwell-Testplatte, die Reihen und Spalten von Reaktionsvertiefungen umfasst;
ii) die Bereitstellung von Nukleinsäuremolekülen, wobei die Nukleinsäuremoleküle in eine Vielzahl von Reaktionsvertiefungen auf der Multiwell-Testplatte verteilt werden, so dass es nicht mehr als ein einzelsträngiges Nukleinsäuremolekül eines beliebigen Locus pro Reaktionsvertiefung vorhanden ist,
iii) die Durchführung der Amplifikation des Nukleinsäuremoleküls, um mehrere DNA-Kopien des Nukleinsäuremoleküls in jeder Reaktionsvertiefung zu erhalten;
iv) die Fragmentierung der DNA-Moleküle jeder Reaktionsvertiefung und die Ligation eines Paares von geschlungenen Adaptern an jedem Ende oder die Tagmentierung unter Verwendung der Transposase-vermittelten Adaptern, um angepasste DNA-Fragmente zu bilden, wobei die geschlungenen Adapter oder die Transposase-vermittelten Adapter entweder eine Spaltenindex-Sequenz (Ci-Sequenz) oder eine Reihenindex-Sequenz (Ri-Sequenz) umfassen, wobei die Ci-Sequenz jedem Schleifenadapter oder Transposase-vermittelten Adapter jeder Reaktionsvertiefung in einer Spalte der Multiwell-Testplatte gemeinsam ist, oder wobei jede Ri-Sequenz jedem Schleifenadapter oder Transposase-vermittelten Adapter jeder Reaktionsvertiefung in einer Reihe der Multiwell-Testplatte gemeinsam ist;
vi) die Bereitstellung der indizierten DNA-Bibliothek durch die Durchführung der PCR-Indizierung an den adaptierten DNA-Fragmenten, wobei die adaptierten DNA-Fragmente amplifiziert werden, um indizierte PCR-Produkte unter Verwendung von indizierenden Vorwärts- und Rückwärts-Primern zu bilden, wobei entweder eine Reihenindex-Sequenz (Ri-Sequenz) oder eine Spaltenindex-Sequenz (Ci-Sequenz) durch jeden indizierenden Vorwärts- und Rückwärts-Primer an jedem Ende der adaptierten DNA-Fragmente eingeführt wird, so dass die resultierenden Produkte der PCR-Indizierung sowohl ein Paar flankierender Spaltenindex-Sequenzen (Ci-Sequenzen), die jeder Vertiefung einer Spalte gemeinsam sind, als auch ein Paar flankierender Reihenindex-Sequenzen (Ri-Sequenzen), die jeder Vertiefung einer Reihe gemeinsam sind, umfassen, und
wobei die Vorwärts- und Rückwärtsprimer für die Indizierung ferner jeweilige 5'- und 3'-Sequenzierungsadapter an den indizierten PCR-Produkten bereitstellen, die für die Verwendung in einer Sequenzierungsreaktion geeignet sind.

14. Ein Verfahren zur Bereitstellung einer DNA-Bibliothek für das gesamte Genom einzelner Zellen oder Zellgruppen zur Identifizierung einzelner Nukleotidvarianten, zur Bestimmung der Chromosomenstrukturvariationen oder zur Bestimmung der Phaseninformation im Genom der einzelnen Zellen oder Zellgruppen, wobei das Verfahren umfasst:

i) die Bereitstellung einer Multiwell-Testplatte, die Reihen und Spalten von Reaktionsvertiefungen umfasst;
ii) die Bereitstellen genomischer DNA von Einzelzellen oder Zellgruppen, wobei die genomische DNA in eine Vielzahl von Reaktionsvertiefungen auf der Multiwell-Testplatte verteilt wird, so dass nicht mehr als ein einzelsträngiges genomisches DNA-Molekül eines beliebigen Locus pro Reaktionsvertiefung vorhanden ist,
iii) die Durchführung der Ganzgenom-Amplifikation (WGA) jedes genomischen DNA-Moleküls, um mehrere Kopien des genomischen DNA-Moleküls in jedem Reaktionsvertiefung zu erhalten;
iv) die Fragmentierung der DNA-Moleküle jeder Reaktionsvertiefung und die Ligation eines Paares von geschlungenen Adaptern an jedem Ende oder die Tagmentierung unter Verwendung der Transposase-vermittelten Adaptern, um angepasste DNA-Fragmente zu bilden, wobei die geschlungenen Adapter oder die Transposase-vermittelten Adapter entweder eine Spaltenindex-Sequenz (Ci-Sequenz) oder eine Reihenindex-Sequenz (Ri-Sequenz) umfassen, wobei die Ci-Sequenz jedem Schleifenadapter oder Transposase-vermittelten Adapter jeder Reaktionsvertiefung in einer Spalte der Multiwell-Testplatte gemeinsam ist, oder wobei jede Ri-Sequenz jedem Schleifenadapter oder Transposase-vermittelten Adapter jeder Reaktionsvertiefung in einer Reihe der Multiwell-Testplatte gemeinsam ist;
vi) die Bereitstellung der indizierten DNA-Bibliothek durch die Durchführung der PCR-Indizierung an den adaptierten DNA-Fragmenten, wobei die adaptierten DNA-Fragmente amplifiziert werden, um indizierte PCR-Produkte unter Verwendung von indizierenden Vorwärts- und Rückwärts-Primern zu bilden, wobei entweder eine Reihenindex-Sequenz (Ri-Sequenz) oder eine Spaltenindex-Sequenz (Ci-Sequenz) durch jeden indizierenden Vorwärts- und Rückwärts-Primer an jedem Ende der adaptierten DNA-Fragmente eingeführt wird, so dass die resultierenden Produkte der PCR-Indizierung sowohl ein Paar flankierender Spaltenindex-Sequenzen (Ci-Sequenzen), die jeder Vertiefung einer Spalte gemeinsam sind, als auch ein Paar flankierender Reihenindex-Sequenzen (Ri-Sequenzen), die jeder Vertiefung einer Reihe gemeinsam sind, umfassen, und
wobei die Vorwärts- und Rückwärtsprimer für die Indizierung ferner jeweilige 5'- und 3'-Sequenzierungsadapter an den indizierten PCR-Produkten bereitstellen, die für die Verwendung in einer Sequenzierungsreaktion geeignet sind.

15. Ein Verfahren zur Sequenzierung des gesamten Genoms einer einzelnen Zelle oder Zellgruppe zur Bereitstellung von Daten zur Identifizierung einzelner Nukleotidvarianten (SNVs), zur Bestimmung der Chromosomenstrukturvariationen oder zur Bestimmung der Phaseninformation im Genom der einzelnen Zelle oder Zellgruppe, wobei das Verfahren umfasst:

i) die Bereitstellung einer indizierten DNA-Bibliothek, indem das Verfahren nach Anspruch 13 oder 14 durchgeführt wird, oder die Bereitstellung einer indizierten DNA-Bibliothek, die gemäß Anspruch 13 oder 14 hergestellt wurde;
ii) die Sequenzierung der indizierten DNA-Bibliothek, um Daten zur Bestimmung einzelner Nukleotidvarianten (SNVs), zur Bestimmung der Chromosomenstrukturvariationen oder zur Bestimmung der Phaseninformation im Genom der einzelnen Zelle oder Zellgruppe zu erhalten.

**Revendications**

1. Procédé de séquençage du génome entier d'une cellule unique ou d'un groupe de cellules pour l'identification de variants nucléotidiques uniques, la détermination de variations structurelles chromosomiques ou la détermination d'informations de phasage dans le génome de la cellule unique ou du groupe de cellules, le procédé comprenant :

i) la fourniture d'une plaque matrice multipuits comprenant des rangées et des colonnes de puits de réaction ;
ii) la fourniture de l'ADN génomique de cellules uniques ou de groupes de cellules, dans lequel l'ADN génomique est distribué dans une pluralité de puits de réaction sur la plaque matrice multipuits, de sorte qu'il n'y a pas plus d'une molécule d'ADN génomique simple brin d'un locus donné par puits de réaction,

iii) la réalisation d'une amplification du génome entier (WGA) de chaque molécule d'ADN génomique pour fournir de multiples copies de la molécule d'ADN génomique dans chaque puits de réaction ;

iv) la fragmentation des molécules d'ADN de chaque puits de réaction et la ligature d'une paire d'adaptateurs en boucle à chaque extrémité ou la tagmentation à l'aide d'adaptateurs délivrés par la transposase pour former des fragments d'ADN adapté, dans lequel les adaptateurs en boucle ou les adaptateurs délivrés par la transposase comprennent soit une séquence d'index de colonne (Ci) soit une séquence d'index de rangée (Ri), dans lequel la séquence Ci est commune à chaque adaptateur en boucle ou adaptateur délivré par transposase de chaque puits de réaction dans une colonne de la plaque matrice multipuits, ou dans lequel chaque séquence Ri est commune à chaque adaptateur en boucle ou adaptateur délivré par transposase de chaque puits de réaction dans une rangée de la plaque matrice multipuits ;

vi) la fourniture de la bibliothèque d'ADN indexée en exécutant une PCR d'indexation sur les fragments d'ADN adapté, dans lequel les fragments d'ADN adapté sont amplifiés pour former des produits de PCR indexés à l'aide d'amorces d'indexation directe et inverse, dans lequel soit une séquence d'index de rangée (Ri) soit une séquence d'index de colonne (Ci) est introduite par chaque amorce d'indexation directe et inverse sur chaque extrémité des fragments d'ADN adapté, de sorte que les produits de PCR indexés résultants comprennent à la fois une paire de séquences d'index de colonne (Ci) flanquantes qui sont communes à chaque puits d'une colonne et une paire de séquences d'index de rangée (Ri) flanquantes qui sont communes à chaque puits d'une rangée, et

vii) le séquençage de la bibliothèque d'ADN indexée pour fournir des données permettant de déterminer des variants nucléotidiques uniques, de déterminer des variations structurelles chromosomiques ou de déterminer des informations de phasage dans le génome de la cellule unique ou du groupe de cellules.

2. Procédé de séquençage du génome entier d'une cellule unique ou d'un groupe de cellules selon la revendication 1, dans lequel des adaptateurs en boucle sont fournis, de sorte que le procédé comprend une étape de fragmentation des molécules d'ADN de chaque puits de réaction et une réaction de ligature ultérieure pour ligaturer des adaptateurs en boucle à l'ADN fragmenté ; ou
dans lequel les adaptateurs délivrés par la transposase sont fournis de sorte que le procédé comprend la fragmentation des molécules d'ADN par le processus de tagmentation.

3. Procédé de séquençage du génome entier d'une cellule unique ou d'un groupe de cellules selon la revendication 1 ou 2, dans lequel, après la fragmentation de l'ADN pour former des fragments d'ADN, les fragments d'ADN sont réparés aux extrémités et possèdent une queue dA, de sorte qu'ils peuvent être ligaturés aux adaptateurs en boucle.

4. Procédé de séquençage du génome entier d'une cellule unique ou d'un groupe de cellules selon une quelconque revendication précédente, dans lequel les adaptateurs en boucle comprennent un oligonucléotide ayant une structure tige-boucle secondaire, et dans lequel l'adaptateur en boucle comprend une paire de régions de séquences complémentaires flanquant une région de boucle, dans lequel la paire de séquences complémentaires sont agencées pour s'hybrider l'une avec l'autre pour former la structure tige-boucle de l'adaptateur en boucle.

5. Procédé de séquençage du génome entier d'une cellule unique ou d'un groupe de cellules selon une quelconque revendication précédente, dans lequel des séquences Ci sont fournies dans les fragments d'ADN adapté, et le procédé peut en outre comprendre l'étape de regroupement des fragments d'ADN adapté de chaque puits de réaction dans une rangée avant la PCR d'indexation ; ou
dans lequel des séquences Ri sont fournies dans les fragments d'ADN adapté, et le procédé comprenant en outre l'étape de regroupement des fragments d'ADN adapté de chaque puits de réaction dans une colonne avant la PCR d'indexation.

6. Procédé de séquençage du génome entier d'une cellule unique ou d'un groupe de cellules selon une quelconque revendication précédente, dans lequel les fragments d'ADN adapté comprennent des séquences Ci, et les amorces PCR d'indexation directe et inverse comprennent chacune des séquences Ri, pour fournir une paire de séquences Ri dans le produit PCR indexé.

7. Procédé de séquençage du génome entier d'une cellule unique ou d'un groupe de cellules selon une quelconque revendication précédente, dans lequel les amorces PCR d'indexation directe et inverse comprennent en outre des séquences d'adaptateurs de séquençage, de sorte que les adaptateurs de séquençage sont incorporés dans le produit PCR indexé.

8. Procédé de séquençage du génome entier d'une cellule unique ou d'un groupe de cellules selon une quelconque revendication précédente, dans lequel le procédé comprend la détermination de tout SNV réel dans le génome de la cellule unique ou du groupe de cellules en déterminant si sensiblement toutes les séquences de bibliothèque d'ADN indexées provenant d'un puits unique comprennent le même SNV, ou si seulement une fraction des séquences de bibliothèque d'ADN indexées comprend le même SNV,

dans lequel un SNV représenté dans sensiblement toutes les séquences de bibliothèque d'ADN indexées provenant d'un puits unique est déterminé comme étant un SNV réel dans l'ADN génomique, et un SNV trouvé dans seulement une fraction des séquences de bibliothèque d'ADN indexées provenant d'un puits unique est déterminé comme étant un SNV faussement positif (FP).

9. Procédé de séquençage du génome entier d'une cellule unique ou d'un groupe de cellules selon une quelconque revendication précédente, dans lequel le procédé comprend en outre la mise en correspondance de séquences de bibliothèque d'ADN indexées provenant d'un puits unique représentant un brin de l'ADN génomique avec des séquences de bibliothèque d'ADN indexées provenant d'un autre puits représentant le brin complémentaire de l'ADN génomique,

dans lequel un SNV sensiblement présent dans toutes les séquences de bibliothèque d'ADN indexées des deux brins complémentaires de l'ADN génomique est déterminé comme étant un SNV réel, et un SNV non sensiblement présent dans toutes les séquences de bibliothèque d'ADN indexées des deux brins complémentaires de l'ADN génomique est déterminé comme étant un faux positif.

10. Procédé de séquençage du génome entier d'une cellule unique ou d'un groupe de cellules selon la revendication 8 ou 9, dans lequel la détermination est effectuée *in silico* à l'aide de données de fichier BAM qui ont été générées à partir du mappage des données de séquence sur un génome de référence, éventuellement dans lequel les déterminations *in silico* ou la mise en correspondance de séquences d'ADN indexées et/ou le calcul de scores de probabilité sont effectués par un modèle de réseau neuronal artificiel (ANN), éventuellement par un perceptron multicouche.

11. Procédé de séquençage du génome entier d'une cellule unique ou d'un groupe de cellules selon une quelconque revendication précédente, dans lequel un score de probabilité qu'un variant nucléotidique particulier soit un SNV réel ou un faux positif est calculé *in silico,* de sorte qu'un nucléotide variant donné est déterminé comme ayant une probabilité statistiquement significative d'être un SNV réel ou un faux positif.

12. Procédé de séquençage du génome entier d'une cellule unique ou d'un groupe de cellules selon une quelconque revendication précédente, dans lequel les données de séquence sont fournies sous la forme de fichiers FastQ à lecture paire, éventuellement dans lequel les données de séquence des fichiers FastQ à lecture paire sont découpées pour suppression de séquences d'adaptateurs et pour la qualité, pour fournir des données découpées.

13. Procédé de préparation d'une bibliothèque d'ADN indexée pour le séquençage de molécules d'acide nucléique, le procédé comprenant :

i) la fourniture d'une plaque matrice multipuits comprenant des rangées et des colonnes de puits de réaction ;
ii) la fourniture de molécules d'acide nucléique, dans lequel les molécules d'acide nucléique sont distribuées dans une pluralité de puits de réaction sur la plaque matrice multipuits, de sorte qu'il n'y a pas plus d'une molécule d'acide nucléique simple brin provenant d'un locus donné par puits de réaction,
iii) la réalisation d'une amplification de la molécule d'acide nucléique pour fournir de multiples copies d'ADN de la molécule d'acide nucléique dans chaque puits de réaction ;
iv) la fragmentation des molécules d'ADN de chaque puits de réaction et la ligature d'une paire d'adaptateurs en boucle à chaque extrémité ou la tagmentation à l'aide d'adaptateurs délivrés par la transposase pour former des fragments d'ADN adapté, dans lequel les adaptateurs en boucle ou les adaptateurs délivrés par la transposase comprennent soit une séquence d'index de colonne (Ci) soit une séquence d'index de rangée (Ri), dans lequel la séquence Ci est commune à chaque adaptateur en boucle ou adaptateur délivré par transposase de chaque puits de réaction dans une colonne de la plaque matrice multipuits, ou dans lequel chaque séquence Ri est commune à chaque adaptateur en boucle ou adaptateur délivré par transposase de chaque puits de réaction dans une rangée de la plaque matrice multipuits ;
vi) la fourniture de la bibliothèque d'ADN indexée en exécutant une PCR d'indexation sur les fragments d'ADN adapté, dans lequel les fragments d'ADN adapté sont amplifiés pour former des produits de PCR indexés à l'aide d'amorces d'indexation directe et inverse, dans lequel soit une séquence d'index de rangée (Ri) soit une séquence d'index de colonne (Ci) est introduite par chaque amorce d'indexation directe et inverse sur chaque extrémité des fragments d'ADN adapté, de sorte que les produits de PCR indexés résultants comprennent à la fois une paire de séquences d'index de colonne (Ci) flanquantes qui sont communes à chaque puits d'une colonne et une paire de séquences d'index de rangée (Ri) flanquantes qui sont communes à chaque puits d'une rangée, et

éventuellement dans lequel les amorces d'indexation directe et inverse fournissent en outre des adaptateurs de séquençage respectifs 5' et 3' sur les produits PCR indexés qui sont appropriés pour une utilisation dans une réaction de séquençage.

**14.** Procédé de préparation d'une bibliothèque d'ADN indexée pour le séquençage du génome entier de cellules uniques ou de groupes de cellules pour l'identification de variants nucléotidiques uniques, la détermination de variations structurelles chromosomiques ou la détermination d'informations de phasage dans le génome de cellules uniques ou de groupes de cellules, le procédé comprenant :

i) la fourniture d'une plaque matrice multipuits comprenant des rangées et des colonnes de puits de réaction ;
ii) la fourniture de l'ADN génomique de cellules uniques ou de groupes de cellules, dans lequel l'ADN génomique est distribué dans une pluralité de puits de réaction sur la plaque matrice multipuits, de sorte qu'il n'y a pas plus d'une molécule d'ADN génomique simple brin d'un locus donné par puits de réaction,
iii) la réalisation d'une amplification du génome entier (WGA) de chaque molécule d'ADN génomique pour fournir de multiples copies de la molécule d'ADN génomique dans chaque puits de réaction ;
iv) la fragmentation des molécules d'ADN de chaque puits de réaction et la ligature d'une paire d'adaptateurs en boucle à chaque extrémité ou la tagmentation à l'aide d'adaptateurs délivrés par la transposase pour former des fragments d'ADN adapté, dans lequel les adaptateurs en boucle ou les adaptateurs délivrés par la transposase comprennent soit une séquence d'index de colonne (Ci) soit une séquence d'index de rangée (Ri), dans lequel la séquence Ci est commune à chaque adaptateur en boucle ou adaptateur délivré par transposase de chaque puits de réaction dans une colonne de la plaque matrice multipuits, ou dans lequel chaque séquence Ri est commune à chaque adaptateur en boucle ou adaptateur délivré par transposase de chaque puits de réaction dans une rangée de la plaque matrice multipuits ;
vi) la fourniture de la bibliothèque d'ADN indexée en exécutant une PCR d'indexation sur les fragments d'ADN adapté, dans lequel les fragments d'ADN adapté sont amplifiés pour former des produits de PCR indexés à l'aide d'amorces d'indexation directe et inverse, dans lequel soit une séquence d'index de rangée (Ri) soit une séquence d'index de colonne (Ci) est introduite par chaque amorce d'indexation directe et inverse sur chaque extrémité des fragments d'ADN adapté, de sorte que les produits de PCR indexés résultants comprennent à la fois une paire de séquences d'index de colonne (Ci) flanquantes qui sont communes à chaque puits d'une colonne et une paire de séquences d'index de rangée (Ri) flanquantes qui sont communes à chaque puits d'une rangée, et

éventuellement dans lequel les amorces d'indexation directe et inverse fournissent en outre des adaptateurs de séquençage respectifs 5' et 3' sur les produits PCR indexés qui sont appropriés pour une utilisation dans une réaction de séquençage.

**15.** Procédé de séquençage du génome entier d'une cellule unique ou d'un groupe de cellules pour fournir des données permettant l'identification de variants nucléotidiques uniques (SNV), la détermination de variations structurelles chromosomiques ou la détermination d'informations de phasage dans le génome de la cellule unique ou du groupe de cellules, le procédé comprenant :

i) la préparation d'une bibliothèque d'ADN indexée en mettant en œuvre le procédé selon la revendication 13 ou 14, ou la fourniture d'une bibliothèque d'ADN indexée préparée selon la revendication 13 ou 14 ;
ii) le séquençage de la bibliothèque d'ADN indexée pour fournir des données permettant de déterminer des variants nucléotidiques uniques (SNV), de déterminer des variations structurelles chromosomiques ou de déterminer des informations de phasage dans le génome de la cellule unique ou du groupe de cellules.

Figure 1A

Figure 1B

LCM

Lysis &
Partitioning

1        2              383      384

WGA &
Fragmentation

Adapter ligation
& Indexing

Pooling, Purification & Sequencing

Figure 1C

Figure 1D

Figure 1E

Figure 1F

Figure 1G

Figure 1H

Figure 1I

EP 4 073 264 B1

Figure 1J

EP 4 073 264 B1

Figure 2A

Figure 2B

without clone-specific mutations

with clone-specific mutations

Model over-fitted on UTDs

Dimension 2

Dimension 1

Dimension 2

Dimension 1

Real mutations    Artefactual mutations

◯ Real mutations  ✦ Artefactual mutations | 1 labels (SNPs) | 0 labels (UTDs) |

Figure 2C

First training

Second training

Final MutLX results

Dimension 2

Dimension 1

Dimension 2

Dimension 1

Probability Score

Uncertainty Score

◯ Real mutations ✦ Artefactual mutations | 1 labels (SNPs) | 0 labels (UTDs) | Not used in training |

Figure 2D

Figure 2E

EP 4 073 264 B1

Figure 2F

Figure 2G

Figure 3A

Figure 3B

66,521,150 bp    66,521,160 bp    66,521,170 bp

T G A T C G C T T C C G A G C A A T A A G A A

66,529,340 bp    66,529,350 bp    66,529,360 bp

C C T T A T T T C T G A T G C T C T T A G A T

66,880,950 bp    66,880,960 bp    66,880,970 bp

G T A T C A G T C A G C C A G A A A A A A G G

Figure 3C

Figure 4

Figure 5

MutLX

Classification Model

Improved Training set

Initial Model

Primary Training set

Clone-specific Variants

Probability Score

Uncertainty Score

Het. SNPs

All UTDs

WGS Data

DigiPico Data

Figure 6

Figure 7

Figure 8

Figure 9A

EP 4 073 264 B1

Figure 9B

| Position | Mutation | Total Well Coverage | VAWF (%) | % Reads supporting the variant | |
|---|---|---|---|---|---|
| | | | | Blood gDNA | PT2R gDNA |
| chr1:43050977 | C>T | 45 | 33.33% | 0.03 | 0.22 |
| chr1:84044398 | T>C | 14 | 78.57% | 0.02 | 0.85 |
| chr1:168274409 | C>G | 15 | 40.00% | 0.33 | 0.49 |
| chr2:33010682 | A>G | 36 | 58.33% | 0.00 | 0.77 |
| chr2:123789390 | C>T | 28 | 46.43% | 0.00 | 0.27 |
| chr3:121522102 | C>G | 46 | 43.47% | 0.19 | 0.30 |
| chr3:129524269 | G>A | 20 | 45.00% | 0.00 | 0.31 |
| chr4:76733361 | C>T | 11 | 27.27% | 0.03 | 0.03 |
| chr4:167932516 | T>C | 33 | 39.40% | 0.02 | 0.09 |
| chr6:73146508 | C>G | 33 | 45.45% | 0.12 | 0.12 |
| chr15:59093127 | G>A | 18 | 61.00% | 0.00 | 0.85 |
| chr17:66520311 | C>T | 18 | 38.89% | 0.06 | 0.46 |
| chr17:66521158 | C>T | 22 | 31.82% | 0.01 | 0.09 |
| chr17:66523728 | C>T | 33 | 39.39% | 0.02 | 0.35 |

Figure 10

| Position | Mutation | Total Well Coverage | VAWF (%) | % Reads supporting the variant | |
| --- | --- | --- | --- | --- | --- |
| | | | | Ascites gDNA | Blood gDNA |
| chr1:198871416 | C>G | 11 | 54.55 | 0.00 | 0.00 |
| chr1:205392201 | G>A | 26 | 38.46 | 0.02 | 0.00 |
| chr1:212152502 | C>A | 28 | 21.43 | 0.04 | 0.03 |
| chr2:165954947 | C>A | 14 | 42.86 | 0.01 | 0.02 |
| chr3:55936181 | C>A | 14 | 78.57 | 0.02 | 0.05 |
| chr3:80372726 | C>A | 9 | 66.67 | 0.01 | 0.02 |
| chr3:148085551 | C>T | 10 | 40.00 | 0.00 | 0.01 |
| chr3:166385800 | G>A | 15 | 46.67 | 0.00 | 0.01 |
| chr4:21723721 | C>T | 8 | 75.00 | 0.02 | 0.00 |
| chr4:114452967 | A>C | 16 | 68.75 | 0.00 | 0.00 |
| chr4:187590704 | G>T | 9 | 88.89 | 0.02 | 0.00 |
| chr5:24745295 | C>A | 11 | 36.36 | 0.01 | 0.01 |
| chr6:29409126 | C>T | 21 | 33.33 | 0.01 | 0.02 |
| chr6:89320707 | C>A | 7 | 57.14 | 0.01 | 0.02 |
| chr6:120042047 | C>A | 12 | 33.33 | 0.00 | 0.00 |
| chr7:11409022 | C>T | 9 | 55.56 | 0.01 | 0.03 |
| chr7:16023731 | C>A | 21 | 71.43 | 0.07 | 0.02 |
| chr7:140434437 | G>T | 28 | 39.29 | 0.01 | 0.00 |
| chr8:28991687 | G>T | 31 | 25.81 | 0.02 | 0.03 |
| chr8:47633895 | G>T | 25 | 16.00 | 0.02 | 0.02 |
| chr8:56078651 | G>T | 24 | 20.83 | 0.01 | 0.00 |
| chr8:133086518 | A>T | 16 | 31.25 | 0.03 | 0.01 |
| chr8:134979562 | G>T | 11 | 36.36 | 0.04 | 0.07 |
| chr11:5653934 | C>A | 26 | 19.23 | 0.01 | 0.03 |
| chr11:119730031 | C>T | 43 | 13.95 | 0.01 | 0.00 |
| chr12:128816427 | G>T | 9 | 55.56 | 0.01 | 0.02 |
| chr13:80467921 | G>T | 8 | 62.50 | 0.03 | 0.01 |
| chr15:34394338 | G>A | 10 | 60.00 | 0.01 | 0.01 |
| chr15:81086223 | C>T | 28 | 78.57 | 0.02 | 0.01 |
| chr16:80389783 | G>T | 11 | 72.73 | 0.04 | 0.02 |
| chr17:10440734 | C>A | 15 | 26.67 | 0.03 | 0.01 |
| chr17:57290575 | T>C | 28 | 35.71 | 0.00 | 0.00 |
| chr18:24891922 | C>A | 21 | 76.19 | 0.06 | 0.01 |
| chr18:34756888 | G>T | 23 | 78.26 | 0.04 | 0.05 |
| chr18:52260039 | G>T | 20 | 75.00 | 0.02 | 0.01 |
| chr21:40855807 | T>A | 10 | 70.00 | 0.01 | 0.01 |
| chr22:25928538 | A>T | 15 | 73.33 | 0.00 | 0.02 |

Figure 11

Distribution of various mutation types in true vs false positive variant calls

◇ True somatic variants

◆ False positive calls identified by MutLX

Relative Frequency

A>C   A>G   A>T   C>A   C>G   C>T

Figure 12

Figure 13

EP 4 073 264 B1

Figure 13 continued

Figure 13 continued

EP 4 073 264 B1

Figure 13 continued

Figure 13 continued

Figure 13 continued

Figure 13 continued

Figure 13 continued

Figure 14A

Figure 14B

Figure 15A

Figure 15B

DigiPico

DigiPico 2

Normalized RFU

Figure 16A

Index Frequency

Figure 16B

EP 4 073 264 B1

70

Figure 16C

Figure 16D

Figure 17A

EP 4 073 264 B1

Figure 17B

Figure 17C

Comparison of SNV calling accuracy in DigiPico vs LFR method

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018152129 A1

### Non-patent literature cited in the description

- **PETERS BA et al.** Accurate whole-genome sequencing and haplotyping from 10 to 20 human cells. *Nature,* 11 July 2012, vol. 487 (7406), 190-5
- **SHO SHONAN et al.** *BMC CANCER,* vol. 17 (1
- **TURAJLIC,S ; SOTTORIVA,A ; GRAHAM,T. ; SWANTON,C.** Resolving genetic heterogeneity in cancer. *Nat. Rev. Genet.,* 2019
- **ZHANG,J. ; SPÄTH,S.S ; MARJANI,S.L ; ZHANG,W. ; PAN,X.** Characterization of cancer genomic heterogeneity by next-generation sequencing advances precision medicine in cancer treatment. *Precis. Clin. Med.,* 2018, vol. 1, 29-48
- **GERSTUNG,M. ; JOLLY,C ; LESHCHINER,I ; DENTRO,S.C ; GONZALEZ,S ; MITCHELL,T.J ; RUBANOVA,Y ; ANUR,P ; ROSEBROCK,D ; YU,K. et al.** The evolutionary history of 2,658 cancers. *bioRxiv,* 2017
- **BARBER,L.J ; DAVIES,M.N. ; GERLINGER,M.** Dissecting cancer evolution at the macro-heterogeneity and micro-heterogeneity scale. *Curr. Opin. Genet. Dev,* 2015, vol. 30, 1-6
- **BOHRSON,C.L ; BARTON,A.R ; LODATO,M.A ; RODIN,R.E ; LUQUETTE,L.J ; VISWANADHAM,V. V ; GULHAN,D.C ; CORTES-CIRIANO,I ; SHERMAN,M.A ; KWON,M. et al.** Linked-read analysis identifies mutations in single-cell DNA-sequencing data. *Nat. Genet.,* 2019
- **CHEN,L ; LIU,P ; EVANS,T.C.J ; ETTWILLER,L.M.** DNA damage is a pervasive cause of sequencing errors, directly confounding variant identification. *Science,* 2017, vol. 355, 752-756
- **COSTELLO,M. ; PUGH,T.J ; FENNELL,T.J. ; STEWART,C ; LICHTENSTEIN,L. ; MELDRIM,J.C. ; FOSTEL,J.L. ; FRIEDRICH,D.C. ; PERRIN,D ; DIONNE,D et al.** Discovery and characterization of artifactual mutations in deep coverage targeted capture sequencing data due to oxidative DNA damage during sample preparation. *Nucleic Acids Res.,* 2013, vol. 41, e67

- **NIK-ZAINAL,S ; ALEXANDROV,L.B ; WEDGE,D.C. ; VAN LOO,P. ; GREENMAN,C.D ; RAINE,K. ; JONES,D. ; HINTON,J ; MARSHALL,J ; STEBBINGS,L.A et al.** Mutational processes molding the genomes of 21 breast cancers. *Cell,* 2012, vol. 149, 979-993
- **MARTINCORENA,I. ; FOWLER,J.C. ; WABIK,A. ; LAWSON,A.R.J ; ABASCAL,F. ; HALL,M.W.J ; CAGAN,A. ; MURAI,K. ; MAHBUBANI,K ; STRATTON,M.R et al.** Somatic mutant clones colonize the human esophagus with age. *Science,* 2018, vol. 362, 911-917
- **TUBBS,A ; NUSSENZWEIG,A.** Endogenous DNA Damage as a Source of Genomic Instability in Cancer. *Cell,* 2017, vol. 168, 644-656
- **DONG,X. ; ZHANG,L. ; MILHOLLAND,B ; LEE,M. ; MASLOV,A.Y ; WANG,T ; VIJG,J.** Accurate identification of single-nucleotide variants in whole-genome-amplified single cells. *Nat. Methods,* 2017, vol. 14, 491-493
- **ZAFAR,H. ; WANG,Y. ; NAKHLEH,L. ; NAVIN,N. ; CHEN,K.** Monovar: single-nucleotide variant detection in single cells. *Nat. Methods,* 2016, vol. 13, 505-507
- **CHEN,C. ; XING,D. ; TAN,L. ; LI,H ; ZHOU,G ; HUANG,L. ; XIE,X.S.** Single-cell whole-genome analyses by Linear Amplification via Transposon Insertion (LIANTI). *Science,* 2017, vol. 356, 189-194
- **KRUEGER F.** *Trim Galore!,* 2016
- **LANGMEAD,B. ; SALZBERG,S.L.** Fast gapped-read alignment with Bowtie 2. *Nat Meth,* 2012, vol. 9, 357-359
- **MCKENNA,A. ; HANNA,M ; BANKS,E ; SIVACHENKO,A ; CIBULSKIS,K. ; KERNYTSKY,A ; GARIMELLA,K. ; ALTSHULER,D ; GABRIEL,S. ; DALY,M et al.** The Genome Analysis Toolkit: a MapReduce framework for analyzing next-generation DNA sequencing data. *Genome Res.,* 2010, vol. 20, 1297-1303

- **KIM,S ; SCHEFFLER,K. ; HALPERN,A.L ; BEKRITSKY,M.A ; NOH,E ; KÄLLBERG,M ; CHEN,X ; KIM,Y. ; BEYTER,D ; KRUSCHE,P et al.** Strelka2: fast and accurate calling of germline and somatic variants. *Nat. Methods,* 2018, vol. 15, 591-594
- **HOSOKAWA,M ; NISHIKAWA,Y. ; KOGAWA,M ; TAKEYAMA,H.** Massively parallel whole genome amplification for single-cell sequencing using droplet microfluidics. *Sci. Rep,* 2017, vol. 7, 5199
- **PETERS,B.A ; KERMANI,B.G ; SPARKS,A.B. ; ALFEROV,O. ; HONG,P ; ALEXEEV,A ; JIANG,Y ; DAHL,F ; TANG,Y.T. ; HAAS,J. et al.** Accurate whole-genome sequencing and haplotyping from 10 to 20 human cells. *Nature,* 2012, vol. 487, 190-195
- *Picard Tools,* 2018
- **RIMMER,A. ; PHAN,H. ; MATHIESON,I. ; IQBAL,Z. ; TWIGG,S.R.F ; WILKIE,A.O.M ; MCVEAN,G ; LUNTER,G.** Integrating mapping-, assembly- and haplotype-based approaches for calling variants in clinical sequencing applications. *Nat. Genet,* 2014, vol. 46, 912-918
- **DERRIEN,T. ; ESTELLÉ,J. ; MARCO SOLA,S ; KNOWLES,D.G. ; RAINERI,E ; GUIG6,R ; RIBECA,P.** Fast computation and applications of genome mappability. *PLoS One,* 2012, vol. 7, e30377-e30377
- **DANECEK,P. ; AUTON,A. ; ABECASIS,G ; ALBERS,C.A ; BANKS,E. ; DEPRISTO,M.A ; HANDSAKER,R.E ; LUNTER,G ; MARTH,G.T ; SHERRY,S.T. et al.** The variant call format and VCFtools. *Bioinformatics,* 2011, vol. 27, 2156-2158
- **CHOLLET,F.** *Keras,* 2015
- **KINGMA,D.P. ; BA,J.** Adam: A Method for Stochastic Optimization. *CoRR,* 2014
- **GAL,Y. ; GHAHRAMANI,Z.** Dropout as a Bayesian Approximation: Representing Model Uncertainty in Deep Learning. *arXiv e-prints,* 2015
- **DRMANAC,R ; SPARKS,A.B ; CALLOW,M.J ; HALPERN,A.L ; BURNS,N.L. ; KERMANI,B.G. ; CARNEVALI,P ; NAZARENKO,I. ; NILSEN,G.B ; YEUNG,G et al.** Human Genome Sequencing Using Unchained Base Reads on Self-Assembling DNA Nanoarrays. *Science,* 2010, vol. 80, 327
- **ARBEITHUBER,B. ; MAKOVA,K.D. ; TIEMANN-BOEGE,I.** Artifactual mutations resulting from DNA lesions limit detection levels in ultrasensitive sequencing applications. *DNA Res.,* 2016, vol. 23, 547-559
- **AMINI,S ; PUSHKAREV,D ; CHRISTIANSEN,L ; KOSTEM,E ; ROYCE,T ; TURK,C ; PIGNATELLI,N ; ADEY,A. ; KITZMAN,J.O ; VIJAY-AN,K et al.** Haplotype-resolved whole-genome sequencing by contiguity-preserving transposition and combinatorial indexing. *Nat. Genet.,* 2014, vol. 46, 1343-1349
- **ZHENG,G.X.Y. ; LAU,B.T ; SCHNALL-LEVIN,M ; JAROSZ,M ; BELL,J.M. ; HINDSON,C.M. ; KYRIAZOPOULOU-PANAGIOTOPOULOU,S ; MASQUELIER,D.A ; MERRILL,L ; TERRY,J.M. et al.** Haplotyping germline and cancer genomes with high-throughput linked-read sequencing. *Nat. Biotechnol.,* 2016, vol. 34, 303-311
- Learning with Confident Examples: Rank Pruning for Robust Classification with Noisy Labels. **NORTHCUTT,C.G ; WU,T. ; CHUANG,I.L.** Proceedings of the Thirty-Third Conference on Uncertainty in Artificial Intelligence. AUAI Press, 2017
- **NATARAJAN,N ; DHILLON,I.S ; RAVIKUMAR,P.K. ; TEWARI,A.** Learning with noisy labels. *Advances in neural information processing systems.,* 2013, 1196-1204
- **HELLNER,K ; MIRANDA,F ; FOTSO CHEDOM,D ; HERRERO-GONZALEZ,S ; HAYDEN,D.M ; TEARLE,R ; ARTIBANI,M. ; KARAMINEJADRANJBAR,M ; WILLIAMS,R ; GAITSKELL,K. et al.** Premalignant SOX2 overexpression in the fallopian tubes of ovarian cancer patients: Discovery and validation studies. *EBioMedicine,* 2016, vol. 10, 137-149
- **LAKS,E ; ZAHN,H ; LAI,D ; MCPHERSON,A ; STEIF,A ; BRIMHALL,J. ; BIELE,J ; WANG,B. ; MASUD,T ; GREWAL,D. et al.** Resource: Scalable whole genome sequencing of 40,000 single cells identifies stochastic aneuploidies, genome replication states and clonal repertoires. *bioRxiv,* 2018
- **MOORE,L ; LEONGAMORNLERT,D. ; COORENS,T.H.H ; SANDERS,M.A. ; ELLIS,P ; DAWSON,K. ; MAURA,F. ; NANGALIA,J ; TARPEY,P.S ; BRUNNER,S.F. et al.** The mutational landscape of normal human endometrial epithelium. *bioRxiv,* 2018
- **LEE-SIX,H. ; ELLIS,P. ; OSBORNE,R.J. ; SANDERS,M.A ; MOORE,L ; GEORGAKOPOULOS,N ; TORRENTE,F. ; NOORANI,A ; GODDARD,M ; ROBINSON,P et al.** The landscape of somatic mutation in normal colorectal epithelial cells. *bioRxiv,* 2018
- **BURGESS,D.J.** Spatial transcriptomics coming of age. *Nat. Rev. Genet.,* 2019, vol. 20, 317